(19) Europäisches Patentamt
European Patent Office
Office européen des brevets

(11) **EP 2 804 630 B1**

(12) **EUROPEAN PATENT SPECIFICATION**

(45) Date of publication and mention
of the grant of the patent:
**18.10.2017 Bulletin 2017/42**

(51) Int Cl.:
*A61K 39/395* (2006.01)       *C07K 16/26* (2006.01)
*C07K 16/22* (2006.01)       *C12Q 1/68* (2006.01)
*A61K 39/00* (2006.01)

(21) Application number: **13701354.6**

(22) Date of filing: **18.01.2013**

(86) International application number:
**PCT/US2013/022103**

(87) International publication number:
**WO 2013/109856 (25.07.2013 Gazette 2013/30)**

(54) **METHODS OF USING FGF19 MODULATORS**

VERFAHREN ZUR VERWENDUNG VON FGF19-MODULATOREN

PROCÉDÉS D'UTILISATION DE MODULATEURS FGF19

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **18.01.2012 US 201261587885 P**

(43) Date of publication of application:
**26.11.2014 Bulletin 2014/48**

(73) Proprietor: **F. Hoffmann-La Roche AG**
**4070 Basel (CH)**

(72) Inventors:
• **PAI, Rama**
**South San Francisco, California 94080 (US)**
• **DAMBACH, Donna M.**
**South San Francisco, California 94080 (US)**
• **FRENCH, Dorothy**
**South San Francisco, California 94080 (US)**
• **LAURIAULT, Veronique V.**
**San Francisco, California 94114 (US)**

(74) Representative: **Brodbeck, Michel**
**F. Hoffmann-La Roche AG**
**Patent Department**
**Grenzacherstrasse 124**
**4070 Basel (CH)**

(56) References cited:
US-A1- 2003 203 939    US-A1- 2007 248 604
US-A1- 2007 248 604    US-A1- 2007 248 604

• **SKELTON F S ET AL: "The effects of cycloheximide, actinomycin D, and ethionine on the biliary excretion of labelled alpha-naphthylisothiocyanate in rats",** EXPERIMENTAL AND MOLECULAR PATHOLOGY, ACADEMIC PRESS, US, vol. 23, no. 2, 1 October 1975 (1975-10-01), pages 171-180, XP026216030, ISSN: 0014-4800, DOI: 10.1016/0014-4800(75)90015-5 [retrieved on 1975-10-01]
• **WALTERS J R F ET AL: "A New Mechanism for Bile Acid Diarrhea: Defective Feedback Inhibition of Bile Acid Biosynthesis",** CLINICAL GASTROENTEROLOGY AND HEPATOLOGY, AMERICAN GASTROENTEROLOGICAL ASSOCIATION, US, vol. 7, no. 11, 1 November 2009 (2009-11-01), pages 1189-1194, XP026876775, ISSN: 1542-3565 [retrieved on 2009-05-06]
• **KWANG-HOON SONG ET AL: "Bile acids activate fibroblast growth factor 19 signaling in human hepatocytes to inhibit cholesterol 7[alpha]-hydroxylase gene expression",** HEPATOLOGY, vol. 49, no. 1, 1 January 2009 (2009-01-01), pages 297-305, XP055058932, ISSN: 0270-9139, DOI: 10.1002/hep.22627
• **BUMBACA DANIELA ET AL: "Highly specific off-target binding identified and eliminated during the humanization of an antibody against FGF receptor 4",** MABS, LANDES BIOSCIENCE, US, vol. 3, no. 4, 1 July 2011 (2011-07-01), pages 376-386, XP009159783, ISSN: 1942-0870, DOI: 10.4161/MABS.3.4.15786

- AI-LUEN WU ET AL: "FGF19 Regulates Cell Proliferation, Glucose and Bile Acid Metabolism via FGFR4-Dependent and Independent Pathways", PLOS ONE, vol. 6, no. 3, 1 January 2011 (2011-01-01) , page e17868, XP055059113, ISSN: 1932-6203, DOI: 10.1371/journal.pone.0017868
- R. PAI ET AL: "Antibody-Mediated Inhibition of Fibroblast Growth Factor 19 Results in Increased Bile Acids Synthesis and Ileal Malabsorption of Bile Acids in Cynomolgus Monkeys", TOXICOLOGICAL SCIENCES, vol. 126, no. 2, 1 April 2012 (2012-04-01) , pages 446-456, XP055059051, ISSN: 1096-6080, DOI: 10.1093/toxsci/kfs011
- LIU Y ET AL: "Hepatoprotection by the farnesoid x receptor agonist GW4064 in rat models of intra- and extrahepatic cholestasis", JOURNAL OF CLINICAL INVESTIGATION, AMERICAN SOCIETY FOR CLINICAL INVESTIGATION, US, vol. 112, no. 11, 1 January 2003 (2003-01-01), pages 1678-1687, XP002992678, ISSN: 0021-9738, DOI: 10.1172/JCI200318945
- AI-LUEN WU ET AL: "FGF19 Regulates Cell Proliferation, Glucose and Bile Acid Metabolism via FGFR4-Dependent and Independent Pathways", PLOS ONE, vol. 6, no. 3, 1 January 2011 (2011-01-01) , page e17868, XP055059113, ISSN: 1932-6203, DOI: 10.1371/journal.pone.0017868
- WALTERS J R F ET AL: "A New Mechanism for Bile Acid Diarrhea: Defective Feedback Inhibition of Bile Acid Biosynthesis", CLINICAL GASTROENTEROLOGY AND HEPATOLOGY, AMERICAN GASTROENTEROLOGICAL ASSOCIATION, US, vol. 7, no. 11, 1 November 2009 (2009-11-01), pages 1189-1194, XP026876775, ISSN: 1542-3565 [retrieved on 2009-05-06]
- KWANG-HOON SONG ET AL: "Bile acids activate fibroblast growth factor 19 signaling in human hepatocytes to inhibit cholesterol 7[alpha]-hydroxylase gene expression", HEPATOLOGY, vol. 49, no. 1, 1 January 2009 (2009-01-01), pages 297-305, XP055058932, ISSN: 0270-9139, DOI: 10.1002/hep.22627
- R. PAI ET AL: "Antibody-Mediated Inhibition of Fibroblast Growth Factor 19 Results in Increased Bile Acids Synthesis and Ileal Malabsorption of Bile Acids in Cynomolgus Monkeys", TOXICOLOGICAL SCIENCES, vol. 126, no. 2, 1 April 2012 (2012-04-01) , pages 446-456, XP055059051, ISSN: 1096-6080, DOI: 10.1093/toxsci/kfs011
- SKELTON F S ET AL: "The effects of cycloheximide, actinomycin D, and ethionine on the biliary excretion of labelled alpha-naphthylisothiocyanate in rats", EXPERIMENTAL AND MOLECULAR PATHOLOGY, ACADEMIC PRESS, US, vol. 23, no. 2, 1 October 1975 (1975-10-01), pages 171-180, XP026216030, ISSN: 0014-4800, DOI: 10.1016/0014-4800(75)90015-5 [retrieved on 1975-10-01]
- WALTERS J R F ET AL: "A New Mechanism for Bile Acid Diarrhea: Defective Feedback Inhibition of Bile Acid Biosynthesis", CLINICAL GASTROENTEROLOGY AND HEPATOLOGY, AMERICAN GASTROENTEROLOGICAL ASSOCIATION, US, vol. 7, no. 11, 1 November 2009 (2009-11-01), pages 1189-1194, XP026876775, ISSN: 1542-3565 [retrieved on 2009-05-06]
- KWANG-HOON SONG ET AL: "Bile acids activate fibroblast growth factor 19 signaling in human hepatocytes to inhibit cholesterol 7[alpha]-hydroxylase gene expression", HEPATOLOGY, vol. 49, no. 1, 1 January 2009 (2009-01-01), pages 297-305, XP055058932, ISSN: 0270-9139, DOI: 10.1002/hep.22627
- BUMBACA DANIELA ET AL: "Highly specific off-target binding identified and eliminated during the humanization of an antibody against FGF receptor 4", MABS, LANDES BIOSCIENCE, US, vol. 3, no. 4, 1 July 2011 (2011-07-01), pages 376-386, XP009159783, ISSN: 1942-0870, DOI: 10.4161/MABS.3.4.15786
- AI-LUEN WU ET AL: "FGF19 Regulates Cell Proliferation, Glucose and Bile Acid Metabolism via FGFR4-Dependent and Independent Pathways", PLOS ONE, vol. 6, no. 3, 1 January 2011 (2011-01-01) , page e17868, XP055059113, ISSN: 1932-6203, DOI: 10.1371/journal.pone.0017868
- R. PAI ET AL: "Antibody-Mediated Inhibition of Fibroblast Growth Factor 19 Results in Increased Bile Acids Synthesis and Ileal Malabsorption of Bile Acids in Cynomolgus Monkeys", TOXICOLOGICAL SCIENCES, vol. 126, no. 2, 1 April 2012 (2012-04-01) , pages 446-456, XP055059051, ISSN: 1096-6080, DOI: 10.1093/toxsci/kfs011

**Description**

**FIELD**

Provided herein are methods of using FGF19 modulators and/or bile acid metabolism biomarkers.

**BACKGROUND**

Bile acids are physiological detergents synthesized from cholesterol in the liver, stored in the gall bladder and released into the small intestine for the absorption of fats and fat-soluble nutrients. Chiang JY. 2004. J Hepatol 40(3):539-51; Russell DW. 2003. Annu Rev Biochem 72:137-74. Over 90% of the bile acids are reabsorbed in the ileum, which is primarily achieved by carrier-mediated mechanisms consisting of apical uptake from the lumen, intracellular trafficking to the basolateral membrane, and subsequent efflux into the portal circulation for return to the liver. Cholesterol is converted into two primary bile acids in human liver, cholic acid (CA) and chenodeoxycholic acid (CDCA), whose synthesis is regulated by key enzymes including, Cyp7α1, Cyp8β1, Cyp27α1, and Cyp7β1. The primary bile acids secreted in bile are conjugated to glycine and taurine, which are then hydrolyzed or dehydroxylated in the intestine to form secondary bile acids, deoxycholic acid (DCA) and lithocholic acid (LCA), respectively that are absorbed by passive diffusion across the epithelium. Chiang JY. 2009. J Lipid Res 50(10):1955-66. Although differences in primary bile acids synthesis and metabolism have been identified in humans (CA and CDCA) and rodents (α- and β-muricholic acid) (Russell DW. 2003. Annu Rev Biochem 72:137-74), cynomolgus monkeys and humans are similar with regard to cholesterol turnover and bile acid synthesis (Dietschy JM and Turley SD. 2002. J Biol Chem 277(6):3801-4; Hayes KC. 1988. Nutr Res Rev 1(1):99-113; Rudel LL et al. 2002. J Biol Chem 277(35):31401-6), but differ in their detoxification pathway (Alnouti Y. 2009. Toxicol Sci 108(2):225-46).

The farnesoid X receptor (FXR, NR1H4), a member of steroid/thyroid hormone receptor family, is a bile acid activated transcription factor. Makishima M. et al. 1999. Science 284(5418):1362-5; Parks DJ et al. 1999. Science 284(5418):1365-8. In liver, FXR-initiated ATP-binding cassette (ABC) transporters, bile salt export pump (BSEP, ABCB 11) and multidrug resistant protein 2 (MRP2, ABCC2) are essential in transporting bile acids and bile constituents from the hepatocytes into the bile. Ananthanarayanan M. et al. 2001. J Biol Chem 276(31):28857-65; Kast HR et al. 2002. J Biol Chem 277(4):2908-15; Plass JR et al. 2002. Hepatology 35(3):589-96. The sodium taurocholate cotransporting polypeptide (NTCP, Slc10a1) and organic anion transporter (OAT) polypeptide family of proteins play important roles in bile salt uptake at the basolateral membrane of hepatocytes. Eloranta JJ and Kullak-Ublick GA. 2008. Physiology (Bethesda) 23:286-95. In the ileum, transporters including NTCP and apical sodium-dependent bile acid transporter (ASBT, Slc10a2) mediate bile acid uptake across the apical brush border membrane, and ileal bile acid binding protein (IBABP) mediates intracellular transport. Subsequently, organic solute transporters α-β (OST-α, OST-β) located on the basolateral membrane efflux bile acids into the portal circulation. Ballatori N. et al. 2005. Hepatology 42(6):1270-9; Dawson PA et al. 2005. J Biol Chem 280(8):6960-8. Thus, in addition to bile acid and lipoprotein metabolism, FXR-regulated transporters play an important role in bile acid transport and enterohepatic cycling and homeostasis. Ballatori N. et al. 2008. Am J Physiol Gastrointest Liver Physiol 295(1):G179-G186; Dawson PA et al. 2003. J Biol Chem 278(36):33920-7; Nakahara M. et al. 2005. J Biol Chem 280(51):42283-9.

Fibroblast growth factor 19 (FGF19) is an atypical member of the fibroblast growth factor family that can regulate, among other targets, glucose metabolism and hepatic bile acid metabolism through repression of the gene encoding Cyp7α1, the first rate-limiting step in the synthesis of bile acids. Inagaki T. et al. 2005. Cell Metab 2(4):217-25. Mouse FGF15 has been identified as a structural and functional homologue of human and chick FGF19 with some conserved enhancer/promoter elements. Wright TJ et al. 2004. Dev Biol 269(1):264-75. FGF15/ FGF19 can bind to fibroblast growth factor receptor 4 (FGFR4) and prevents activation of Cyp7α1, thereby preventing bile acid synthesis. FGF19 secreted by the gut acts as an endocrine hormone by binding to FGFR4 on hepatocytes to initiate the c-jun N-terminal kinase (JNK) signaling pathway and subsequently suppress hepatic bile acid synthesis. Jones S. 2008. Mol. Pharma. 5(1):42-48. FGFR4 KO mice have a larger bile acid pool, increased excretion of bile acids, and bile acid-depleted gall bladders, indicating negative regulation of cholesterol and bile acid synthesis. Xie MH et al. 1999. Cytokine 11(10):729-35; Yu C. et al. 2000. J Biol Chem 275(20):15482-9. FXR has been shown to directly regulate FGF19, which signals through the FGFR4 receptor tyrosine kinase, and that FGF19 strongly represses expression of Cyp7α1 in primary hepatocytes and in mouse liver. Holt JA et al. 2003. Genes Dev 17(13):1581-91. Recently β-klotho, a transmembrane protein with no predicted kinase activity, has been identified as a co-factor required for liver specific activities of FGF19 (Lin BC et al. 2007. J Biol Chem 282(37):27277-84) and impaired negative feedback suppression of bile acid synthesis has been described in β-klotho-deficient mice. Ito S. et al. 2005. J Clin Invest 115(8):2202-8.

Previous work showed that therapeutic FGF19 neutralization using anti-FGF19 antibodies inhibits tumor growth in rodent models of colon and hepatocellular carcinoma (Desnoyers LR et al. 2008. Oncogene 27(1):85-97) without any significant toxicity. However, in cynomolgus monkeys, which have a similar bile acid synthesis pathway comprising CA and CDCA and FGF19 with greater homology to human FGF19, dose related toxicity resulted. In particular, a repeat-dose safety study in cynomolgus monkeys, which is described herein, resulted in a dose-related liver toxicity accompanied by severe diarrhea and low food consumption. The mechanisms of toxicity associated with FGF19 modulator (*e.g.,* FGF19 antagonist, *e.g.,* anti-FGF19 antibody) treatment needs to be further understood in order to develop therapies in which mitigate any toxicity associated with FGF19 modulator (*e.g.,* FGF19 antagonist, *e.g.,* anti-FGF19 antibody).

US 2007/248604 relates to a therapeutically useful anti-FGF19 antibodies. It is disclosed in Examples 11 and 12 that the treatment with anti-FGF19 mAb 1A6 inhibited tumor growth and tumor formation of colon and hepatocellular carcinoma. Song KH et al., Hepatology. 49(1):297-305 (2009) discloses that anti-FGF19 antibody abrogated the inhibition of CYP7A1, the enzyme known to initiate the pathway of bile acid synthesis.

## SUMMARY

The description includes methods for treating a disease or disorder in an individual comprising administering to the individual an effective amount of an FGF19 modulator and assessing levels of one or more bile acid metabolism biomarkers in a sample from the individual (*e.g.,* compared to a reference) during treatment with the FGF19 modulator.

In another aspect, disclosed herein are methods of treating a disease or disorder in an individual comprising administering to the individual an effective amount of an FGF19 modulator, wherein treatment is based upon levels of one or more bile acid metabolism biomarkers in a sample from the individual (e.g., compared to a reference).

Further disclosed herein are methods for treating a disease or disorder in an individual, the method comprising: determining that a sample from the individual comprises substantially normal levels or reduced levels of one or more bile acid metabolism biomarkers (*e.g.,* compared to a reference), and administering an effective amount of an FGF19 modulator to the individual, whereby the disease or disorder is treated.

In addition, disclosed herein are methods of treating a disease or disorder, comprising: (a) selecting an individual having the disease or disorder, wherein the individual comprising substantially normal levels or reduced levels of one or more bile acid metabolism biomarkers in a sample from the individual (*e.g.,* compared to a reference); and (b) administering to the individual thus selected an effective amount of an FGF19 modulator, whereby the disease or disorder is treated.

Disclosed herein are methods of identifying an individual who is more or less likely to exhibit benefit from treatment comprising an FGF19 modulator, the method comprising: determining levels of one or more bile acid metabolism biomarkers in a sample from the individual, wherein reduced levels and/or substantially normal levels of one or more bile acid metabolism biomarkers (*e.g.,* compared to a reference) in the sample indicates that the individual is more likely to exhibit benefit from treatment comprising the FGF19 modulator or elevated levels of one or more bile acid metabolism biomarkers (*e.g.,* compared to a reference) indicates that the individual is less likely to exhibit benefit from treatment comprising the FGF19 modulator.

Further disclosed herein are methods for predicting whether an individual with a disease or disorder is more or less likely to develop toxicity to treatment comprising an FGF19 modulator, the method comprising determining levels of one or more bile acid metabolism biomarkers in a sample from the individual, whereby elevated levels of one or more bile acid metabolism biomarkers (*e.g.,* compared to a reference) indicates that the individual is more likely to develop toxicity to treatment comprising the FGF19 modulator and reduced levels and/or substantially normal levels of one or more bile acid metabolism biomarkers (*e.g.,* compared to a reference) indicates that the individual is less likely to develop toxicity to treatment comprising the FGF19 modulator. In some embodiments, the toxicity is diarrhea (*e.g.,* sever diarrhea), dehydration, low food consumption, decreased body weight, and/or morbidity.

The invention provides methods of determining whether an individual with cancer should continue or discontinue treatment comprising an FGF19 antagonist antibody, the method comprising measuring in a sample obtained from the individual levels of one or more bile acid metabolism biomarkers in a sample from the individual, i) wherein elevated levels of one or more bile acid metabolism biomarkers indicates that the individual should discontinue treatment comprising the FGF19 antagonist antibody as the individual is more likely to develop toxicity to the treatment and ii) reduced levels of one or more bile acid metabolism biomarkers indicates that the individual should continue treatment comprising the FGF19 antagonist antibody as the individual is less likely to develop toxicity to the treatment, wherein the bile acid metabolism biomarker is BSEP, MRP2 and/or MRP3, wherein the anti-FGF19 antagonist antibody comprises: (i) a light chain comprising (a) hypervariable region (HVR)-L1 comprising amino acid sequence KASQDINSFLA (SEQ ID NO:1) or KASQDINSFLS (SEQ ID NO:7); (b) HVR-L2 comprising amino acid sequence RANRLVD (SEQ ID NO:2), RANRLVS (SEQ ID NO:8), or RANRLVE (SEQ ID NO:9); and (c) HVR-L3 comprising amino acid sequence LQYDEFPLT (SEQ ID NO:3); and (ii) a heavy chain comprising (a) HVR-H1 comprising amino acid sequence GFSLTTYGVH (SEQ ID NO:4); (b) HVR-H2 comprising amino acid sequence is GVIWPGGGTDYNAAFIS (SEQ ID NO:5); and (c) HVR-H3 comprising amino acid sequence VRKEYANLYAMDY (SEQ ID NO:6).

Disclosed herein are also methods of identifying an individual as more likely suitable or less likely suitable to continue a treatment, wherein treatment comprises an FGF19 modulator, based upon levels of one or more bile acid metabolism biomarkers in a sample from the individual, wherein elevated levels of one or more bile acid metabolism biomarkers (*e.g.,* compared to a reference) identifies the individual less likely suitable to continue the treatment comprising the FGF19 modulator and reduced levels and/or substantially normal levels of one or more bile acid metabolism biomarkers (*e.g.,* compared to a reference) identifies the individual more likely suitable to continue the treatment comprising the FGF19 modulator.

Disclosed herein are methods of optimizing therapeutic efficacy and/or reducing toxicity associated with a treatment of a disease or disorder in an individual having the disease or disorder undergoing the treatment, comprising: determining the levels of one or more bile acid metabolism biomarkers in a sample from the individual, wherein elevated levels of one or more bile acid metabolism biomarkers indicates a need to decrease the amount and/or frequency of subsequently administered of the FGF19 modulator to the individual.

Further disclosed herein are methods of identifying an individual having a disease or disorder as more likely suitable or less likely suitable to continue a dose and/or dosage schedule of a treatment, wherein treatment comprises an FGF19 modulator, based upon levels of one or more bile acid metabolism biomarkers in a sample from the individual, wherein elevated levels of one or more bile acid metabolism biomarkers (*e.g.,* compared to a reference) identifies the individual less likely suitable to continue the dose and/or dosage schedule of the treatment comprising the FGF19 modulator and reduced levels and/or substantially normal levels of one or more bile acid metabolism biomarkers (*e.g.,* compared to a reference) identifies the individual more likely suitable to continue the dose and/or dosage schedule of the treatment comprising the FGF19 modulator.

Disclosed herein are assay methods for optimizing dose efficacy in an individual receiving an FGF19 modulator, the method comprising: (a) determining levels of one or more bile acid metabolism biomarkers in a sample from the individual; (b) recommending a subsequent dose of the FGF19 modulator based upon the level of the bile acid metabolism biomarker.

In another aspect, disclosed are assay methods for evaluating the risk that an individual will develop toxicity to an FGF19 modulator, the method comprising: (a) determining levels of one or more bile acid metabolism biomarkers in a sample from the individual; (b) evaluating the risk that the individual will develop toxicity to the FGF19 modulator based upon the levels of one or more bile acid metabolism biomarkers.

In some embodiments of any of the methods, the method further comprises administering an effective amount of the FGF19 modulator (*e.g.,* to the individual having reduced levels and/or substantially normal levels of one or more bile acid metabolism biomarkers (*e.g.,* compared to a reference)).

In some embodiments of any of the methods, the bile acid metabolism biomarker is a liver enzyme. In some embodiments, the liver enzyme is a serum liver enzymes.

In some embodiments of any of the methods, the bile acid metabolism biomarker is a bile acid. In some embodiments, the bile acid is total bile acid. In some embodiments, the bile acid is a hydrophobic bile acid. In some embodiments, the bile acid is a secondary bile acid. In some embodiments, wherein the secondary bile acid is lithocholic acid. In some embodiments, the secondary bile acid is deoxycholic acid.

In some embodiments of any of the methods, the bile acid metabolism biomarker is aspartate aminotransferase (AST), alanine transaminase (ALT), gamma glutamyl transpeptidase (GGT), alpha-L-fucosidase (AFU), adenosine deaminase (ADA), cholinesterase activity (CHE), alpha-fetoprotein (AFP), and/or total bilirubin levels (TBIL).

In some embodiments of any of the methods, the bile acid metabolism biomarker is Cyp7$\alpha$1, BSEP, MRP2, MRP3, Ost-$\alpha$, Ost-$\beta$, and/or IBABP.

In some embodiments of any of the methods, the bile acid metabolism biomarker is increase by greater than about 1.1-fold, about 1.5-fold, about 2-fold, about 2.5-fold, about 3-fold, about 4-fold, about 5-fold, about 10-fold, about 15-fold, and/or about 20-fold.

In some embodiments of any of the methods, the reference is an average level of the one or more bile acid metabolism biomarker of healthy individual and/or healthy population of individuals (*e.g.,* wherein the level is measure by a similar and/or same method). In some embodiments of any of the methods, the reference is an average level of the one or more bile acid metabolism biomarker of a second individual having the disease or disorder and/or population of individuals having the disease or disorder (*e.g.,* wherein the level is measure by a similar and/or same method). In some embodiments of any of the methods, the reference is the level of the one or more bile acid metabolism biomarkers prior to starting treatment and/or at the time of starting treatment with the FGF19 modulator.

In some embodiments of any of the methods, the sample is a serum sample. In some embodiments of any of the methods, the sample is a fecal sample. In some embodiments of any of the methods, the sample is a urine sample. In some embodiments, the sample is a tissue sample (*e.g.,* liver tissue sample and/or ileum tissue sample).

In some embodiments of any of the methods, the FGF19 modulator is an FGF19 antagonist. In some embodiments, the FGF19 antagonist inhibits and/or binds FGF19, FGFR4, and/or klotho (*e.g.,* KLB). In some embodiments, the FGF19 antagonist is an antibody, binding polypeptide, small molecule, and/or polynucleotide. In some embodiments, the FGF19 antagonist is an anti-FGF19 antibody. In some embodiments, the antibody is a monoclonal antibody. In some embodiments, the antibody is a human, humanized, or chimeric antibody. In some embodiments, the anti-FGF19 antibody comprises: (i) a light chain comprising (a) hypervariable region (HVR)-L1 comprising amino acid sequence KASQDINSFLA (SEQ ID NO:1) or KASQDINSFLS (SEQ ID NO:7); (b) HVR-L2 comprising amino acid sequence RANRLVD (SEQ ID NO:2), RANRLVS (SEQ ID NO:8), or RANRLVE (SEQ ID NO:9); and (c) HVR-L3 comprising amino acid sequence LQYDEFPLT (SEQ ID NO:3); and (ii) a heavy chain comprising (a) HVR-H1 comprising amino acid sequence GFSLTTYGVH (SEQ ID NO:4); (b) HVR-H2 comprising amino acid sequence is GVIWPGGGTDYNAAFIS (SEQ ID NO:5); and (c) HVR-H3 comprising amino acid sequence VRKEYANLYAMDY (SEQ ID NO:6).

In some embodiments, HVR-L2 comprises amino acid sequence RANRLVD (SEQ ID NO:2). In some embodiments, HVR-L1 comprises amino acid sequence KASQDINSFLS (SEQ ID NO:7).

In some embodiments, HVR-L2 comprises amino acid sequence RANRLVS (SEQ ID NO:8). In some embodiments, HVR-L2 comprises amino acid sequence RANRLVE (SEQ ID NO:9). In some embodiments, HVR-L1 comprises amino acid sequence KASQDINSFLA (SEQ ID NO:1).

In some embodiments, the antibody comprises (i) human κ subgroup 1 consensus framework sequence, or (ii) heavy chain human subgroup III consensus framework sequence.

In some embodiments of any of the methods, the dosage of the FGF19 modulator (*e.g.,* FGF19 antagonist, *e.g.,* anti-FGF19 antibody) is greater than or equal to 3 mg/kg (*e.g.,* greater than or equal to about 10 mg/kg, about 30 mg/kg, and/or about 100 mg/kg and/or about 10-100 mg/kg).

In some embodiments of any of the methods, the disease or disorder is a proliferative disease or disorder. In some embodiments, the proliferative disease or disorder is cancer. In some embodiments, the cancer is hepatocellular carcinoma.

The article of manufacture comprises a container and a label or package insert on or associated with the container, wherein the container comprises an FGF19 modulator (*e.g.,* FGF19 antagonist) and the label on the container indicates that the composition can be used for treating and/or continuing treatment of an individual having a disease or disorder based upon levels of one or bile acids. In some embodiments, the FGF19 modulator is an anti-FGF19 antibody described herein. In some embodiments of any of the methods, the disease or disorder is a proliferative disease or disorder. In some embodiments, the proliferative disease or disorder is cancer.

## BRIEF DESCRIPTION OF THE FIGURES

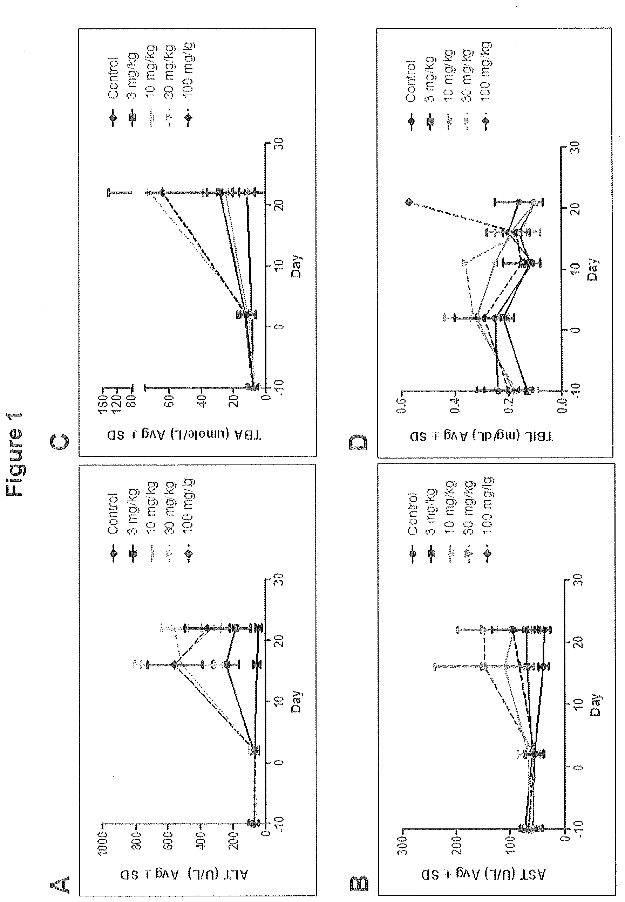

**Figure 1:** Anti-FGF19 antibody treatment of cynomolgus monkeys caused increase in serum **A)** aspartate aminotransferase (AST), **B)** alanine transaminase (ALT), **C)** total bile acids (TBA), and **D)** total bilirubin (TBIL) levels across all doses. However, 3 mg/kg treated animals indicated some tolerance with subsequent doses. Values are Mean ± SD U/L for AST and ALT, μmole/ L for TBA and mg/dL for TBIL (n =5 ± SD).

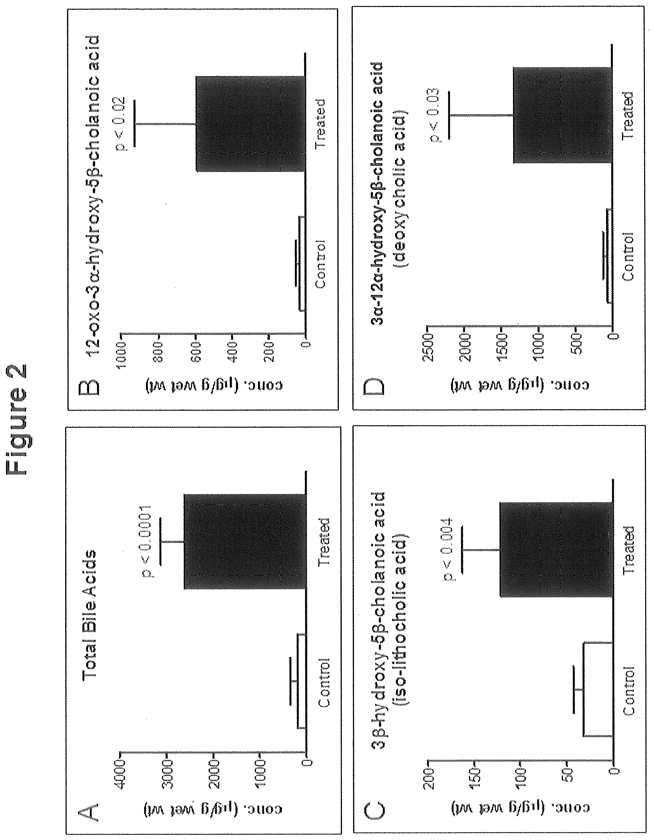

**Figure 2A-D:** Anti-FGF19 antibody treatment causes increased fecal excretion of bile acids and their derivatives. Total bile acid amounts and characterization of secondary bile acids in monkey excretions from vehicle and 100 mg/kg were analyzed using GC-MS method. Concentration of bile acids was normalized to μg/g wet weight. Values are Mean ± SD (Control, n = 4, Treated, n = 5).

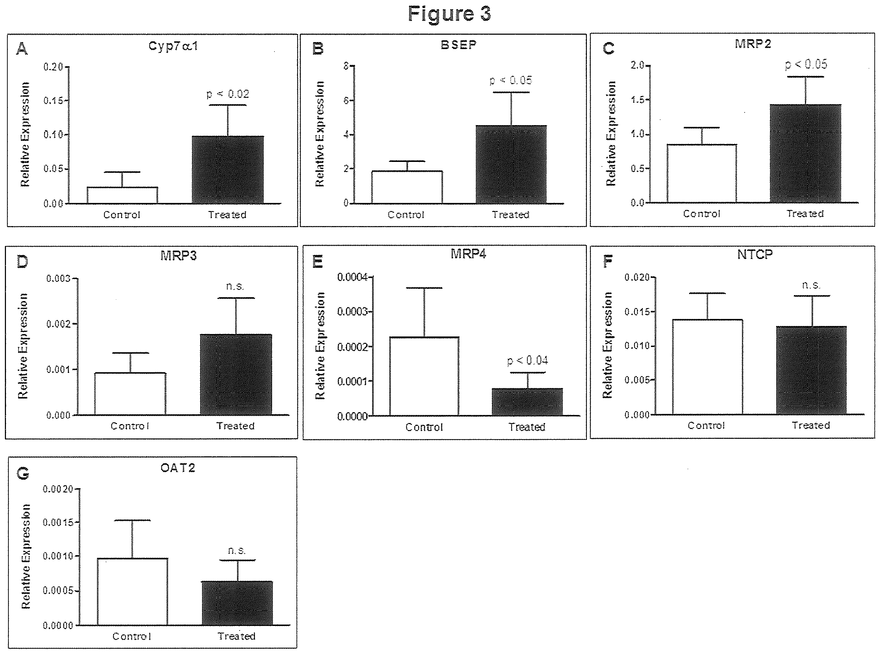

**Figure 3A-G:** Anti-FGF19 antibody treatment modulates Cyp7α1 and bile acids-related gene expression levels in cyno monkey livers. Total RNAs were isolated from the livers of cyno monkeys treated with either anti-FGF19 antibody (100 mg/kg) or vehicle. Relative expression levels of Cyp7α1, BSEP, MRP2, MRP3, MRP4, NTCP and OAT2 were analyzed by quantitative real-time PCR analysis and results were normalized to RPL19. Values are Mean ± SD (Control, n = 4, Treated, n = 6).

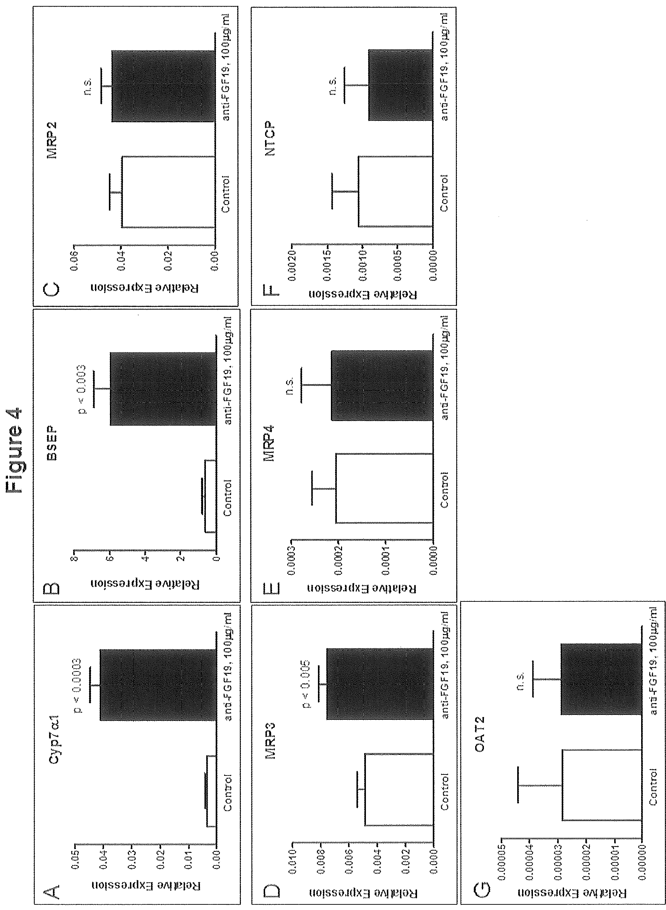

**Figure 4A-G:** Anti-FGF19 antibody treatment modulates Cyp7α1 and bile acids-related gene expression levels in cyno monkey primary hepatocytes. Cryopreserved cyno primary hepatocytes were treated with either isotype control antibody or anti-FGF19 antibody (100 μg/ mL) for 24 h. Relative expression levels of Cyp7α1, BSEP, MRP2, MRP3, MRP4, NTCP and OAT2 were analyzed by quantitative real-time PCR analysis and results were normalized to RPL19. Values are Mean ± SD from three separate experiments performed in duplicate.

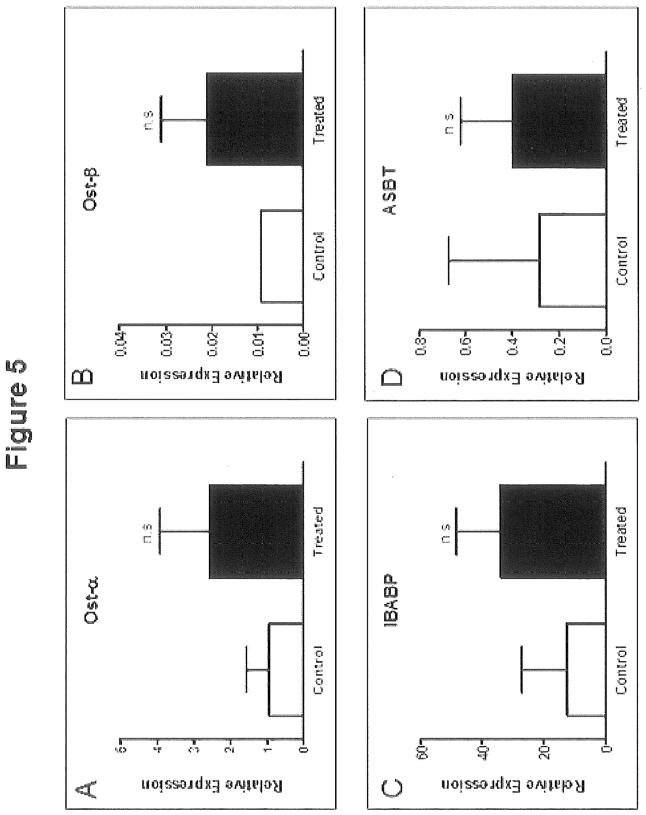

**Figure 5A-D:** Anti-FGF19 antibody treatment modulates bile acids transporter gene expression levels in cynomolgus monkey ileum. Total RNAs were isolated from the ileal tissues of cyno monkeys treated with either anti-FGF19 antibody (100 mg/kg) or vehicle. Relative expression levels of Ost-α, Ost-β, IBABP and ASBT were analyzed by quantitative real-time PCR analysis and results were normalized to RPL19. Values are Mean ± SD (Control, n = 4, Treated, n = 6).

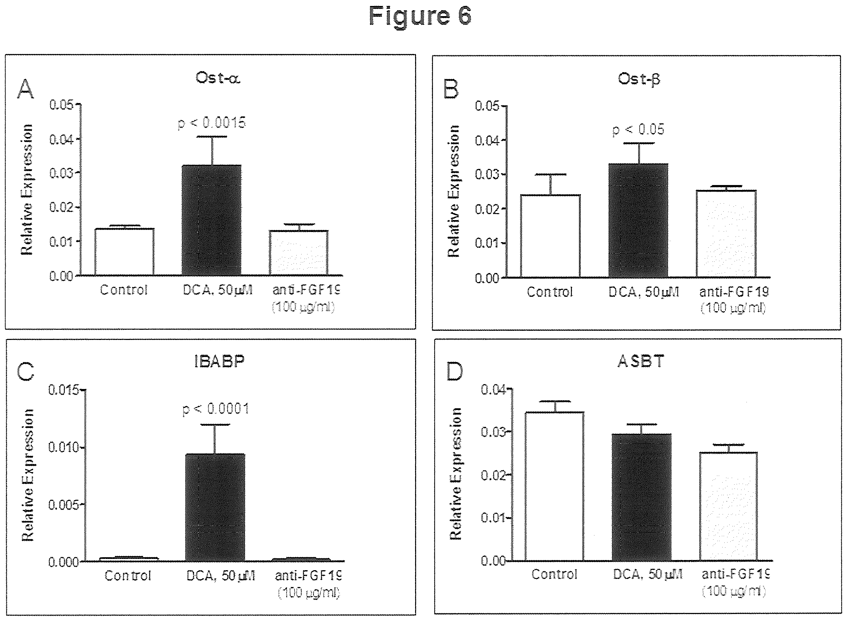

**Figure 6A-D:** Effect of anti-FGF19 antibody and deoxycholic acid (DCA) on bile acids transporter gene expression levels in intestinal epithelial (Caco-2) cells. Caco-2 monolayers were treated with either: a) isotype control antibody, b) anti-FGF19 antibody (both 100 μg/ mL), or c) DCA, 50 μM for 24 h. Total RNAs were isolated and relative expression levels of Ost-α, Ost-β, IBABP and ASBT were analyzed by quantitative real-time PCR analysis and results were normalized to RPL19. Values are Mean ± SD from three separate experiments performed in duplicate.

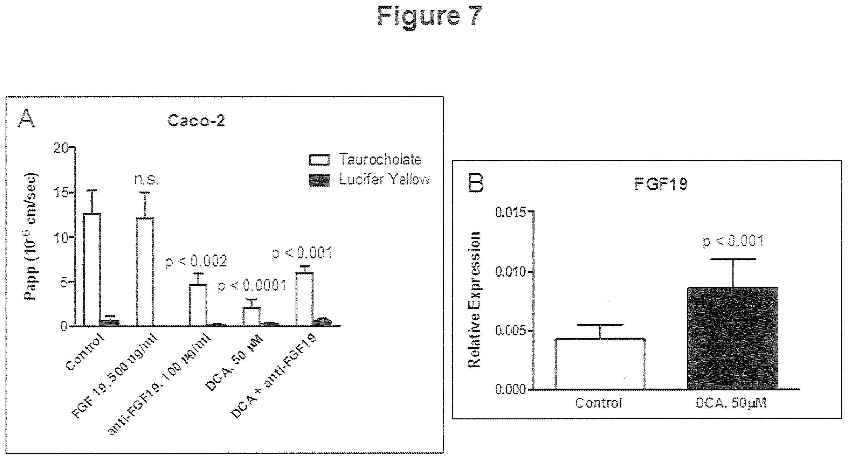

**Figure 7: A)** DCA increases FGF19 mRNA expression in intestinal epithelial cells. Total RNA was isolated from Caco-2 monolayers treated with and without DCA (50 μM) for 24 h. Relative expression level of FGF19 was analyzed by quantitative real-time PCR analysis and results were normalized to RPL19. Values are Mean ± SD from two separate experiments performed in triplicate. **B)** Anti-FGF19 antibody treatment reduces transepithelial electrical resistance without altering membrane integrity. Caco-2 Cells were seeded on the transwell inserts and treated with either isotype control antibody (100 μg/mL), FGF19 (500 ng/mL), anti-FGF19 antibody (100 μg/mL), DCA (50 μM) or DCA (50 μM) plus anti-FGF19 antibody (100 μg/mL) for 24 h and the permeability of LY, atenolol, and taurocholate was evaluated in the apical to basolateral (A-B) direction. Values are Mean ± SD (n = 6).

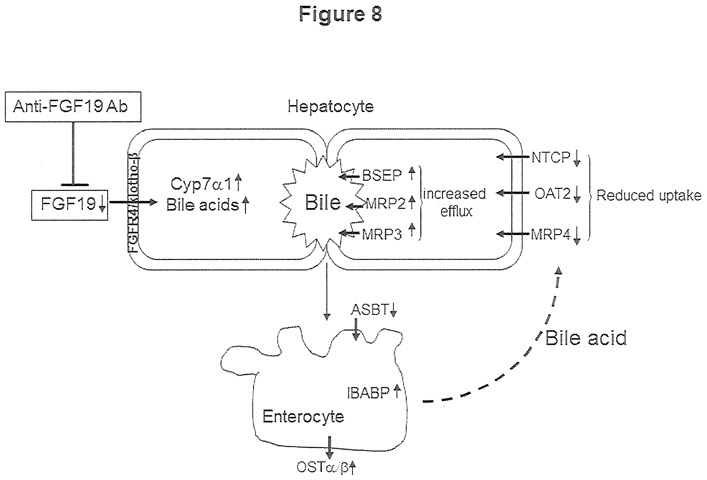

**Figure 8:** Inhibition of FGF19 with antibody increases Cyp7α1 and elevates bile acid synthesis resulting in enhanced bile acid efflux and reduced uptake into the hepatocytes. Increased bile acid alters solute transporters in enterocytes and disrupts enterohepatic recirculation of bile acids subsequently causing diarrhea and liver toxicity.

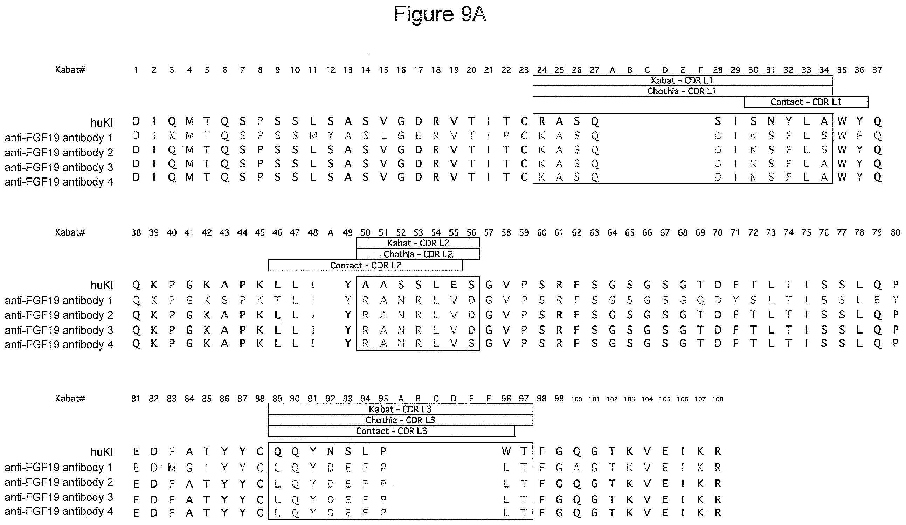

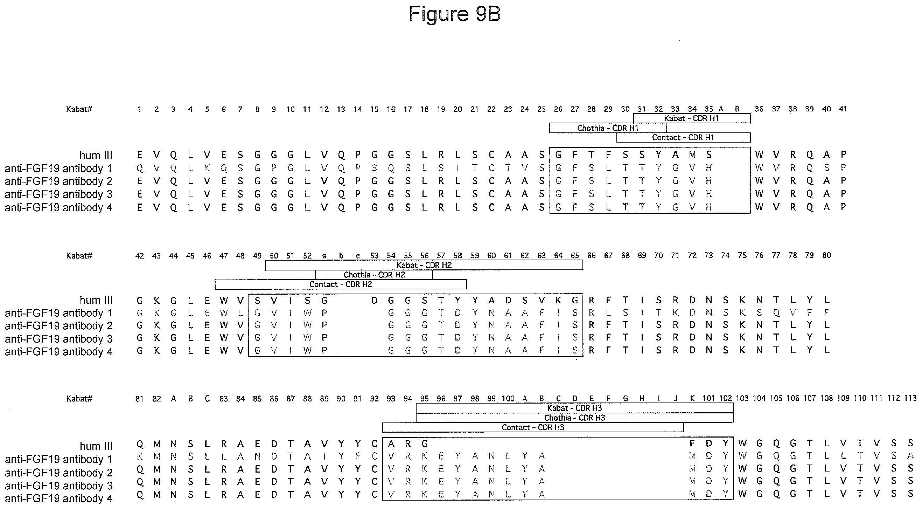

**Figure 9: A)** Variable light chain sequences of anti-FGF19 antibodies (SEQ ID NOS 11-15, respectively, in order of appearance). **B)** Variable heavy chain sequences of anti-FGF19 antibodies (SEQ ID NOS 16-20, respectively, in order of appearance).

## DETAILED DESCRIPTION

### I. DEFINITIONS

The terms "FGF19" and "fibroblast growth factor 19" refer herein to a native FGF19 from any vertebrate source, including mammals such as primates (*e.g.,* humans) and rodents (*e.g.,* mice and rats), unless otherwise indicated. The term encompasses "full-length," unprocessed FGF19 as well as any form of FGF19 that results from processing in the cell. The term also encompasses naturally occurring variants of FGF19, *e.g.,* splice variants or allelic variants. The sequence of an exemplary human FGF19 nucleic acid sequence is Genebank sequence AB018122, AF110400, AY358302, BC017664, and/or BT006729 or an exemplary human FGF19 amino acid sequence is Genebank sequence NP_005108.1.

"FGF19 variant," " fibroblast growth factor 19 variant," or variations thereof, means an FGF19 polypeptide or polynucleotide, generally being or encoding an active FGF19 polypeptide, as defined herein having at least about 80% amino acid sequence identity with any of the FGF19 as disclosed herein. Such FGF19 variants include, for instance, FGF19 wherein one or more nucleic acid or amino acid residues are added or deleted. Ordinarily, an FGF19 variant will have at least about 80% sequence identity, alternatively at least about 81%, 82%, 83%, 84%, 85%, 86%, 87%, 88%, 89%, 90%, 91%, 92%, 93%, 94%, 95%, 96%, 97%, 98%, or 99% sequence identity, to FGF19 as disclosed herein. Ordinarily, FGF19 variant are at least about 10 residues in length, alternatively at least about 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600 in length, or more. Optionally, FGF19 variant will have or encode a sequence having no more than one conservative amino acid substitution as compared to FGF19, alternatively no more than 2, 3, 4, 5, 6, 7, 8, 9, or 10 conservative amino acid substitution as compared to FGF19.

The terms "FGFR4" and "fibroblast growth factor receptor 4" refer herein to a native FGFR4 from any vertebrate source, including mammals such as primates (*e.g.,* humans) and rodents (*e.g.,* mice and rats), unless otherwise indicated. The term encompasses "full-length," unprocessed FGFR4 as well as any form of FGFR4 that results from processing in the cell. The term also encompasses naturally occurring variants of FGF19, *e.g.,* splice variants or allelic variants. The sequence of an exemplary human FGFR4 nucleic acid sequence is Genebank sequence AB209631, AF202063, AF359241, AF359246, AF487555, AK301169, BC011847, EF571596, EU826602, EU826603, L03840, M59373, X57205, and/or Y13901 or an exemplary human FGFR4 amino acid sequence is Genebank sequence NP_998812.1.

The terms "KLB" and " β-Klotho" refer herein to a native KLB from any vertebrate source, including mammals such as primates (*e.g.,* humans) and rodents (*e.g.,* mice and rats), unless otherwise indicated. The term encompasses "full-length," unprocessed KLB as well as any form of KLB that results from processing in the cell. The term also encompasses naturally occurring variants of KLB, *e.g.,* splice variants or allelic variants. The sequence of an exemplary human KLB nucleic acid sequence is Genebank sequence AB079373, AK302436, BC033021, BC104871, and/or BC113653 or an exemplary human KLB amino acid sequence is Genebank sequence NP_783864.1.

The term "FGF19 antagonist" as defined herein is any molecule that partially or fully blocks, inhibits, or neutralizes a biological activity mediated by the FGF19 (*e.g.,* FGF19 polypeptide). In some embodiments, such FGF19 antagonist inhibits and/or binds to FGF19 polypeptide. In some embodiments, such FGF19 antagonist inhibits and/or binds to FGFR4 polypeptide. In some embodiments, such FGF19 antagonist inhibits and/or binds to klotho (*e.g.,* KLB) polypeptide. According to one embodiment, the antagonist is a polypeptide. According to another embodiment, the antagonist is an antibody. According to another embodiment, the antagonist is a small molecule antagonist. According to another embodiment, the antagonist is a polynucleotide antagonist.

"Polynucleotide" or "nucleic acid" as used interchangeably herein, refers to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. A sequence of nucleotides may be interrupted by non-nucleotide components. A polynucleotide may comprise modification(s) made after synthesis, such as conjugation to a label. Other types of modifications include, for example, "caps," substitution of one or more of the naturally occurring nucleotides with an analog, internucleotide modifications such as, for example, those with uncharged linkages (*e.g.,* methyl phosphonates, phosphotriesters, phosphoamidates, carbamates, etc.) and with charged linkages (*e.g.,* phosphorothioates, phosphorodithioates, etc.), those containing pendant moieties, such as, for example, proteins (*e.g.,* nucleases, toxins, antibodies, signal peptides, ply-L-lysine, etc.), those with intercalators (*e.g.,* acridine, psoralen, etc.), those containing chelators (*e.g.,* metals, radioactive metals, boron, oxidative metals, etc.), those containing alkylators, those with modified linkages (*e.g.,* alpha anomeric nucleic acids, etc.), as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, $\alpha$-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR$_2$ ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

"Oligonucleotide," as used herein, refers to generally single-stranded, synthetic polynucleotides that are generally, but not necessarily, less than about 200 nucleotides in length. The terms "oligonucleotide" and "polynucleotide" are not mutually exclusive. The description above for polynucleotides is equally and fully applicable to oligonucleotides.

The term "primer" refers to a single stranded polynucleotide that is capable of hybridizing to a nucleic acid and following polymerization of a complementary nucleic acid, generally by providing a free 3'-OH group.

The term "small molecule" refers to any molecule with a molecular weight of about 2000 Daltons or less, preferably of about 500 Daltons or less.

The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors."

An "isolated" antibody is one which has been separated from a component of its natural environment. In some embodiments, an antibody is purified to greater than 95% or 99% purity as determined by, for example, electrophoretic (*e.g.,* SDS-PAGE, isoelectric focusing (IEF), capillary electrophoresis) or chromatographic (*e.g.,* ion exchange or reverse phase HPLC). For review of methods for assessment of antibody purity, *see, e.g.,* Flatman et al., J. Chromatogr. B 848:79-87 (2007).

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

The term "antibody" herein is used in the broadest sense and encompasses various antibody structures, including but not limited to monoclonal antibodies, polyclonal antibodies, multispecific antibodies (*e.g.,* bispecific antibodies), and antibody fragments so long as they exhibit the desired antigen-binding activity.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')$_2$; diabodies; linear antibodies; single-chain antibody molecules (*e.g.,* scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more. An exemplary competition assay is provided herein.

The terms "full length antibody," "intact antibody," and "whole antibody" are used herein interchangeably to refer to an antibody having a structure substantially similar to a native antibody structure or having heavy chains that contain an Fc region as defined herein.

The term "monoclonal antibody" as used herein refers to an antibody obtained from a population of substantially homogeneous antibodies, *i.e.,* the individual antibodies comprising the population are identical and/or bind the same epitope, except for possible variant antibodies, *e.g.*, containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

"Native antibodies" refer to naturally occurring immunoglobulin molecules with varying structures. For example, native IgG antibodies are heterotetrameric glycoproteins of about 150,000 Daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3). Similarly, from N- to C-terminus, each light chain has a variable region (VL), also called a variable light domain or a light chain variable domain, followed by a constant light (CL) domain. The light chain of an antibody may be assigned to one of two types, called kappa ($\kappa$) and lambda ($\lambda$), based on the amino acid sequence of its constant domain.

The term "chimeric" antibody refers to an antibody in which a portion of the heavy and/or light chain is derived from a particular source or species, while the remainder of the heavy and/or light chain is derived from a different source or species.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (*e.g.,* CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, *e.g.*, a non-human antibody, refers to an antibody that has undergone humanization.

The "class" of an antibody refers to the type of constant domain or constant region possessed by its heavy chain. There are five major classes of antibodies: IgA, IgD, IgE, IgG, and IgM, and several of these may be further divided into subclasses (isotypes), *e.g.,* IgG$_1$, IgG$_2$, IgG$_3$, IgG$_4$, IgA$_1$, and IgA$_2$. The heavy chain constant domains that correspond to the different classes of immunoglobulins are called $\alpha$, $\delta$, $\varepsilon$, $\gamma$, and $\mu$, respectively.

"Effector functions" refer to those biological activities attributable to the Fc region of an antibody, which vary with the antibody isotype. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (*e.g.,* B cell receptor); and B cell activation.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

A "human consensus framework" is a framework which represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, Fifth Edition, NIH Publication 91-3242, Bethesda MD (1991), vols. 1-3. In one embodiment, for the VL, the subgroup is subgroup kappa I as in Kabat *et al., supra.* In one embodiment, for the VH, the subgroup is subgroup III as in Kabat *et al., supra.*

An "acceptor human framework" for the purposes herein is a framework comprising the amino acid sequence of a light chain variable domain (VL) framework or a heavy chain variable domain (VH) framework derived from a human immunoglobulin framework or a human consensus framework, as defined below. An acceptor human framework "derived from" a human immunoglobulin framework or a human consensus framework may comprise the same amino acid sequence thereof, or it may contain amino acid sequence changes. In some embodiments, the number of amino acid changes are 10 or less, 9 or less, 8 or less, 7 or less, 6 or less, 5 or less, 4 or less, 3 or less, or 2 or less. In some embodiments, the VL acceptor human framework is identical in sequence to the VL human immunoglobulin framework sequence or human consensus framework sequence.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs). (*See, e.g.,* Kindt et al. Kuby Immunology, 6th ed., W.H. Freeman and Co., page 91 (2007).) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively. *See, e.g.,* Portolano et al., J. Immunol. 150:880-887 (1993); Clarkson et al., Nature 352:624-628 (1991).

The term "hypervariable region" or "HVR," as used herein, refers to each of the regions of an antibody variable domain which are hypervariable in sequence and/or form structurally defined loops ("hypervariable loops"). Generally, native four-chain antibodies comprise six HVRs; three in the VH (HI, H2, H3), and three in the VL (L1, L2, L3). HVRs generally comprise amino acid residues from the hypervariable loops and/or from the "complementarity determining regions" (CDRs), the latter being of highest sequence variability and/or involved in antigen recognition. Exemplary hypervariable loops occur at amino acid residues 26-32 (L1), 50-52 (L2), 91-96 (L3), 26-32 (H1), 53-55 (H2), and 96-101 (H3). (Chothia and Lesk, J. Mol. Biol. 196:901-917 (1987).) Exemplary CDRs (CDR-L1, CDR-L2, CDR-L3, CDR-H1, CDR-H2, and CDR-H3) occur at amino acid residues 24-34 of L1, 50-56 of L2, 89-97 of L3, 31-35B of H1, 50-65 of H2, and 95-102 of H3. (Kabat et al., Sequences of Proteins of Immunological Interest, 5th Ed. Public Health Service, National Institutes of Health, Bethesda, MD (1991).) With the exception of CDR1 in VH, CDRs generally comprise the amino acid residues that form the hypervariable loops. CDRs also comprise "specificity determining residues," or "SDRs," which are residues that contact antigen. SDRs are contained within regions of the CDRs called abbreviated-CDRs, or a-CDRs. Exemplary a-CDRs (a-CDR-L1, a-CDR-L2, a-CDR-L3, a-CDR-H1, a-CDR-H2, and a-CDR-H3) occur at amino acid residues 31-34 of L1, 50-55 of L2, 89-96 of L3, 31-35B of H1, 50-58 of H2, and 95-102 of H3. (*See* Almagro and Fransson, Front. Biosci. 13:1619-1633 (2008).) Unless otherwise indicated, HVR residues and other residues in the variable domain (*e.g.,* FR residues) are numbered herein according to Kabat *et al., supra.*

"Affinity" refers to the strength of the sum total of noncovalent interactions between a single binding site of a molecule (*e.g.,* an antibody) and its binding partner (*e.g.,* an antigen). Unless indicated otherwise, as used herein, "binding affinity" refers to intrinsic binding affinity which reflects a 1:1 interaction between members of a binding pair (*e.g.,* antibody and antigen). The affinity of a molecule X for its partner Y can generally be represented by the dissociation constant (Kd). Affinity can be measured by common methods known in the art, including those described herein. Specific illustrative and exemplary embodiments for measuring binding affinity are described in the following.

An "affinity matured" antibody refers to an antibody with one or more alterations in one or more hypervariable regions (HVRs), compared to a parent antibody which does not possess such alterations, such alterations resulting in an improvement in the affinity of the antibody for antigen.

The terms "anti-FGF19 antibody" and "an antibody that binds to FGF19 polypeptide" refer to an antibody that is capable of binding FGF19 polypeptide with sufficient affinity such that the antibody is useful as a diagnostic and/or therapeutic agent in targeting FGF19. In one embodiment, the extent of binding of an anti-FGF19 antibody to an unrelated, non-FGF19 polypeptide is less than about 10% of the binding of the antibody to FGF19 polypeptides measured, *e.g.,* by a radioimmunoassay (RIA). In certain embodiments, an antibody that binds to FGF19 polypeptide has a dissociation constant (Kd) of $\leq 1\mu$M, $\leq 100$ nM, $\leq 10$ nM, $\leq 1$ nM, $\leq 0.1$ nM, $\leq 0.01$ nM, or $\leq 0.001$ nM (*e.g.,* $10^{-8}$ M or less, *e.g.,* from $10^{-8}$ M to $10^{-13}$ M, *e.g.,* from $10^{-9}$ M to $10^{-13}$ M). In certain embodiments, an anti- FGF19 antibody binds to an epitope of FGF19 that is unique among FGF19.

A "blocking" antibody or an "antagonist" antibody is one which inhibits or reduces biological activity of the antigen it binds. Preferred blocking antibodies or antagonist antibodies substantially or completely inhibit the biological activity of the antigen.

A "naked antibody" refers to an antibody that is not conjugated to a heterologous moiety (*e.g.,* a cytotoxic moiety) or radiolabel. The naked antibody may be present in a pharmaceutical formulation.

An "immunoconjugate" is an antibody conjugated to one or more heterologous molecule(s), including but not limited to a cytotoxic agent.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

100 times the fraction X/Y

where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "detection" includes any means of detecting, including direct and indirect detection.

The term "biomarker" as used herein refers to an indicator, *e.g.*, predictive, diagnostic, toxicity, and/or prognostic, which can be detected in a sample. The biomarker may serve as an indicator of a particular subtype of a disease or disorder (*e.g.,* cancer) characterized by certain, molecular, pathological, histological, and/or clinical features. In some embodiments, the biomarker is a gene. In some embodiments, the biomarker is a variation (*e.g.,* mutation and/or polymorphism) of a gene. In some embodiments, the biomarker is a metabolic product. Biomarkers include, but are not limited to, polynucleotides (*e.g.,* DNA, and/or RNA), polypeptides, polypeptide and polynucleotide modifications (*e.g.,* posttranslational modifications), carbohydrates, and/or glycolipid-based molecular markers.

The "presence," "amount," or "level" of a biomarker associated with an increased clinical benefit to an individual is a detectable level in a biological sample. These can be measured by methods known to one skilled in the art and also disclosed herein. The expression level or amount of biomarker assessed can be used to determine the response to the treatment.

The terms "level of expression" or "expression level" in general are used interchangeably and generally refer to the amount of a biomarker in a biological sample. "Expression" generally refers to the process by which information (*e.g.,* gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications (*e.g.,* posttranslational modification of a polypeptide). Fragments of the transcribed polynucleotide, the translated polypeptide, or polynucleotide and/or polypeptide modifications (*e.g.,* post-translational modification of a polypeptide) shall also be regarded as expressed whether they originate from a transcript generated by alternative splicing or a degraded transcript, or from a post-translational processing of the polypeptide, *e.g.,* by proteolysis. "Expressed genes" include those that are transcribed into a polynucleotide as mRNA and then translated into a polypeptide, and also those that are transcribed into RNA but not translated into a polypeptide (for example, transfer and ribosomal RNAs).

"Elevated expression," "elevated expression levels," or "elevated levels" refers to an increased expression or increased levels of a biomarker in an individual relative to a control, such as an individual or individuals who are not suffering from the disease or disorder (*e.g.,* cancer) or an internal control (*e.g.,* housekeeping biomarker).

"Reduced expression," "reduced expression levels," or "reduced levels" refers to a decrease expression or decreased levels of a biomarker in an individual relative to a control, such as an individual or individuals who are not suffering from the disease or disorder (*e.g.,* cancer) or an internal control (*e.g.,* housekeeping biomarker).

The term "housekeeping biomarker" refers to a biomarker or group of biomarkers *(e.g.,* polynucleotides and/or polypeptides) which are typically similarly present in all cell types. In some embodiments, the housekeeping biomarker is a "housekeeping gene." A "housekeeping gene" refers herein to a gene or group of genes which encode proteins whose activities are essential for the maintenance of cell function and which are typically similarly present in all cell types.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "multiplex-PCR" refers to a single PCR reaction carried out on nucleic acid obtained from a single source (*e.g.,* an individual) using more than one primer set for the purpose of amplifying two or more DNA sequences in a single reaction.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of de*Nature*d DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, *see* Ausubel et al., Current Protocols in Molecular Biology, Wiley InterScience Publishers, (1995).

"Stringent conditions" or "high stringency conditions", as defined herein, can be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) overnight hybridization in a solution that employs 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with a 10 minute wash at 42°C in 0.2 x SSC (sodium chloride/sodium citrate) followed by a 10 minute high-stringency wash consisting of 0.1 x SSC containing EDTA at 55°C.

"Moderately stringent conditions" can be identified as described by Sambrook et al., Molecular Cloning: A Laboratory Manual, New York: Cold Spring Harbor Press, 1989, and include the use of washing solution and hybridization conditions (*e.g.,* temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml de*Nature*d sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The term "diagnosis" is used herein to refer to the identification or classification of a molecular or pathological state, disease or condition (*e.g.,* cancer). For example, "diagnosis" may refer to identification of a particular type of cancer. "Diagnosis" may also refer to the classification of a particular subtype of cancer, *e.g.,* by histopathological criteria, or by molecular features (*e.g.,* a subtype characterized by expression of one or a combination of biomarkers (*e.g.,* particular genes or proteins encoded by said genes)).

The term "aiding diagnosis" is used herein to refer to methods that assist in making a clinical determination regarding the presence, or *Nature,* of a particular type of symptom or condition of a disease or disorder (*e.g.,* cancer). For example, a method of aiding diagnosis of a disease or condition (*e.g.,* cancer) can comprise measuring certain biomarkers in a biological sample from an individual.

The term "sample," as used herein, refers to a composition that is obtained or derived from a subject and/or individual of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof.

By "tissue sample" or "cell sample" is meant a collection of similar cells obtained from a tissue of a subject or individual. The source of the tissue or cell sample may be solid tissue as from a fresh, frozen and/or preserved organ, tissue sample, biopsy, and/or aspirate; blood or any blood constituents such as plasma; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in *Nature* such as preservatives, anticoagulants, buffers, fixatives, nutrients, antibiotics, or the like.

A "reference sample", "reference cell", "reference tissue", "control sample", "control cell", or "control tissue", as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (*e.g.,* tissue or cells) of the same subject or individual. For example, healthy and/or non-diseased cells or tissue adjacent to the diseased cells or tissue (*e.g.,* cells or tissue adjacent to a tumor). In another embodiment, a reference sample is obtained from an untreated tissue and/or cell of the body of the same subject or individual. In yet another embodiment, a reference sample, reference cell, reference tissue, control sample, control cell, or control tissue is obtained from a healthy and/or non-diseased part of the body (*e.g.,* tissues or cells) of an individual who is not the subject or individual. In even another embodiment, a reference sample, reference cell, reference tissue, control sample, control cell, or control tissue is obtained from an untreated tissue and/or cell of the body of an individual who is not the subject or individual.

For the purposes herein a "section" of a tissue sample is meant a single part or piece of a tissue sample, *e.g.,* a thin slice of tissue or cells cut from a tissue sample. It is understood that multiple sections of tissue samples may be taken and subjected to analysis, provided that it is understood that the same section of tissue sample may be analyzed at both morphological and molecular levels, or analyzed with respect to both polypeptides and polynucleotides.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the embodiment of polynucleotide analysis or protocol, one may use the results of the polynucleotide expression analysis or protocol to determine whether a specific therapeutic regimen should be performed.

"Individual response" or "response" can be assessed using any endpoint indicating a benefit to the individual, including, without limitation, (1) inhibition, to some extent, of disease progression (*e.g.,* cancer progression), including slowing down and complete arrest; (2) a reduction in tumor size; (3) inhibition (*i.e.,* reduction, slowing down or complete stopping) of cancer cell infiltration into adjacent peripheral organs and/or tissues; (4) inhibition (*i.e.* reduction, slowing down or complete stopping) of metasisis; (5) relief, to some extent, of one or more symptoms associated with the disease or disorder (*e.g.,* cancer); (6) increase in the length of progression free survival; and/or (9) decreased mortality at a given point of time following treatment.

The phrase "substantially similar," as used herein, refers to a sufficiently high degree of similarity between two numeric values (generally one associated with a molecule and the other associated with a reference/comparator molecule) such that one of skill in the art would consider the difference between the two values to not be of statistical significance within the context of the biological characteristic measured by said values (*e.g.,* Kd values). The difference between said two values may be, for example, less than about 20%, less than about 10%, and/or less than about 5% as a function of the reference/comparator value. The phrase "substantially normal" refers to substantially similar to a reference (*e.g.,* normal reference).

The phrase "substantially different," refers to a sufficiently high degree of difference between two numeric values (generally one associated with a molecule and the other associated with a reference/comparator molecule) such that one of skill in the art would consider the difference between the two values to be of statistical significance within the context of the biological characteristic measured by said values (*e.g.,* Kd values). The difference between said two values may be, for example, greater than about 10%, greater than about 20%, greater than about 30%, greater than about 40%, and/or greater than about 50% as a function of the value for the reference/comparator molecule.

The word "label" when used herein refers to a detectable compound or composition. The label is typically conjugated or fused directly or indirectly to a reagent, such as a polynucleotide probe or an antibody, and facilitates detection of the reagent to which it is conjugated or fused. The label may itself be detectable (*e.g.,* radioisotope labels or fluorescent labels) or, in the case of an enzymatic label, may catalyze chemical alteration of a substrate compound or composition which results in a detectable product.

An "effective amount" of an agent refers to an amount effective, at dosages and for periods of time necessary, to achieve the desired therapeutic or prophylactic result.

A "therapeutically effective amount" of a substance/molecule of the invention, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies are used to delay development of a disease or to slow the progression of a disease.

The terms "cell proliferative disorder" and "proliferative disorder" refer to disorders that are associated with some degree of abnormal cell proliferation. In one embodiment, the cell proliferative disorder is cancer.

The terms "cancer" and "cancerous" refer to or describe the physiological condition in mammals that is typically characterized by unregulated cell growth/proliferation. Examples of cancer include, but are not limited to, carcinoma, lymphoma (e.g., Hodgkin's and non-Hodgkin's lymphoma), blastoma, sarcoma, and leukemia. More particular examples of such cancers include lung cancer, cancer of the peritoneum, hepatocellular cancer, gastrointestinal cancer, pancreatic cancer, glioma, cervical cancer, ovarian cancer, liver cancer, bladder cancer, hepatoma, breast cancer, colon cancer, colorectal cancer, renal cell cancer, endometrial or uterine carcinoma, salivary gland carcinoma, kidney cancer, liver cancer, prostate cancer, vulval cancer, thyroid cancer, hepatic carcinoma, leukemia and other lymphoproliferative disorders, and various types of head and neck cancer.

The term "anti-cancer therapy" refers to a therapy useful in treating cancer. Examples of anti-cancer therapeutic agents include, but are limited to, e.g., chemotherapeutic agents, growth inhibitory agents, cytotoxic agents, agents used in radiation therapy, anti-angiogenesis agents, apoptotic agents, anti-tubulin agents, and other agents to treat cancer , anti-CD20 antibodies, platelet derived growth factor inhibitors (e.g., Gleevec™ (Imatinib Mesylate)), a COX-2 inhibitor (e.g., celecoxib), interferons, cytokines, antagonists (e.g., neutralizing antibodies) that bind to one or more of the following targets PDGFR-beta, BlyS, APRIL, BCMA receptor(s), TRAIL/Apo2, and other bioactive and organic chemical agents, etc. Combinations thereof are also included in the disclosure.

The term "cytotoxic agent" as used herein refers to a substance that inhibits or prevents a cellular function and/or causes cell death or destruction. Cytotoxic agents include, but are not limited to, radioactive isotopes (e.g., $At^{211}$, $I^{131}$, $I^{125}$, $Y^{90}$, $Re^{186}$, $Re^{188}$, $Sm^{153}$, $Bi^{212}$, $P^{32}$, $Pb^{212}$ and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

A "chemotherapeutic agent" refers to a chemical compound useful in the treatment of cancer. Examples of chemotherapeutic agents include alkylating agents such as thiotepa and cyclosphosphamide (CYTOXAN®); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL®); beta-lapachone; lapachol; colchicines; betulinic acid; a camptothecin (including the synthetic analogue topotecan (HYCAMTIN®), CPT-11 (irinotecan, CAMPTOSAR®), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegal1 (see, e.g., Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN®, morpholino-doxorubicin, cyanomorpholino-doxorubicin, 2-pyrrolino-doxorubicin, doxorubicin HCl liposome injection (DOXIL®), liposomal doxorubicin TLC D-99 (MYOCET®), pegylated liposomal doxorubicin (CAELYX®), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR®), tegafur (UFTORAL®), capecitabine (XELODA®), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK® polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE®, FILDESIN®); dacarbazine; mannomustine; mitobronitol; mitolactol; pipobroman; gacytosine; arabinoside ("Ara-C"); thiotepa; taxoid, e.g., paclitaxel (TAXOL®), albumin-engineered nanoparticle formulation of paclitaxel (ABRAXANE™), and docetaxel (TAXOTERE®); chloranbucil; 6-thioguanine; mercaptopurine; methotrexate; platinum agents such as cisplatin, oxaliplatin (e.g., ELOXATIN®), and carboplatin; vincas, which prevent tubulin polymerization from forming microtubules, including vinblastine (VELBAN®), vincristine (ONCOVIN®), vindesine (ELDISINE®, FILDESIN®), and vinorelbine (NAVELBINE®); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN®); bisphosphonates such as clodronate (for example, BONEFOS® or OSTAC®), etidronate (DIDROCAL®), NE-58095, zoledronic acid/zoledronate (ZOMETA®), alendronate (FOSAMAX®), pamidronate (AREDIA®), tiludronate (SKELID®), or risedronate (ACTONEL®); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE® vaccine and gene therapy vaccines, for example, ALLOVECTIN® vaccine, LEUVECTIN® vaccine, and VAXID® vaccine; topoisomerase 1 inhibitor (e.g., LURTOTECAN®); rmRH (e.g., ABARELIX®); BAY439006 (sorafenib; Bayer); SU-11248 (sunitinib, SUTENT®, Pfizer); perifosine, COX-2 inhibitor (e.g., celecoxib or etoricoxib), proteosome inhibitor (e.g., PS341); bortezomib (VELCADE®); CCI-779; tipifarnib (R11577); orafenib, ABT510; Bcl-2 inhibitor such as oblimersen sodium (GENASENSE®); pixantrone; EGFR inhibitors (see definition below); tyrosine kinase inhibitors (see definition below); serine-threonine kinase inhibitors such as rapamycin (sirolimus, RAPAMUNE®); farnesyltransferase inhibitors such as lonafarnib (SCH 6636, SARASAR™); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATIN™) combined with 5-FU and leucovorin.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX®), 4-hydroxytamoxifen, toremifene (FARESTON®), idoxifene, droloxifene, raloxifene (EVISTA®), trioxifene, keoxifene, and selective estrogen receptor modulators (SERMs) such as SERM3; pure anti-estrogens without agonist properties, such as fulvestrant (FASLODEX®), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN®), and nonsteroidal aromatase inhibitors such as anastrazole (ARIMIDEX®), letrozole (FEMARA®) and aminoglutethimide, and other aromatase inhibitors include vorozole (RIVISOR®), megestrol acetate (MEGASE®), fadrozole, and 4(5)-imidazoles; lutenizing hormone-releaseing hormone agonists, including leuprolide (LUPRON® and ELIGARD®), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); antiandrogens such as flutamide, nilutamide and bicalutamide; and pharmaceutically acceptable salts, acids or derivatives of any of the above; as well as combinations of two or more of the above.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy" Biochemical Society Transactions, 14, pp. 375-382, 615th Meeting Belfast (1986) and Stella et al., "Prodrugs: A Chemical Approach to Targeted Drug Delivery," Directed Drug Delivery, Borchardt et al., (ed.), pp. 247-267, Humana Press (1985). The prodrugs include, but are not limited to, phosphate-containing prodrugs, thiophosphate-containing prodrugs, sulfate-containing prodrugs, peptide-containing prodrugs, D-amino acid-modified prodrugs, glycosylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this disclosure include, but are not limited to, those chemotherapeutic agents described above.

A "growth inhibitory agent" when used herein refers to a compound or composition which inhibits growth of a cell (*e.g.,* a cell whose growth is dependent upon an FGF19, FGFR4, and/or klotho (*e.g.,* KLB) gene and/or FGF19, FGFR4, and/or klotho (*e.g.,* KLB) expression either *in vitro* or *in vivo*). Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE®, Rhone-Poulenc Rorer), derived from the European yew, is a semisynthetic analogue of paclitaxel (TAXOL®, Bristol-Myers Squibb). Paclitaxel and docetaxel promote the assembly of microtubules from tubulin dimers and stabilize microtubules by preventing depolymerization, which results in the inhibition of mitosis in cells.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one time administration and typical dosages range from 10 to 200 units (Grays) per day.

An "individual" or "subject" is a mammal. Mammals include, but are not limited to, domesticated animals (*e.g.,* cows, sheep, cats, dogs, and horses), primates (*e.g.,* humans and non-human primates such as monkeys), rabbits, and rodents (*e.g.,* mice and rats). In certain embodiments, the individual or subject is a human.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

By "reduce" or "inhibit" is meant the ability to cause an overall decrease of 20%, 30%, 40%, 50%, 60%, 70%, 75%, 80%, 85%, 90%, 95%, or greater. Reduce or inhibit can refer to the symptoms of the disorder being treated, the presence or size of metastases, or the size of the primary tumor.

The term "package insert" is used to refer to instructions customarily included in commercial packages of therapeutic products, that contain information about the indications, usage, dosage, administration, combination therapy, contraindications and/or warnings concerning the use of such therapeutic products.

An "article of manufacture" is any manufacture (*e.g.,* a package or container) or kit comprising at least one reagent, *e.g.,* a medicament for treatment of a disease or disorder (*e.g.,* cancer), or a probe for specifically detecting a biomarker described herein. In certain embodiments, the manufacture or kit is promoted, distributed, or sold as a unit for performing the methods described herein.

A "target audience" is a group of people or an institution to whom or to which a particular medicament is being promoted or intended to be promoted, as by marketing or advertising, especially for particular uses, treatments, or indications, such as individuals, populations, readers of newspapers, medical literature, and magazines, television or internet viewers, radio or internet listeners, physicians, drug companies, etc.

As is understood by one skilled in the art, reference to "about" a value or parameter herein includes (and describes) embodiments that are directed to that value or parameter per se. For example, description referring to "about X" includes description of "X".

It is understood that aspect and embodiments described herein include "consisting" and/or "consisting essentially of" aspects and embodiments. As used herein, the singular form "a", "an", and "the" includes plural references unless indicated otherwise.

## II. Methods and Uses

Provided herein are methods of assessing levels of one or more bile acid metabolism biomarkers in a sample from an individual (*e.g.,* compared to a reference) and/or treating a disease or disorder in the individual with a FGF19 modulator. Specifically, provided herein are methods for treating a disease or disorder in an individual comprising administering to the individual an effective amount of an FGF19 modulator and assessing levels of one or more bile acid metabolism biomarkers (*e.g.,* compared to a reference) in the individual during treatment with the FGF19 modulator. For example, provided herein are methods for treating cancer in an individual comprising administering to the individual an effective amount of an FGF19 antagonist (*e.g.,* anti-FGF19 antibody) and assessing levels of one or more bile acid metabolism biomarkers (*e.g.,* compared to a reference) in the individual during treatment with the FGF19 antagonist (*e.g.,* anti-FGF19 antibody).

Further provided herein are methods of treating a disease or disorder in an individual comprising administering to the individual an effective amount of an FGF19 modulator, wherein treatment is based upon levels of one or more bile acid metabolism biomarkers (*e.g.,* compared to a reference). For example, provided herein are methods of treating cancer in an individual comprising administering to the individual an effective amount of an FGF19 antagonist (*e.g.,* anti-FGF19 antibody), wherein treatment is based upon levels of one or more bile acid metabolism biomarkers (*e.g.,* compared to a reference).

In addition, provided herein are methods for treating a disease or disorder in an individual, the method comprising: determining levels of one or more bile acid metabolism biomarkers in a sample from the individual (*e.g.,* compared to a reference), and administering an effective amount of an FGF19 modulator to the individual, whereby the disease or disorder is treated. For example, provided herein are methods for treating cancer in an individual, the method comprising: determining the levels of one or more bile acid metabolism biomarkers in a sample from the individual (*e.g.,* compared to a reference), and administering an effective amount of an FGF19 antagonist (*e.g.,* anti-FGF19 antibody) to the individual, whereby the disease or disorder is treated.

Provided herein are methods of treating a disease or disorder, comprising: (a) selecting an individual having the disease or disorder, wherein the individual comprises levels of one or more bile acid metabolism biomarkers (*e.g.,* compared to a reference); and (b) administering to the individual thus selected an effective amount of an FGF19 modulator, whereby the disease or disorder is treated. For example, provided herein are methods of treating cancer, comprising: (a) selecting an individual having cancer, wherein the individual comprises levels of one or more bile acid metabolism biomarkers (*e.g.,* compared to a reference); and (b) administering to the individual thus selected an effective amount of an FGF19 antagonist (*e.g.,* anti-FGF19 antibody), whereby the disease or disorder is treated.

Also provided herein are methods of identifying an individual who is more or less likely to exhibit benefit from treatment comprising an FGF19 modulator, the method comprising: determining levels of one or more bile acid metabolism biomarkers in a sample obtained from the individual. For example, provided herein are methods of identifying an individual who is more or less likely to exhibit benefit from treatment comprising an FGF19 antagonist (*e.g.,* anti-FGF19 antibody), the method comprising: determining levels of one or more bile acid metabolism biomarkers in a sample obtained from the individual. In some embodiments, the method further comprises administering an effective amount of the FGF19 modulator.

Provided herein are methods for predicting whether an individual with a disease or disorder is more or less likely to develop toxicity to treatment comprising an FGF19 modulator, the method comprising determining levels of one or more bile acid metabolism biomarkers. For example, provided herein are methods for predicting whether an individual with cancer is more or less likely to develop toxicity to treatment comprising an FGF19 antagonist (*e.g.,* anti-FGF19 antibody), the method comprising determining levels of one or more bile acid metabolism biomarkers. In some embodiments, the toxicity is diarrhea (*e.g.,* sever diarrhea), dehydration, low food consumption, decreased body weight, and/or morbidity. In some embodiments, the method further comprises administering an effective amount of the FGF19 modulator.

Also provided herein are methods of determining whether an individual with a disease or disorder should continue or discontinue treatment comprising an FGF19 modulator, the method comprising measuring in a sample obtained from the individual levels of one or more bile acid metabolism biomarkers, wherein levels of one or more bile acid metabolism biomarkers (*e.g.,* compared to a reference) determines the individual should continue or discontinue treatment comprising the FGF19 modulator. For example, provided herein are methods of determining whether an individual with cancer should continue or discontinue treatment comprising an FGF19 antagonist (*e.g.,* anti-FGF19 antibody), the method comprising measuring in a sample obtained from the individual levels of one or more bile acid metabolism biomarkers, wherein levels of one or more bile acid metabolism biomarkers (*e.g.,* compared to a reference) determines the individual should continue or discontinue treatment comprising the FGF19 modulator. In some embodiments, the method further comprises administering an effective amount of the FGF19 modulator.

In addition, provided herein are methods of identifying an individual as more likely suitable or less likely suitable to continue a treatment, wherein treatment comprises an FGF19 modulator, based upon levels of one or more bile acid metabolism biomarkers in a sample. For example, provided herein are methods of identifying an individual as more likely suitable or less likely suitable to continue a treatment, wherein treatment comprises an FGF19 antagonist (*e.g.,* anti-FGF19 antibody), based upon levels of one or more bile acid metabolism biomarkers in a sample. In some embodiments, the method further comprises administering an effective amount of the FGF19 modulator.

Provided herein are methods of optimizing therapeutic efficacy and/or reducing toxicity associated with a treatment of a disease or disorder in an individual having the disease or disorder undergoing the treatment, comprising: determining the levels of one or more bile acid metabolism biomarkers in the individual. For example, provided herein are methods of optimizing therapeutic efficacy and/or reducing toxicity associated with a treatment of cancer in an individual having the disease or disorder undergoing the treatment, comprising: determining the levels of one or more bile acid metabolism biomarkers in the individual. In some embodiments, the method further comprises administering an effective amount of the FGF19 modulator.

Further provided herein are methods of identifying an individual having a disease or disorder as more likely suitable or less likely suitable to continue a dose and/or dosage schedule of a treatment, wherein treatment comprises an FGF19 modulator, based upon levels of one or more bile acid metabolism biomarkers in a sample. For example, provided herein are methods of identifying an individual having cancer as more likely suitable or less likely suitable to continue a dose and/or dosage schedule of a treatment, wherein treatment comprises an FGF19 antagonist (*e.g.,* anti-FGF19 antibody), based upon levels of one or more bile acid metabolism biomarkers in a sample. In some embodiments, the method further comprises administering an effective amount of the FGF19 modulator.

Provided herein are assay methods for optimizing dose efficacy in an individual receiving an FGF19 modulator, the method comprising: (a) determining levels of one or more bile acid metabolism biomarkers in a sample from the individual; (b) recommending a subsequent dose of the FGF19 modulator based upon the level of the bile acid metabolism biomarker. For example, provided are assay methods for optimizing dose efficacy in an individual receiving an FGF19 antagonist (*e.g.,* anti-FGF19 antibody), the method comprising: (a) determining levels of one or more bile acid metabolism biomarkers in a sample from the individual; (b) recommending a subsequent dose of the FGF19 antagonist (*e.g.,* anti-FGF19 antibody) based upon the level of the bile acid metabolism biomarker. In some embodiments, the method further comprises administering an effective amount of the FGF19 modulator.

In another aspect, provided are assay methods for evaluating the risk that an individual will develop toxicity to an FGF19 modulator, the method comprising: (a) determining levels of one or more bile acid metabolism biomarkers in a sample from the individual; (b) evaluating the risk that the individual will develop toxicity to the FGF19 modulator based upon the levels of one or more bile acid metabolism biomarkers. For example, provided are assay methods for evaluating the risk that an individual will develop toxicity to an FGF19 antagonist (*e.g.,* anti-FGF19 antibody), the method comprising: (a) determining levels of one or more bile acid metabolism biomarkers in a sample from the individual; (b) evaluating the risk that the individual will develop toxicity to the FGF19 antagonist (*e.g.,* anti-FGF19 antibody) based upon the levels of one or more bile acid metabolism biomarkers. In some embodiments, the method further comprises administering an effective amount of the FGF19 modulator.

In some embodiments of any of the methods, reduced levels and/or substantially normal levels of one or more bile acid metabolism biomarkers (*e.g.,* compared to a reference) in the sample from the individual indicates and/or determines that the individual is more likely to exhibit benefit from treatment comprising the FGF19 modulator, the individual is less likely to develop toxicity to treatment comprising the FGF19 modulator, the individual should continue treatment comprising the FGF19 modulator, the individual more likely suitable to continue the treatment comprising the FGF19 modulator, and/or the individual more likely suitable to continue the dose and/or dosage schedule of the treatment comprising the FGF19 modulator.

In some embodiments of any of the methods, elevated levels of one or more bile acid metabolism biomarkers (*e.g.,* compared to a reference) indicates and/or that the individual is less likely to exhibit benefit from treatment comprising the FGF19 modulator, the individual is more likely to develop toxicity to treatment comprising the FGF19 modulator, the individual should discontinue treatment comprising the FGF19 modulator, the individual less likely suitable to continue the treatment comprising the FGF19 modulator, a need to decrease the amount and/or frequency of subsequently administered of the FGF19 modulator to the individual, and/or he individual less likely suitable to continue the dose and/or dosage schedule of the treatment comprising the FGF19 modulator.

In some embodiments of any of the methods, the bile acid metabolism biomarker is a liver enzyme. In some embodiments, the liver enzyme is a serum liver enzyme.

In some embodiments of any of the methods, the bile acid metabolism biomarker is a bile acid. In some embodiments, the bile acid is total bile acid. In some embodiments, the bile acid is a hydrophobic bile acid. In some embodiments, the bile acid is a primary bile acid. In some embodiments, the primary bile acid is cholic acid ($3\alpha$-$7\alpha$, $12\alpha$-trihydroxy-$5\beta$ cholanic acid), chenodeoxycholic acid ($3\alpha$, $7\alpha$-dihydroxy-$5\beta$-cholanic acid), and/or conjugates forms thereof. In some embodiments, the primary bile acid is glycocholic acid, and/or taurocholic acid. In some embodiments, the bile acid is a secondary bile acid. In some embodiments, the secondary bile acid is deoxycholic acid (3β, 12α-dihydroxy-5β-cholanic acid), lithocholic acid (3α-hydroxy-5β-cholanic acid, and/or conjugate forms thereof (*e.g.,* glycine and/or taurine). In some embodiments, the secondary bile acid is 3α, 12α-hydroxy-5β-cholanoic acid, iso-lithocholic acid, and/or 12-oxo-3α-hydroxy-5β-cholanoic acid. In some embodiments, the secondary bile acid is lithocholic acid. In some embodiments, the secondary bile acid is deoxycholic acid. In some embodiments, the bile acid is a tertiary bile acid. In some embodiments, the tertiary bile acid is ursodexycholic acid (3a,7β-dioxycholanic acid) and/or conjugate forms thereof (*e.g.,* glycine and/or taurine).

In some embodiments of any of the methods, the bile acid metabolism biomarker is aspartate aminotransferase (AST), alanine transaminase (ALT), gamma glutamyl transpeptidase (GGT), alpha-L-fucosidase (AFU), adenosine deaminase (ADA), cholinesterase activity (CHE), alpha-fetoprotein (AFP), and/or total bilirubin levels (TBIL).

In some embodiments of any of the methods, the bile acid metabolism biomarker is cytochrome P450, subfamily VIIA (cholesterol 7 alpha-monooxygenase), polypeptide 1 (Cyp7α1), bile salt export pump (BSEP), multidrug resistance-associated protein 2 (MRP2/ABCC2), multidrug resistance-associated protein 3 (MRP3/ABCC3), organic solute transporter alpha (Ost-α), organic solute transporter alpha (Ost-β), and/or illeal bile acid binding protein (IBABP). In some embodiments, the bile acid metabolism biomarker is Cyp7α1.

In some embodiments of any of the methods, the bile acid metabolism biomarker is hepatocellular injury and/or dysfunction. In some embodiment of any of the methods, the bile acid metabolism biomarker is a hepatocellular single cell necrosis.

In some embodiments of any of the methods, substantially normal levels of one or more bile acid metabolism biomarkers are levels between about any of 36-38% for cholic acid, 32-34% for chenodeoxycholic acid, 26-28% for deoxycholic acid, and/or 1-2% for lithocholic acid and/or less than 1% for ursodexycholic acid of total bile acid. In some embodiments of any of the methods, reduced levels of one or more bile acid metabolism biomarkers are levels less (*e.g.,* significantly less) than about any of 36% for cholic acid, 32% for chenodeoxycholic acid, 26% for deoxycholic acid, and/or 1% for lithocholic acid of total bile acid. In some embodiments of any of the methods, elevated levels of one or more bile acid metabolism biomarkers are levels greater (*e.g.,* significantly greater) than about any of 38% for cholic acid, 34% for chenodeoxycholic acid, 28% for deoxycholic acid, 2% for lithocholic acid, and/or 1% for ursodexycholic acid of total bile acid.

In some embodiments of any of the methods, substantially normal levels of one or more bile acid metabolism biomarkers is between about any of 2-10 μmol/L of bile acid in serum (*e.g.,* peripheral serum), 0.2-0.5 g/d of bile acid in feces, and/or 8 μmol/L of bile acid in urine. In some embodiments of any of the methods, elevated levels of one or more bile acid metabolism biomarkers is greater than about any of 12, 25, 50, 75, 100, 125, 150, 175, 200, 225, 250, 275 or 300 μmol/L of bile acid in serum (*e.g.,* peripheral serum). In some embodiments of any of the methods, elevated levels of one or more bile acid metabolism biomarkers is between about 12 to about 300 μmol/L and/or about 100 to about 300 μmol/L of bile acid in serum (*e.g.,* peripheral serum). In some embodiments of any of the methods, elevated levels of one or more bile acid metabolism biomarkers is greater than about any of 10, 20, 30, 40, 50, 60, 70, 80, 90, 100, 110, or 120 μmol/L of bile acid in urine. In some embodiments of any of the methods, elevated levels of one or more bile acid metabolism biomarkers is about 120 μmol/L. In some embodiments, the levels are measured as fasting levels. In some embodiments, the levels are measured as postprandial levels.

In some embodiments of any of the methods, elevated levels of one or more bile acid metabolism biomarkers (*e.g.,* compared to a reference) in the sample from the individual indicates and/or determines that the individual is more likely to exhibit benefit from treatment comprising the FGF19 modulator, the individual is less likely to develop toxicity to treatment comprising the FGF19 modulator, the individual should continue treatment comprising the FGF19 modulator, the individual more likely suitable to continue the treatment comprising the FGF19 modulator, and/or the individual more likely suitable to continue the dose and/or dosage schedule of the treatment comprising the FGF19 modulator.

In some embodiments of any of the methods, reduced levels and/or substantially normal levels of one or more bile acid metabolism biomarkers (*e.g.,* compared to a reference) indicates and/or that the individual is less likely to exhibit benefit from treatment comprising the FGF19 modulator, the individual is more likely to develop toxicity to treatment comprising the FGF19 modulator, the individual should discontinue treatment comprising the FGF19 modulator, the individual less likely suitable to continue the treatment comprising the FGF19 modulator, a need to decrease the amount and/or frequency of subsequently administered of the FGF19 modulator to the individual, and/or he individual less likely suitable to continue the dose and/or dosage schedule of the treatment comprising the FGF19 modulator.

In some embodiments of any of the methods, the bile acid metabolism biomarker is sodium-taurocholate cotransporting polypeptide (NTCP), an organic anion transporting polypeptide, organic anion transporter 2 (OAT2) and multidrug resistance-associated protein 4 (MRP4/ABCC4), biliary excretion index (BEI), apical sodium dependent bile acid transporter (ASBT/SLC10A2), bile acid absorption, and/or taurocholate uptake.

In some embodiments of any of the methods, elevated expression levels and/or levels refers to an overall increase of greater than about and/or about any of 5%, 10%, 20%, 25%, 30%, 40%, 50%, 60%, 70%, 80%, 85%, 90%, 95%, 96%, 97%, 98%, 99% or greater, in the level of bile acid metabolism biomarker (*e.g.,* metabolite, protein, and/or nucleic acid (*e.g.,* gene or mRNA)), detected by standard art known methods such as those described herein, as compared to a reference sample, reference cell, reference tissue, control sample, control cell, or control tissue. In certain embodiments, the elevated expression levels and/or levels refers to the increase in expression level and/or levels of one or more bile acid metabolism biomarkers in the sample wherein the increase is at least about any of 1.5X, 1.75X, 2X, 3X, 4X, 5X, 6X, 7X, 8X, 9X, 10X, 25X, 50X, 75X, or 100X the expression level and/or level of the respective bile acid metabolism biomarker in a reference sample, reference cell, reference tissue, control sample, control cell, or control tissue. In some embodiments, elevated expression levels and/or levels of one or more bile acid metabolism biomarkers refers to an overall increase of greater than about and/or about any of 1.1-fold, 1.5-fold, 2-fold, 2.5-fold, 3-fold, 4-fold, 5-fold, 10-fold, 12-fold, 15-fold, 17- fold, about 20-fold, 25-fold, and/or 30-fold as compared to a reference sample, reference cell, reference tissue, control sample, control cell, control tissue, or internal control (*e.g.,* housekeeping gene).

In some embodiments of any of the methods, reduced expression levels and/or levels refers to an overall reduction of greater than about and/or about any of 5%, 8%, 10%, 20%, 25%, 30%, 35% 40%, 50%, 60%, 64% 70%, 80%, 85%, 90%, 95%, 96%, 97%, 98%, 99% or greater, in the level of bile acid metabolism biomarker (*e.g.,* metabolite, protein, and/or nucleic acid (*e.g.,* gene or mRNA)), detected by standard art known methods such as those described herein, as compared to a reference sample, reference cell, reference tissue, control sample, control cell, or control tissue. In certain embodiments, the reduced expression levels and/or levels refers to the decrease in expression level and/or levels of one or more bile acid metabolism biomarkers in the sample wherein the decrease is at least about any of 1.5X, 1.75X, 2X, 3X, 4X, 5X, 6X, 7X, 8X, 9X, 10X, 25X, 50X, 75X, or 100X the expression level and/or level of the respective bile acid metabolism biomarker in a reference sample, reference cell, reference tissue, control sample, control cell, or control tissue. In certain embodiments, reduced expression levels and/or levels refers to the decrease in expression level and/or levels of one or more bile acid metabolism biomarkers in the sample wherein the decrease is at least about and/or about any of 0.9X, 0.8X, 0.7X, 0.6X, 0.5X, 0.4X, 0.3X, 0.2X, 0.1X, 0.05X, or 0.01X the expression level/level of the respective biomarker in a reference sample, reference cell, reference tissue, control sample, control cell, or control tissue.

In some embodiments of any of the methods, the reference is an average level of the one or more bile acid metabolism biomarker of healthy individual and/or healthy population of individuals (*e.g.,* wherein the level is measure by a similar and/or same method). In some embodiments of any of the methods, the reference is an average level of the one or more bile acid metabolism biomarker of a second individual having the disease or disorder and/or population of individuals having the disease or disorder (*e.g.,* wherein the level is measure by a similar and/or same method). In some embodiments of any of the methods, the reference is the level of the one or more bile acid metabolism biomarker of the individual having the disease or disorder prior to starting treatment and/or at the time of starting treatment comprising the FGF19 modulator. In some embodiments of any of the methods, the reference is the level of the one or more bile acid metabolism biomarker of the individual having the disease or disorder at a time-point during treatment comprising the FGF19 modulator.

In some embodiments of any of the methods, the disease or disorder is a proliferative disease or disorder. In some embodiments, the proliferative disease or disorder is cancer. Examples of cancers and cancer cells include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include squamous cell cancer (*e.g.,* epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, testicular cancer, esophageal cancer, tumors of the biliary tract, as well as head and neck cancer. In some embodiments, the cancer is colorectal cancer. In some embodiments, the cancer is renal cell carcinoma. In some embodiments, the cancer is hepatocellular carcinoma. In some embodiments, the cancer is metastatic cancer.

Presence and/or expression levels/levels of one or more bile acid metabolism biomarkers can be determined qualitatively and/or quantitatively based on any suitable criterion known in the art, including but not limited to metabolite, DNA, mRNA, cDNA, proteins, protein fragments and/or gene copy number. In certain embodiments, presence and/or expression/amount of a bile acid metabolism biomarker in a first sample is increased as compared to presence and/or expression/amount in a second sample. In certain embodiments, presence and/or expression/amount of a bile acid metabolism biomarker in a first sample is decreased as compared to presence and/or expression/amount in a second sample. In certain embodiments, the second sample is a reference sample, reference cell, reference tissue, control sample, control cell, or control tissue. Additional disclosures for determining presence and/or expression level/level of a gene are described herein.

Presence and/or expression level and/or levels of various bile acid metabolism biomarker in a sample can be analyzed by a number of methodologies, many of which are known in the art and understood by the skilled artisan, including, but not limited to, immunohistochemical ("IHC"), Western blot analysis, immunoprecipitation, molecular binding assays, ELISA, ELIFA, fluorescence activated cell sorting ("FACS"), MassARRAY, proteomics, quantitative blood based assays (as for example Serum ELISA), biochemical enzymatic activity assays, in situ hybridization, Northern analysis, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), gas-liquid chromatography, high performance liquid chromatography, mass spectrometry, enzymatic assays as well as any one of the wide variety of assays that can be performed by protein, gene, and/or tissue array analysis. Typical protocols for evaluating the status of genes and gene products are found, for example in Ausubel et al. eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used. In some embodiments, expression is measured by gas-liquid chromatography, high performance liquid chromatography, mass spectrometry (*e.g.,* GS-MS) and/or enzymatic assays

Methods for evaluation of bile acids and/or bile acid uptake and efflux are well known in the art and described in the examples. Bile acid uptake and efflux can be evaluated using $d_8$-taurocholate as a probe substrate using B-Clear Technology™ (Qualyst Inc., NC). The total mass of endogenous bile acids (taurocholate, TCA: glycocholate, GCA; taurochenodeoxycholate, TCDCA, glycochenodeoxycholate, GCDCA) can be determined in calcium free buffer and the total mass of compound taken up and excreted can be determined in calcium-containing buffer using LC/MS/MS and quantified against standard curves prepared with stable isotope equivalents ($d_8$-TCA, $d_4$-GCA, $d_4$-TCDCA, or $d_4$-GCDCA). Biliary excretion index and in vitro biliary clearance can be calculated as described previously in Liu X. et al. 1999. Drug Metab Dispos 27(6):637-44.

The enzymatic TBA assay method uses 3-$\alpha$-hydroxysteroid dehydrogenase to catalyze the oxidation reaction convering 3-$\alpha$hydroxyl group of all bile acids to 3-keto group with concomitant formation of a co-enzyme NADH from NAD+. The NADH is further reacted with nitrotetrazolium blue which can be monitored by measure absorbance at 540nm, which is proportional to the bile acid concentration in the serum. In the enzyme cycling based TBA assay, serum bile acid molecules are repeatedly oxidized and reduced by 3-$\alpha$-hydroxysteroid dehydrogenase with a concomitant accumulation of reduced co-enzyme thio-NADH which is detected at 405nm.

Methods for the evaluation of mRNAs in cells are well known and include, for example, hybridization assays using complementary DNA probes (such as in situ hybridization using labeled riboprobes specific for the one or more genes, Northern blot and related techniques) and various nucleic acid amplification assays (such as RT-PCR using complementary primers specific for one or more of the genes, and other amplification type detection methods, such as, for example, branched DNA, SISBA, TMA and the like).

Samples from mammals can be conveniently assayed for mRNAs using Northern, dot blot or PCR analysis. In addition, such methods can include one or more steps that allow one to determine the levels of target mRNA in a biological sample (*e.g.,* by simultaneously examining the levels a comparative control mRNA sequence of a "housekeeping" gene such as an actin family member). Optionally, the sequence of the amplified target cDNA can be determined.

Optional methods include protocols which examine or detect mRNAs, such as target mRNAs, in a tissue or cell sample by microarray technologies. Using nucleic acid microarrays, test and control mRNA samples from test and control tissue samples are reverse transcribed and labeled to generate cDNA probes. The probes are then hybridized to an array of nucleic acids immobilized on a solid support. The array is configured such that the sequence and position of each member of the array is known. For example, a selection of genes whose expression correlates with increased or reduced clinical benefit of anti-angiogenic therapy may be arrayed on a solid support. Hybridization of a labeled probe with a particular array member indicates that the sample from which the probe was derived expresses that gene.

According in some embodiments, presence and/or expression level/level is measured by observing protein expression levels of an aforementioned gene. In certain embodiments, the method comprises contacting the biological sample with antibodies to a bile acid metabolism biomarker described herein under conditions permissive for binding of the biomarker, and detecting whether a complex is formed between the antibodies and biomarker. Such method may be an in vitro or in vivo method. In one embodiment, an antibody is used to select subjects eligible for therapy with an FGF19 modulator, in particular an anti-FGF19 antibody, *e.g.*, a bile acid metabolism biomarker for selection of patients.

In certain embodiments, the presence and/or expression level/level of bile acid metabolism biomarker proteins in a sample is examined using IHC and staining protocols. IHC staining of tissue sections has been shown to be a reliable method of determining or detecting presence of proteins in a sample. In one aspect, expression level and/or levels of one or more bile acid metabolism biomarkers is determined using a method comprising: (a) performing IHC analysis of a sample (such as a patient cancer sample) with an antibody; and b) determining expression levels of one or more bile acid metabolism biomarkers in the sample. In some embodiments, IHC staining intensity is determined relative to a reference value.

IHC may be performed in combination with additional techniques such as morphological staining and/or fluorescence in-situ hybridization. Two general methods of IHC are available; direct and indirect assays. According to the first assay, binding of antibody to the target antigen is determined directly. This direct assay uses a labeled reagent, such as a fluorescent tag or an enzyme-labeled primary antibody, which can be visualized without further antibody interaction. In a typical indirect assay, unconjugated primary antibody binds to the antigen and then a labeled secondary antibody binds to the primary antibody. Where the secondary antibody is conjugated to an enzymatic label, a chromogenic or fluorogenic substrate is added to provide visualization of the antigen. Signal amplification occurs because several secondary antibodies may react with different epitopes on the primary antibody.

The primary and/or secondary antibody used for IHC typically will be labeled with a detectable moiety. Numerous labels are available which can be generally grouped into the following categories: (a) Radioisotopes, such as $^{35}$S, $^{14}$C, $^{125}$I, $^{3}$H, and $^{131}$I; (b) colloidal gold particles; (c) fluorescent labels including, but are not limited to, rare earth chelates (europium chelates), Texas Red, rhodamine, fluorescein, dansyl, Lissamine, umbelliferone, phycocrytherin, phycocyanin, or commercially available fluorophores such SPECTRUM ORANGE7 and SPECTRUM GREEN7 and/or derivatives of any one or more of the above; (d) various enzyme-substrate labels are available and U.S. Patent No. 4,275,149 provides a review of some of these. Examples of enzymatic labels include luciferases (*e.g.,* firefly luciferase and bacterial luciferase; U.S. Patent No. 4,737,456), luciferin, 2,3-dihydrophthalazinediones, malate dehydrogenase, urease, peroxidase such as horseradish peroxidase (HRPO), alkaline phosphatase, β-galactosidase, glucoamylase, lysozyme, saccharide oxidases (*e.g.,* glucose oxidase, galactose oxidase, and glucose-6-phosphate dehydrogenase), heterocyclic oxidases (such as uricase and xanthine oxidase), lactoperoxidase, microperoxidase, and the like.

Examples of enzyme-substrate combinations include, for example, horseradish peroxidase (HRPO) with hydrogen peroxidase as a substrate; alkaline phosphatase (AP) with para-Nitrophenyl phosphate as chromogenic substrate; and β-D-galactosidase (β-D-Gal) with a chromogenic substrate (*e.g.,* p-nitrophenyl-β-D-galactosidase) or fluorogenic substrate (*e.g.,* 4-methylumbelliferyl-β-D-galactosidase). For a general review of these, *see* U.S. Patent Nos. 4,275,149 and 4,318,980.

Specimens thus prepared may be mounted and coverslipped. Slide evaluation is then determined, *e.g.,* using a microscope, and staining intensity criteria, routinely used in the art, may be employed. In some embodiments, a staining pattern score of about 1+ or higher is diagnostic and/or prognostic. In certain embodiments, a staining pattern score of about 2+ or higher in an IHC assay is diagnostic and/or prognostic. In other embodiments, a staining pattern score of about 3 or higher is diagnostic and/or prognostic. In one embodiment, it is understood that when cells and/or tissue from a tumor or colon adenoma are examined using IHC, staining is generally determined or assessed in tumor cell and/or tissue (as opposed to stromal or surrounding tissue that may be present in the sample).

In alternative methods, the sample may be contacted with an antibody specific for said bile acid metabolism biomarker under conditions sufficient for an antibody-biomarker complex to form, and then detecting said complex. The presence of the bile acid metabolism biomarker may be detected in a number of ways, such as by Western blotting and ELISA procedures for assaying a wide variety of tissues and samples, including plasma or serum. A wide range of immunoassay techniques using such an assay format are available, *see, e.g.,* U.S. Pat. Nos. 4,016,043, 4,424,279 and 4,018,653. These include both single-site and two-site or "sandwich" assays of the non-competitive types, as well as in the traditional competitive binding assays. These assays also include direct binding of a labeled antibody to a target bile acid metabolism biomarker.

Presence and/or expression level/level of a selected bile acid metabolism biomarker in a sample may also be examined by way of functional or activity-based assays. For instance, if the bile acid metabolism biomarker is an enzyme, one may conduct assays known in the art to determine or detect the presence of the given enzymatic activity in the sample.

In certain embodiments, the samples are normalized for both differences in the amount of the biomarker assayed and variability in the quality of the samples used, and variability between assay runs. Such normalization may be accomplished by measuring and incorporating the expression of certain normalizing biomarkers, including well known housekeeping genes, such as ACTB. Alternatively, normalization can be based on the mean or median signal of all of the assayed genes or a large subset thereof (global normalization approach). On a gene-by-gene basis, measured normalized amount of a patient tumor mRNA or protein is compared to the amount found in a reference set. Normalized expression levels for each mRNA or protein per tested tumor per patient can be expressed as a percentage of the expression level measured in the reference set. The presence and/or expression level/level measured in a particular patient sample to be analyzed will fall at some percentile within this range, which can be determined by methods well known in the art.

In certain embodiments, relative expression level of a gene is determined as follows:

$$\text{Relative expression gene1 sample1} = 2 \exp (\text{Ct housekeeping gene} - \text{Ct gene1}) \text{ with Ct}$$

determined in a sample.

$$\text{Relative expression gene1 reference RNA} = 2 \exp (\text{Ct housekeeping gene} - \text{Ct gene1}) \text{ with Ct}$$

determined in the reference sample.

$$\text{Normalized relative expression gene1 sample1} = (\text{relative expression gene1 sample1} / \text{relative}$$

expression gene1 reference RNA) x 100

Ct is the threshold cycle. The Ct is the cycle number at which the fluorescence generated within a reaction crosses the threshold line.

All experiments are normalized to a reference RNA, which is a comprehensive mix of RNA from various tissue sources (*e.g.,* reference RNA #636538 from Clontech, Mountain View, CA). Identical reference RNA is included in each qRT-PCR run, allowing comparison of results between different experimental runs.

In one embodiment, the sample is a clinical sample. In another embodiment, the sample is used in a diagnostic assay. In some embodiments, the sample is obtained from a primary or metastatic tumor. Tissue biopsy is often used to obtain a representative piece of tumor tissue. Alternatively, tumor cells can be obtained indirectly in the form of tissues or fluids that are known or thought to contain the tumor cells of interest. For instance, samples of lung cancer lesions may be obtained by resection, bronchoscopy, fine needle aspiration, bronchial brushings, or from sputum, pleural fluid or blood. Genes or gene products can be detected from cancer or tumor tissue or from other body samples such as urine, fecal samples, ileum tissue, liver tissue, sputum, serum or plasma. In some embodiments, the bile acid metabolism biomarker is detected from a fecal sample. In some embodiments, the bile acid metabolism biomarker is detected from a serum sample. In some embodiments, the sample is a tissue sample (*e.g.,* liver tissue sample and/or ileum tissue sample). The same techniques discussed above for detection of target genes or gene products in cancerous samples can be applied to other body samples. Cancer cells may be sloughed off from cancer lesions and appear in such body samples. By screening such body samples, a simple early diagnosis can be achieved for these cancers. In addition, the progress of therapy can be monitored more easily by testing such body samples for target genes or gene products.

In certain embodiments, a reference sample, reference cell, reference tissue, control sample, control cell, or control tissue is a single sample or combined multiple samples from the same subject or individual that are obtained at one or more different time points than when the test sample is obtained. For example, a reference sample, reference cell, reference tissue, control sample, control cell, or control tissue is obtained at an earlier time point from the same subject or individual than when the test sample is obtained. Such reference sample, reference cell, reference tissue, control sample, control cell, or control tissue may be useful if the reference sample is obtained during initial diagnosis of cancer and the test sample is later obtained when the cancer becomes metastatic.

In certain embodiments, a reference sample, reference cell, reference tissue, control sample, control cell, or control tissue is a combined multiple samples from one or more healthy individuals who are not the subject or patient. In certain embodiments, a reference sample, reference cell, reference tissue, control sample, control cell, or control tissue is a combined multiple samples from one or more individuals with a disease or disorder (*e.g.,* cancer) who are not the subject or patient. In certain embodiments, a reference sample, reference cell, reference tissue, control sample, control cell, or control tissue is pooled RNA samples from normal tissues or pooled plasma or serum samples from one or more individuals who are not the subject or patient. In certain embodiments, a reference sample, reference cell, reference tissue, control sample, control cell, or control tissue is pooled RNA samples from tumor tissues or pooled plasma or serum samples from one or more individuals with a disease or disorder (*e.g.,* cancer) who are not the subject or patient.

In some embodiments of any of the methods, the FGF19 modulator is an FGF19 antagonist. In some embodiments, the FGF19 antagonist is an antibody, binding polypeptide, binding small molecule, or polynucleotide. In some embodiments, the FGF19 antagonist is an antibody. In some embodiments, the antibody is a monoclonal antibody. In some embodiments, the antibody is a human, humanized, or chimeric antibody. In some embodiments, the antibody is an antibody fragment and the antibody fragment binds FGF19.

In some embodiments of any of the methods, the individual according to any of the above embodiments may be a human. In some embodiments, the human is a female. In some embodiments, the human is a male.

In some embodiments of any of the methods, the method comprises administering to an individual having such cancer an effective amount of an FGF19 modulator in particular FGF19 antagonist. In one such embodiment, the method further comprises administering to the individual an effective amount of at least one additional therapeutic agent, as described below. In some embodiments, the individual may be a human.

The FGF19 modulator, in particular FGF19 antagonist, described herein can be used either alone or in combination with other agents in a therapy. For instance, an FGF19 modulator, in particular FGF19 antagonist, described herein may be co-administered with at least one additional therapeutic agent including another FGF19 modulator. In certain embodiments, an additional therapeutic agent is a chemotherapeutic agent.

Such combination therapies noted above encompass combined administration (where two or more therapeutic agents are included in the same or separate formulations), and separate administration, in which case, administration of the FGF19 modulator, in particular FGF19 antagonist, can occur prior to, simultaneously, and/or following, administration of the additional therapeutic agent and/or adjuvant. FGF19 modulator, in particular FGF19 antagonist, can also be used in combination with radiation therapy.

An FGF19 modulator, in particular FGF19 antagonist (*e.g.,* an antibody, binding polypeptide, and/or small molecule) described herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, *e.g.,* by injections, such as intravenous or subcutaneous injections, depending in part on whether the administration is brief or chronic. Various dosing schedules including but not limited to single or multiple administrations over various time-points, bolus administration, and pulse infusion are contemplated herein.

FGF19 modulator, in particular FGF19 antagonist (*e.g.,* an antibody, binding polypeptide, and/or small molecule) described herein may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The FGF19 modulator, in particular FGF19 antagonist, need not be, but is optionally formulated with one or more agents currently used to prevent or treat the disorder in question. The effective amount of such other agents depends on the amount of the FGF19 modulator, in particular FGF19 antagonist, present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an FGF19 modulator, in particular FGF19 antagonist, described herein (when used alone or in combination with one or more other additional therapeutic agents) will depend on the type of disease to be treated, the severity and course of the disease, whether the FGF19 modulator, in particular FGF19 antagonist, is administered for preventive or therapeutic purposes, previous therapy, the patient's clinical history and response to the FGF19 modulator, and the discretion of the attending physician. The FGF19 modulator, in particular FGF19 antagonist, is suitably administered to the patient at one time or over a series of treatments. One typical daily dosage might range from about 1 $\mu$g/kg to 100 mg/kg or more, depending on the factors mentioned above. In some embodiments, the dosage of the FGF19 modulator (*e.g.,* FGF19 antagonist, *e.g.,* anti-FGF19 antibody) is greater than and/or greater than or equal to about any of 3, 10, 20, 30, 40, 50, 60, 70, 80, 90, and/or 100 mg/kg. In some some embodiments, the dosage of the FGF19 modulator (e.g., FGF19 antagonist, *e.g.*, anti-FGF19 antibody) is between about any of 3-100 mg/kg, 10-100 mg/kg, 10-30 mg/kg, 30-100 mg/kg, and/or 3-30 mg/kg. In some some embodiments, the dosage of the FGF19 modulator (e.g., FGF19 antagonist, *e.g.*, anti-FGF19 antibody) is about 3 mg/kg, about 10 mg/kg, about 30 mg/kg, and/or about 100 mg/kg. In some embodiments, the dosage of the FGF19 modulator (*e.g.,* FGF19 antagonist, *e.g.,* anti-FGF19 antibody) is greater than and/or greater than or equal to about any of 0.5, 1, 1.5, 5, 10, 25, 50, 75, and/or 100 $\mu$g/mL. In some embodiments, the dosage of the FGF19 modulator (*e.g.,* FGF19 antagonist, *e.g.*, anti-FGF19 antibody) is between about any of 1.56 - 100 $\mu$g/mL and/or 500 ng/ml-100 $\mu$g/mL.

For repeated administrations over several days or longer, depending on the condition, the treatment would generally be sustained until a desired suppression of disease symptoms occurs. Such doses may be administered intermittently, *e.g.,* every week or every three weeks (*e.g.,* such that the patient receives from about two to about twenty, or *e.g.,* about six doses of the FGF19 modulator). An initial higher loading dose, followed by one or more lower doses may be administered. An exemplary dosing regimen comprises administering. However, other dosage regimens may be useful. The progress of this therapy is easily monitored by conventional techniques and assays.

It is understood that any of the above formulations or therapeutic methods may be carried out using an immunoconjugate in place of or in addition to the FGF19 modulator, in particular FGF19 antagonist.

### III. Therapeutic Compositions

Provided herein are FGF19 modulators useful in the methods described herein. In some embodiments, the FGF19 modulators are an antibody, binding polypeptide, binding small molecule, and/or polynucleotide. In some embodiments, the FGF19 modulators are FGF19 antagonists.

### A. Antibodies

In one aspect, provided herein isolated antibodies that bind to an FGF19, klotho (*e.g.,* KLB), and/or FGFR4 polypeptide for use in the methods described herein. In any of the above embodiments, an antibody is humanized. In a further aspect of the disclosure, an anti-FGF19 antibody, anti-klotho antibody (e.g., anti-KLB antibody), and/or anti-FGFR4 antibody according to any of the above embodiments is a monoclonal antibody, including a chimeric, humanized or human antibody. In one embodiment, an anti-FGF19 antibody, anti-klotho antibody (e.g., anti-KLB antibody), and/or anti-FGFR4 antibody is an antibody fragment, e.g., an Fv, Fab, Fab', scFv, diabody, or F(ab')$_2$ fragment. In another embodiment, the antibody is a full length antibody, e.g., an intact IgG1 antibody or other antibody class or isotype as defined herein.

In some embodiments, the anti-FGF19 antibodies for use in any of the methods described herein are an anti-FGF19 antibody described in U.S. Patent Application No. 11/673,411, filed February 9, 2007, and/or U.S. Patent Application No. 12/671,974, filed December 6, 2011.

In some embodiments, the anti-FGF19 antibody comprises: (i) a light chain comprising (a) hypervariable region (HVR)-L1 comprising amino acid sequence KASQDINSFLA (SEQ ID NO:1) or KASQDINSFLS (SEQ ID NO:7); (b) HVR-L2 comprising amino acid sequence RANRLVD (SEQ ID NO:2), RANRLVS (SEQ ID NO:8), or RANRLVE (SEQ ID NO:9); and (c) HVR-L3 comprising amino acid sequence LQYDEFPLT (SEQ ID NO:3); and (ii) a heavy chain comprising (a) HVR-H1 comprising amino acid sequence GFSLTTYGVH (SEQ ID NO:4); (b) HVR-H2 comprising amino acid sequence is GVIWPGGGTDYNAAFIS (SEQ ID NO:5); and (c) HVR-H3 comprising amino acid sequence VRKEYANLYAMDY (SEQ ID NO:6).

In some embodiments, HVR-L2 comprises amino acid sequence RANRLVD (SEQ ID NO:2). In some embodiments, HVR-L1 comprises amino acid sequence KASQDINSFLS (SEQ ID NO:7).

In some embodiments, HVR-L2 comprises amino acid sequence RANRLVS (SEQ ID NO:8). In some embodiments, HVR-L2 comprises amino acid sequence RANRLVE (SEQ ID NO:9). In some embodiments, HVR-L1 comprises amino acid sequence KASQDINSFLA (SEQ ID NO:1).

In some embodiments, the antibody comprises human κ subgroup 1 consensus framework sequence. In some embodiments, the antibody comprises heavy chain human subgroup III consensus framework sequence. In some embodiments, the humanized antibody inhibits binding of human FGF19 to human FGFR4, or inhibits human FGFR4 activation, with an IC50 that is substantially the same as that of the reference antibody. In some embodiments, the IC50 is determined across an antibody concentration range from about 0.01 nm to around 1000 nM.

In some embodiments, an anti-FGF19 antibody competes for binding with the antibody described above. In some embodiments, an anti-FGF19 antibody that binds the same epitopes as the antibody described above. In some embodiments, an anti-FGF19 antibody that binds amino acid numbers G133-R155 based on the mature human FGF19 amino acid sequence on human FGF19. In some embodiments, the anti-FGF19 antibody that binds a peptide consisting of GFLPLSHFLPMLPMVPEEPEDLR (SEQ ID NO:10).

In a further aspect, an anti-FGF19 antibody, anti-klotho antibody (e.g., anti-KLB antibody), and/or anti-FGFR4 antibody, according to any of the above embodiments may incorporate any of the features, singly or in combination, as described in Sections below:

### 1. Antibody Affinity

In certain embodiments, an antibody provided herein has a dissociation constant (Kd) of ≤ 1μM. In one embodiment, Kd is measured by a radiolabeled antigen binding assay (RIA) performed with the Fab version of an antibody of interest and its antigen as described by the following assay. Solution binding affinity of Fabs for antigen is measured by equilibrating Fab with a minimal concentration of ($^{125}$I)-labelled antigen in the presence of a titration series of unlabeled antigen, then capturing bound antigen with an anti-Fab antibody-coated plate (see, e.g., Chen et al., J. Mol. Biol. 293:865-881(1999)). To establish conditions for the assay, MICROTITER® multi-well plates (Thermo Scientific) are coated overnight with 5 μg/ml of a capturing anti-Fab antibody (Cappel Labs) in 50 mM sodium carbonate (pH 9.6), and subsequently blocked with 2% (w/v) bovine serum albumin in PBS for two to five hours at room temperature (approximately 23°C). In a non-adsorbent plate (Nunc #269620), 100 pM or 26 pM [$^{125}$I]-antigen are mixed with serial dilutions of a Fab of interest (e.g., consistent with assessment of the anti-VEGF antibody, Fab-12, in Presta et al., Cancer Res. 57:4593-4599 (1997)). The Fab of interest is then incubated overnight; however, the incubation may continue for a longer period (e.g., about 65 hours) to ensure that equilibrium is reached. Thereafter, the mixtures are transferred to the capture plate for incubation at room temperature (e.g., for one hour). The solution is then removed and the plate washed eight times with 0.1% polysorbate 20 (TWEEN-20®) in PBS. When the plates have dried, 150 μl/well of scintillant (MICROSCINT-20™; Packard) is added, and the plates are counted on a TOPCOUNT™ gamma counter (Packard) for ten minutes. Concentrations of each Fab that give less than or equal to 20% of maximal binding are chosen for use in competitive binding assays.

According to another embodiment, Kd is measured using surface plasmon resonance assays using a BIACORE®-2000 or a BIACORE®-3000 (BIAcore, Inc., Piscataway, NJ) at 25°C with immobilized antigen CM5 chips at ~10 response units (RU). Briefly, carboxymethylated dextran biosensor chips (CM5, BIACORE, Inc.) are activated with N-ethyl-N'- (3-dimethylaminopropyl)-carbodiimide hydrochloride (EDC) and N-hydroxysuccinimide (NHS) according to the supplier's instructions. Antigen is diluted with 10 mM sodium acetate, pH 4.8, to 5 $\mu$g/ml (~0.2 $\mu$M) before injection at a flow rate of 5 $\mu$l/minute to achieve approximately 10 response units (RU) of coupled protein. Following the injection of antigen, 1 M ethanolamine is injected to block unreacted groups. For kinetics measurements, two-fold serial dilutions of Fab (0.78 nM to 500 nM) are injected in PBS with 0.05% polysorbate 20 (TWEEN-20™) surfactant (PBST) at 25°C at a flow rate of approximately 25 $\mu$l/min. Association rates ($k_{on}$) and dissociation rates ($k_{off}$) are calculated using a simple one-to-one Langmuir binding model (BIACORE® Evaluation Software version 3.2) by simultaneously fitting the association and dissociation sensorgrams. The equilibrium dissociation constant (Kd) is calculated as the ratio $k_{off}/k_{on}$. *See, e.g.,* Chen et al., J. Mol. Biol. 293:865-881 (1999). If the on-rate exceeds 106 $M^{-1}$ $s^{-1}$ by the surface plasmon resonance assay above, then the on-rate can be determined by using a fluorescent quenching technique that measures the increase or decrease in fluorescence emission intensity (excitation = 295 nm; emission = 340 nm, 16 nm band-pass) at 25°C of a 20 nM anti-antigen antibody (Fab form) in PBS, pH 7.2, in the presence of increasing concentrations of antigen as measured in a spectrometer, such as a stop-flow equipped spectrophometer (Aviv Instruments) or a 8000-series SLM-AMINCO™ spectrophotometer (ThermoSpectronic) with a stirred cuvette.

## 2. Antibody Fragments

In certain embodiments, an antibody provided herein is an antibody fragment. Antibody fragments include, but are not limited to, Fab, Fab', Fab'-SH, F(ab')$_2$, Fv, and scFv fragments, and other fragments described below. For a review of certain antibody fragments, *see* Hudson et al. Nat. Med. 9:129-134 (2003). For a review of scFv fragments, *see, e.g.,* Pluckthün, in The Pharmacology of Monoclonal Antibodies, vol. 113, Rosenburg and Moore eds., (Springer-Verlag, New York), pp. 269-315 (1994); *see also* WO 93/16185; and U.S. Patent Nos. 5,571,894 and 5,587,458. For discussion of Fab and F(ab')$_2$ fragments comprising salvage receptor binding epitope residues and having increased in vivo half-life, *see* U.S. Patent No. 5,869,046.

Diabodies are antibody fragments with two antigen-binding sites that may be bivalent or bispecific. *See,* for example, EP 404,097; WO 1993/01161; Hudson et al., Nat. Med. 9:129-134 (2003); and Hollinger et al., Proc. Natal. Acad. Sci. USA 90: 6444-6448 (1993). Triabodies and tetrabodies are also described in Hudson et al., Nat. Med. 9:129-134 (2003).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; *see, e.g.,* U.S. Patent No. 6,248,516 B1).

Antibody fragments can be made by various techniques, including but not limited to proteolytic digestion of an intact antibody as well as production by recombinant host cells (*e.g.,* E. coli or phage), as described herein.

## 3. Chimeric and Humanized Antibodies

In certain embodiments, an antibody provided herein is a chimeric antibody. Certain chimeric antibodies are described, *e.g.,* in U.S. Patent No. 4,816,567; and Morrison et al., Proc. Natal. Acad. Sci. USA, 81:6851-6855 (1984)). In one example, a chimeric antibody comprises a non-human variable region (*e.g.,* a variable region derived from a mouse, rat, hamster, rabbit, or non-human primate, such as a monkey) and a human constant region. In a further example, a chimeric antibody is a "class switched" antibody in which the class or subclass has been changed from that of the parent antibody. Chimeric antibodies include antigen-binding fragments thereof.

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, *e.g.*, CDRs, (or portions thereof) are derived from a non-human antibody, and FRs (or portions thereof) are derived from human antibody sequences. A humanized antibody optionally will also comprise at least a portion of a human constant region. In some embodiments, some FR residues in a humanized antibody are substituted with corresponding residues from a non-human antibody (*e.g.,* the antibody from which the HVR residues are derived), *e.g.*, to restore or improve antibody specificity or affinity.

Humanized antibodies and methods of making them are reviewed, *e.g.*, in Almagro and Fransson, Front. Biosci. 13:1619-1633 (2008), and are further described, *e.g.,* in Riechmann et al., Nature 332:323-329 (1988); Queen et al., Proc. Nat'l Acad. Sci. USA 86:10029-10033 (1989); US Patent Nos. 5, 821,337, 7,527,791, 6,982,321, and 7,087,409; Kashmiri et al., Methods 36:25-34 (2005) (describing SDR (a-CDR) grafting); Padlan, Mol. Immunol. 28:489-498 (1991) (describing "resurfacing"); Dall'Acqua et al., Methods 36:43-60 (2005) (describing "FR shuffling"); and Osbourn et al., Methods 36:61-68 (2005) and Klimka et al., Br. J. Cancer, 83:252-260 (2000) (describing the "guided selection" approach to FR shuffling).

Human framework regions that may be used for humanization include but are not limited to: framework regions selected using the "best-fit" method (*see, e.g.,* Sims et al. J. Immunol. 151:2296 (1993)); framework regions derived from the consensus sequence of human antibodies of a particular subgroup of light or heavy chain variable regions *(see, e.g.,* Carter et al. Proc. Natl. Acad. Sci. USA, 89:4285 (1992); and Presta et al. J. Immunol., 151:2623 (1993)); human mature (somatically mutated) framework regions or human germline framework regions *(see, e.g.,* Almagro and Fransson, Front. Biosci. 13:1619-1633 (2008)); and framework regions derived from screening FR libraries *(see, e.g.,* Baca et al., J. Biol. Chem. 272:10678-10684 (1997) and Rosok et al., J. Biol. Chem. 271:22611-22618 (1996)).

### 4. Human Antibodies

In certain embodiments, an antibody provided herein is a human antibody. Human antibodies can be produced using various techniques known in the art. Human antibodies are described generally in van Dijk and van de Winkel, Curr. Opin. Pharmacol. 5: 368-74 (2001) and Lonberg, Curr. Opin. Immunol. 20:450-459 (2008).

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, *see* Lonberg, Nat. Biotech. 23:1117-1125 (2005). *See also, e.g.,* U.S. Patent Nos. 6,075,181 and 6,150,584 describing XENOMOUSE™ technology; U.S. Patent No. 5,770,429 describing HuMab® technology; U.S. Patent No. 7,041,870 describing K-M MOUSE® technology, and U.S. Patent Application Publication No. US 2007/0061900, describing VelociMouse® technology). Human variable regions from intact antibodies generated by such animals may be further modified, *e.g.*, by combining with a different human constant region.

Human antibodies can also be made by hybridoma-based methods. Human myeloma and mouse-human heteromyeloma cell lines for the production of human monoclonal antibodies have been described. (*See, e.g.,* Kozbor J. Immunol., 133: 3001 (1984); and Boerner et al., J. Immunol., 147: 86 (1991).) Human antibodies generated via human B-cell hybridoma technology are also described in Li et al., Proc. Natl. Acad. Sci. USA, 103:3557-3562 (2006). Additional methods include those described, for example, in U.S. Patent No. 7,189,826 (describing production of monoclonal human IgM antibodies from hybridoma cell lines) and Ni, Xiandai Mianyixue, 26(4):265-268 (2006) (describing human-human hybridomas). Human hybridoma technology (Trioma technology) is also described in Vollmers and Brandlein, Histology and Histopathology, 20(3):927-937 (2005) and Vollmers and Brandlein, Methods and Findings in Exp. & Clin. Pharma., 27(3):185-91 (2005).

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### 5. Library-Derived Antibodies

Antibodies may be isolated by screening combinatorial libraries for antibodies with the desired activity or activities. For example, a variety of methods are known in the art for generating phage display libraries and screening such libraries for antibodies possessing the desired binding characteristics. Such methods are reviewed, *e.g.*, in Hoogenboom et al. in Methods in Molecular Biology 178:1-37 (O'Brien et al., ed., Human Press, Totowa, NJ, 2001) and further described, *e.g.,* in the McCafferty et al., Nature 348:552-554; Clackson et al., Nature 352: 624-628 (1991); Marks et al., J. Mol. Biol. 222: 581-597 (1992); Marks and Bradbury, in Methods in Molecular Biology 248:161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natal. Acad. Sci. USA 101(34): 12467-12472 (2004); and Lee et al., J. Immunol. Methods 284(1-2): 119-132(2004).

In certain phage display methods, repertoires of VH and VL genes are separately cloned by polymerase chain reaction (PCRzz) and recombined randomly in phage libraries, which can then be screened for antigen-binding phage as described in Winter et al., Ann. Rev. Immunol., 12: 433-455 (1994). Phage typically display antibody fragments, either as single-chain Fv (scFv) fragments or as Fab fragments. Libraries from immunized sources provide high-affinity antibodies to the immunogen without the requirement of constructing hybridomas. Alternatively, the naive repertoire can be cloned (*e.g.,* from human) to provide a single source of antibodies to a wide range of non-self and also self antigens without any immunization as described by Griffiths et al., EMBO J, 12: 725-734 (1993). Finally, naive libraries can also be made synthetically by cloning unrearranged V-gene segments from stem cells, and using PCR primers containing random sequence to encode the highly variable CDR3 regions and to accomplish rearrangement in vitro, as described by Hoogenboom and Winter, J. Mol. Biol., 227: 381-388 (1992). Patent publications describing human antibody phage libraries include, for example: US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

Antibodies or antibody fragments isolated from human antibody libraries are considered human antibodies or human antibody fragments herein.

### 6. Multispecific Antibodies

In certain embodiments, an antibody provided herein is a multispecific antibody, *e.g*., a bispecific antibody. Multispecific antibodies are monoclonal antibodies that have binding specificities for at least two different sites. In certain embodiments, one of the binding specificities is for FGF19, klotho (*e.g.,* KLB), and/or FGFR4 and the other is for any other antigen. In certain embodiments, bispecific antibodies may bind to two different epitopes of FGF19, klotho (*e.g.,* KLB), and/or FGFR4 polypeptide. Bispecific antibodies may also be used to localize cytotoxic agents to cells which express polypeptide such as FGF19, klotho (*e.g.,* KLB), and/or FGFR4 polypeptide. Bispecific antibodies can be prepared as full length antibodies or antibody fragments.

Techniques for making multispecific antibodies include, but are not limited to, recombinant co-expression of two immunoglobulin heavy chain-light chain pairs having different specificities (*see* Milstein and Cuello, Nature 305: 537 (1983)), WO 93/08829, and Traunecker et al., EMBO J. 10: 3655 (1991)), and "knob-in-hole" engineering (*see, e.g.,* U.S. Patent No. 5,731,168). Multi-specific antibodies may also be made by engineering electrostatic steering effects for making antibody Fc-heterodimeric molecules (WO 2009/089004A1); cross-linking two or more antibodies or fragments (*see, e.g.,* US Patent No. 4,676,980, and Brennan et al., Science, 229: 81 (1985)); using leucine zippers to produce bispecific antibodies (*see, e.g.,* Kostelny et al., J. Immunol., 148(5):1547-1553 (1992)); using "diabody" technology for making bispecific antibody fragments (*see, e.g.,* Hollinger et al., Proc. Natal. Acad. Sci. USA, 90:6444-6448 (1993)); and using single-chain Fv (sFv) dimers (*see, e.g.,* Gruber et al., J. Immunol., 152:5368 (1994)); and preparing trispecific antibodies as described, *e.g.,* in Tutt et al. J. Immunol. 147: 60 (1991).

Engineered antibodies with three or more functional antigen binding sites, including "Octopus antibodies," are also included herein (*see, e.g.,* US 2006/0025576A1).

The antibody or fragment herein also includes a "Dual Acting FAb" or "DAF" comprising an antigen binding site that binds to FGF19, klotho (*e.g.,* KLB), and/or FGFR4 polypeptide as well as another, different antigen (*see,* US 2008/0069820, for example).

### 7. Antibody Variants

#### a) Glycosylation variants

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. *See, e.g.,* Wright et al. TIBTECH 15:26-32 (1997). The oligosaccharide may include various carbohydrates, *e.g.,* mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in an antibody may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fc region residues); however, Asn297 may also be located about ± 3 amino acids upstream or downstream of position 297, *i.e.,* between positions 294 and 300, due to minor sequence variations in antibodies. Such fucosylation variants may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al., Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/157108 A1, Presta, L; and WO 2004/056312 A1, Adams et al., especially at Example 11), and knockout cell lines, such as alpha-1,6-fucosyltransferase gene, FUT8, knockout CHO cells (*see, e.g.,* Yamane-Ohnuki et al. Biotech. Bioeng. 87: 614 (2004); Kanda, Y. et al., Biotech. Bioeng., 94(4):680-688 (2006); and WO2003/085107).

Antibodies variants are further provided with bisected oligosaccharides, *e.g.*, in which a biantennary oligosaccharide attached to the Fc region of the antibody is bisected by GlcNAc. Such antibody variants may have reduced fucosylation and/or improved ADCC function. Examples of such antibody variants are described, *e.g.*, in WO 2003/011878 (Jean-Mairet et al.); US Patent No. 6,602,684 (Umana et al.); and US 2005/0123546 (Umana et al.). Antibody variants with at least one galactose residue in the oligosaccharide attached to the Fc region are also provided. Such antibody variants may have improved CDC function. Such antibody variants are described, *e.g.*, in WO 1997/30087 (Patel et al.); WO 1998/58964 (Raju, S.); and WO 1999/22764 (Raju, S.).

### b) Fc region variants

In certain embodiments, one or more amino acid modifications may be introduced into the Fc region of an antibody provided herein, thereby generating an Fc region variant. The Fc region variant may comprise a human Fc region sequence (*e.g.*, a human IgG1, IgG2, IgG3 or IgG4 Fc region) comprising an amino acid modification (*e.g.*, a substitution) at one or more amino acid positions.

In certain embodiments, the invention contemplates an antibody variant that possesses some but not all effector functions, which make it a desirable candidate for applications in which the half life of the antibody in vivo is important yet certain effector functions (such as complement and ADCC) are unnecessary or deleterious. In vitro and/or in vivo cytotoxicity assays can be conducted to confirm the reduction/depletion of CDC and/or ADCC activities. For example, Fc receptor (FcR) binding assays can be conducted to ensure that the antibody lacks FcγR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Nonlimiting examples of in vitro assays to assess ADCC activity of a molecule of interest is described in U.S. Patent No. 5,500,362 (*see, e.g.,* Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (*see* Bruggemann, M. et al., J. Exp. Med. 166:1351-1361 (1987)). Alternatively, non-radioactive assays methods may be employed (*see,* for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed in vivo, *e.g.*, in a animal model such as that disclosed in Clynes et al. Proc. Nat'l Acad. Sci. USA 95:652-656 (1998). C1q binding assays may also be carried out to confirm that the antibody is unable to bind C1q and hence lacks CDC activity. *See, e.g.,* C1q and C3c binding ELISA in WO 2006/029879 and WO 2005/100402. To assess complement activation, a CDC assay may be performed (*see,* for example, Gazzano-Santoro et al., J. Immunol. Methods 202:163 (1996); Cragg, M.S. et al., Blood 101:1045-1052 (2003); and Cragg, M.S. and M.J. Glennie, Blood 103:2738-2743 (2004)). FcRn binding and in vivo clearance/half life determinations can also be performed using methods known in the art (*see, e.g.,* Petkova, S.B. et al., Int'l. Immunol. 18(12):1759-1769 (2006)).

Antibodies with reduced effector function include those with substitution of one or more of Fc region residues 238, 265, 269, 270, 297, 327 and 329 (U.S. Patent No. 6,737,056). Such Fc mutants include Fc mutants with substitutions at two or more of amino acid positions 265, 269, 270, 297 and 327, including the so-called "DANA" Fc mutant with substitution of residues 265 and 297 to alanine (US Patent No. 7,332,581).

Certain antibody variants with improved or diminished binding to FcRs are described. (*See, e.g.,* U.S. Patent No. 6,737,056; WO 2004/056312, and Shields et al., J. Biol. Chem. 9(2): 6591-6604 (2001).) In certain embodiments, an antibody variant comprises an Fc region with one or more amino acid substitutions which improve ADCC, *e.g.*, substitutions at positions 298, 333, and/or 334 of the Fc region (EU numbering of residues). In some embodiments, alterations are made in the Fc region that result in altered (*i.e.*, either improved or diminished) C1q binding and/or Complement Dependent Cytotoxicity (CDC), *e.g.*, as described in US Patent No. 6,194,551, WO 99/51642, and Idusogie et al. J. Immunol. 164: 4178-4184 (2000).

Antibodies with increased half lives and improved binding to the neonatal Fc receptor (FcRn), which is responsible for the transfer of maternal IgGs to the fetus (Guyer et al., J. Immunol. 117:587 (1976) and Kim et al., J. Immunol. 24:249 (1994)), are described in US2005/0014934A1 (Hinton et al.). Those antibodies comprise an Fc region with one or more substitutions therein which improve binding of the Fc region to FcRn. Such Fc variants include those with substitutions at one or more of Fc region residues: 238, 256, 265, 272, 286, 303, 305, 307, 311, 312, 317, 340, 356, 360, 362, 376, 378, 380, 382, 413, 424 or 434, *e.g.*, substitution of Fc region residue 434 (US Patent No. 7,371,826). *See* also Duncan & Winter, Nature 322:738-40 (1988); U.S. Patent No. 5,648,260; U.S. Patent No. 5,624,821; and WO 94/29351 concerning other examples of Fc region variants.

*c) Cysteine engineered antibody variants*

In certain embodiments, it may be desirable to create cysteine engineered antibodies, *e.g.*, "thioMAbs," in which one or more residues of an antibody are substituted with cysteine residues. In particular embodiments, the substituted residues occur at accessible sites of the antibody. By substituting those residues with cysteine, reactive thiol groups are thereby positioned at accessible sites of the antibody and may be used to conjugate the antibody to other moieties, such as drug moieties or linker-drug moieties, to create an immunoconjugate, as described further herein. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antibodies may be generated as described, *e.g.*, in U.S. Patent No. 7,521,541.

*B. Immunoconjugates*

Further provided herein are immunoconjugates comprising an anti-FGF19 antibody, anti-klotho antibody (*e.g.*, anti-KLB antibody), and/or anti-FGFR4 antibody herein conjugated to one or more cytotoxic agents, such as chemotherapeutic agents or drugs, growth inhibitory agents, toxins (*e.g.*, protein toxins, enzymatically active toxins of bacterial, fungal, plant, or animal origin, or fragments thereof), or radioactive isotopes.

In one embodiment, an immunoconjugate is an antibody-drug conjugate (ADC) in which an antibody is conjugated to one or more drugs, including but not limited to a maytansinoid (*see* U.S. Patent Nos. 5,208,020, 5,416,064 and European Patent EP 0 425 235 B1); an auristatin such as monomethylauristatin drug moieties DE and DF (MMAE and MMAF) (*see* U.S. Patent Nos. 5,635,483 and 5,780,588, and 7,498,298); a dolastatin; a calicheamicin or derivative thereof (*see* U.S. Patent Nos. 5,712,374, 5,714,586, 5,739,116, 5,767,285, 5,770,701, 5,770,710, 5,773,001, and 5,877,296; Hinman et al., Cancer Res. 53:3336-3342 (1993); and Lode et al., Cancer Res. 58:2925-2928 (1998)); an anthracycline such as daunomycin or doxorubicin (*see* Kratz et al., Current Med. Chem. 13:477-523 (2006); Jeffrey et al., Bioorganic & Med. Chem. Letters 16:358-362 (2006); Torgov et al., Bioconj. Chem. 16:717-721 (2005); Nagy et al., Proc. Natal. Acad. Sci. USA 97:829-834 (2000); Dubowchik et al., Bioorg. & Med. Chem. Letters 12:1529-1532 (2002); King et al., J. Med. Chem. 45:4336-4343 (2002); and U.S. Patent No. 6,630,579); methotrexate; vindesine; a taxane such as docetaxel, paclitaxel, larotaxel, tesetaxel, and ortataxel; a trichothecene; and CC1065.

In another embodiment, an immunoconjugate comprises an antibody as described herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises an antibody as described herein conjugated to a radioactive atom to form a radioconjugate. A variety of radioactive isotopes are available for the production of radioconjugates. Examples include $At^{211}, I^{131}, I^{125}, Y^{90}, Re^{186}, Re^{188}, Sm^{153}, Bi^{212}, P^{32}, Pb^{212}$ and radioactive isotopes of Lu. When the radioconjugate is used for detection, it may comprise a radioactive atom for scintigraphic studies, for example $Tc^{99}$ or $I^{123}$, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron.

Conjugates of an antibody and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HCl), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta et al., Science 238:1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. *See* WO94/11026. The linker may be a "cleavable linker" facilitating release of a cytotoxic drug in the cell. For example, an acid-labile linker, peptidase-sensitive linker, photolabile linker, dimethyl linker or disulfide-containing linker (Chari et al., Cancer Res. 52:127-131 (1992); U.S. Patent No. 5,208,020) may be used.

The immunuoconjugates or ADCs herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (*e.g.,* from Pierce Biotechnology, Inc., Rockford, IL., U.S.A).

*C. Binding Polypeptides*

Provided herein are FGF19, klotho (*e.g.*, KLB), and/or FGFR4 binding polypeptides for use as an FGF19 modulator, *e.g.*, FGF19 antagonist, in any of the methods described herein. In some embodiments, the FGF19, klotho (*e.g.*, KLB), and/or FGFR4 binding polypeptide is an FGF19, klotho (*e.g.*, KLB), and/or FGFR4 binding polypeptide antagonist. FGF19, klotho (*e.g.*, KLB), and/or FGFR4 binding polypeptides antagonists are polypeptides that bind, preferably specifically, to FGF19, klotho (*e.g.*, KLB), and/or FGFR4 polypeptide.

Binding polypeptides may be chemically synthesized using known polypeptide synthesis methodology or may be prepared and purified using recombinant technology. Binding polypeptides are usually at least about 5 amino acids in length, alternatively at least about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100 amino acids in length or more, wherein such binding polypeptides that are capable of binding, preferably specifically, to a target, FGF19, klotho (*e.g.*, KLB), and/or FGFR4 polypeptide, as described herein. Binding polypeptides may be identified without undue experimentation using well known techniques. In this regard, it is noted that techniques for screening polypeptide libraries for binding polypeptides that are capable of specifically binding to a polypeptide target are well known in the art (*see, e.g.*, U.S. Patent Nos. 5,556,762, 5,750,373, 4,708,871, 4,833,092, 5,223,409, 5,403,484, 5,571,689, 5,663,143; PCT Publication Nos. WO 84/03506 and WO84/03564; Geysen et al., Proc. Nat'l Acad. Sci. USA, 81:3998-4002 (1984); Geysen et al., Proc. Nat'l Acad. Sci. USA, 82:178-182 (1985); Geysen et al., in Synthetic Peptides as Antigens, 130-149 (1986); Geysen et al., J. Immunol. Meth., 102:259-274 (1987); Schoofs et al., J. Immunol., 140:611-616 (1988), Cwirla, S. E. et al. (1990) Proc. Natal. Acad. Sci. USA, 87:6378; Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natal. Acad. Sci. USA, 88:8363, and Smith, G. P. (1991) Current Opin. Biotechnol., 2:668).

In this regard, bacteriophage (phage) display is one well known technique which allows one to screen large polypeptide libraries to identify member(s) of those libraries which are capable of specifically binding to a target polypeptide, *e.g.*, FGF19, klotho (*e.g.*, KLB), and/or FGFR4 polypeptide. Phage display is a technique by which variant polypeptides are displayed as fusion proteins to the coat protein on the surface of bacteriophage particles (Scott, J.K. and Smith, G. P. (1990) Science, 249: 386). The utility of phage display lies in the fact that large libraries of selectively randomized protein variants (or randomly cloned cDNAs) can be rapidly and efficiently sorted for those sequences that bind to a target molecule with high affinity. Display of peptide (Cwirla, S. E. et al. (1990) Proc. Natal. Acad. Sci. USA, 87:6378) or protein (Lowman, H.B. et al. (1991) Biochemistry, 30:10832; Clackson, T. et al. (1991) Nature, 352: 624; Marks, J. D. et al. (1991), J. Mol. Biol., 222:581; Kang, A.S. et al. (1991) Proc. Natal. Acad. Sci. USA, 88:8363) libraries on phage have been used for screening millions of polypeptides or oligopeptides for ones with specific binding properties (Smith, G. P. (1991) Current Opin. Biotechnol., 2:668). Sorting phage libraries of random mutants requires a strategy for constructing and propagating a large number of variants, a procedure for affinity purification using the target receptor, and a means of evaluating the results of binding enrichments. U.S. Patent Nos. 5,223,409, 5,403,484, 5,571,689, and 5,663,143.

Although most phage display methods have used filamentous phage, lambdoid phage display systems (WO 95/34683; U.S. 5,627,024), T4 phage display systems (Ren et al., Gene, 215: 439 (1998); Zhu et al., Cancer Research, 58(15): 3209-3214 (1998); Jiang et al., Infection & Immunity, 65(11): 4770-4777 (1997); Ren et al., Gene, 195(2):303-311 (1997); Ren, Protein Sci., 5: 1833 (1996); Efimov et al., Virus Genes, 10: 173 (1995)) and T7 phage display systems (Smith and Scott, Methods in Enzymology, 217: 228-257 (1993); U.S. 5,766,905) are also known.

Additional improvements enhance the ability of display systems to screen peptide libraries for binding to selected target molecules and to display functional proteins with the potential of screening these proteins for desired properties. Combinatorial reaction devices for phage display reactions have been developed (WO 98/14277) and phage display libraries have been used to analyze and control bimolecular interactions (WO 98/20169; WO 98/20159) and properties of constrained helical peptides (WO 98/20036). WO 97/35196 describes a method of isolating an affinity ligand in which a phage display library is contacted with one solution in which the ligand will bind to a target molecule and a second solution in which the affinity ligand will not bind to the target molecule, to selectively isolate binding ligands. WO 97/46251 describes a method of biopanning a random phage display library with an affinity purified antibody and then isolating binding phage, followed by a micropanning process using microplate wells to isolate high affinity binding phage. The use of Staphylococcus aureus protein A as an affinity tag has also been reported (Li et al. (1998) Mol Biotech., 9:187). WO 97/47314 describes the use of substrate subtraction libraries to distinguish enzyme specificities using a combinatorial library which may be a phage display library. A method for selecting enzymes suitable for use in detergents using phage display is described in WO 97/09446. Additional methods of selecting specific binding proteins are described in U.S. Patent Nos. 5,498,538, 5,432,018, and WO 98/15833.

Methods of generating peptide libraries and screening these libraries are also disclosed in U.S. Patent Nos.

5,723,286, 5,432,018, 5,580,717, 5,427,908, 5,498,530, 5,770,434, 5,734,018, 5,698,426, 5,763,192, and 5,723,323.

*D. Binding Small Molecules*

Provided herein are FGF19, klotho (*e.g.,* KLB), and/or FGFR4 small molecules for use as an FGF19 modulator (*e.g.,* FGF19 antagonist) in any of the methods described herein. In some embodiments, the FGF19, klotho (*e.g.,* KLB), and/or FGFR4 small molecule is an FGF19, klotho (*e.g.,* KLB), and/or FGFR4 small molecule antagonist.

In some embodiments of any of the small molecules, the FGF19 antagonist is an FGF19 small molecule antagonist. In some embodiment, the FGF19 antagonist is a klotho (*e.g.,* KLB) small molecule antagonist. In some embodiment, the FGF19 antagonist is FGFR4 small molecule antagonist.

In some embodiments of any of the small molecules, the small molecule binds to an FGF19 polypeptide. In some embodiments, the small molecule binds klotho (*e.g.,* KLB) polypeptide. In some embodiments, the small molecule binds FGFR4.

Small molecules are preferably organic molecules other than binding polypeptides or antibodies as defined herein that bind, preferably specifically, to an FGF19, FGFR4, and/or klotho (*e.g.,* KLB) polypeptide as described herein. Organic small molecules may be identified and chemically synthesized using known methodology (*see, e.g.,* PCT Publication Nos. WO00/00823 and WO00/39585). Organic small molecules are usually less than about 2000 Daltons in size, alternatively less than about 1500, 750, 500, 250 or 200 Daltons in size, wherein such organic small molecules that are capable of binding, preferably specifically, to a polypeptide as described herein may be identified without undue experimentation using well known techniques. In this regard, it is noted that techniques for screening organic small molecule libraries for molecules that are capable of binding to a polypeptide target are well known in the art (*see, e.g.,* PCT Publication Nos. WO00/00823 and WO00/39585). Organic small molecules may be, for example, aldehydes, ketones, oximes, hydrazones, semicarbazones, carbazides, primary amines, secondary amines, tertiary amines, N-substituted hydrazines, hydrazides, alcohols, ethers, thiols, thioethers, disulfides, carboxylic acids, esters, amides, ureas, carbamates, carbonates, ketals, thioketals, acetals, thioacetals, aryl halides, aryl sulfonates, alkyl halides, alkyl sulfonates, aromatic compounds, heterocyclic compounds, anilines, alkenes, alkynes, diols, amino alcohols, oxazolidines, oxazolines, thiazolidines, thiazolines, enamines, sulfonamides, epoxides, aziridines, isocyanates, sulfonyl chlorides, diazo compounds, acid chlorides, or the like.

*E. Antagonist Polynucdeotides*

Provided herein are FGF19, klotho (*e.g.,* KLB), and/or FGFR4 polynucleotides for use as an FGF19 modulator (*e.g.,* FGF19 antagonist) in any of the methods described herein. In some embodiments, the FGF19, klotho (*e.g.,* KLB), and/or FGFR4 polynucleotide is an FGF19, klotho (*e.g.,* KLB), and/or FGFR4 polynucleotide antagonist. The polynucleotide may be an antisense nucleic acid and/or a ribozyme. The antisense nucleic acids comprise a sequence complementary to at least a portion of an RNA transcript of an FGF19, klotho (*e.g.,* KLB), and/or FGFR4 gene. However, absolute complementarity, although preferred, is not required.

In some embodiments of any of the polynucleotides, the polynucleotide binds to an FGF19 polynucleotide. In some embodiments, polynucleotide specifically binds a klotho (*e.g.,* KLB) polynucleotide. In some embodiments, the polynucleotide specifically binds an FGFR4 polynucleotide.

A sequence "complementary to at least a portion of an RNA," referred to herein, means a sequence having sufficient complementarity to be able to hybridize with the RNA, forming a stable duplex; in the case of double stranded antisense nucleic acids, a single strand of the duplex DNA may thus be tested, or triplex formation may be assayed. The ability to hybridize will depend on both the degree of complementarity and the length of the antisense nucleic acid. Generally, the larger the hybridizing nucleic acid, the more base mismatches with an RNA it may contain and still form a stable duplex (or triplex as the case may be). One skilled in the art can ascertain a tolerable degree of mismatch by use of standard procedures to determine the melting point of the hybridized complex.

Polynucleotides that are complementary to the 5' end of the message, e.g., the 5' untranslated sequence up to and including the AUG initiation codon, should work most efficiently at inhibiting translation. However, sequences complementary to the 3' untranslated sequences of mRNAs have been shown to be effective at inhibiting translation of mRNAs as well. *See* generally, Wagner, R., 1994, Nature 372:333-335. Thus, oligonucleotides complementary to either the 5'- or 3'-non-translated, non-coding regions of the gene, could be used in an antisense approach to inhibit translation of endogenous mRNA. Polynucleotides complementary to the 5' untranslated region of the mRNA should include the complement of the AUG start codon. Antisense polynucleotides complementary to mRNA coding regions are less efficient inhibitors of translation but could be used in accordance with the disclosure. Whether designed to hybridize to the 5'-, 3'- or coding region of mRNA, antisense nucleic acids should be at least six nucleotides in length, and are preferably oligonucleotides ranging from 6 to about 50 nucleotides in length. In specific aspects the oligonucleotide is at least 10 nucleotides, at least 17 nucleotides, at least 25 nucleotides or at least 50 nucleotides.

In one embodiment, the antisense nucleic acid is produced intracellularly by transcription from an exogenous sequence. For example, a vector or a portion thereof, is transcribed, producing an antisense nucleic acid (RNA) of the gene. Such a vector would contain a sequence encoding the antisense nucleic acid. Such a vector can remain episomal or become chromosomally integrated, as long as it can be transcribed to produce the desired antisense RNA. Such vectors can be constructed by recombinant DNA technology methods standard in the art. Vectors can be plasmid, viral, or others know in the art, used for replication and expression in vertebrate cells. Expression of the sequence encoding FGF19, FGFR4, and/or klotho (e.g., KLB), or fragments thereof, can be by any promoter known in the art to act in vertebrate, preferably human cells. Such promoters can be inducible or constitutive. Such promoters include, but are not limited to, the SV40 early promoter region (Bernoist and Chambon, Nature 29:304-310 (1981), the promoter contained in the 3' long terminal repeat of Rous sarcoma virus (Yamamoto et al., Cell 22:787-797 (1980), the herpes thymidine promoter (Wagner et al., Proc. Nat'l Acad. Sci. USA 78:1441-1445 (1981), the regulatory sequences of the metallothionein gene (Brinster, et al., Nature 296:39-42 (1982)), etc.

## F. Antibody and Binding Polypeptide Variants

In certain embodiments, amino acid sequence variants of the antibodies and/or the binding polypeptides provided herein are contemplated. For example, it may be desirable to improve the binding affinity and/or other biological properties of the antibody and/or binding polypeptide. Amino acid sequence variants of an antibody and/or binding polypeptides may be prepared by introducing appropriate modifications into the nucleotide sequence encoding the antibody and/or binding polypeptide, or by peptide synthesis. Such modifications include, for example, deletions from, and/or insertions into and/or substitutions of residues within the amino acid sequences of the antibody and/or binding polypeptide. Any combination of deletion, insertion, and substitution can be made to arrive at the final construct, provided that the final construct possesses the desired characteristics, e.g., target-binding.

In certain embodiments, antibody variants and/or binding polypeptide variants having one or more amino acid substitutions are provided. Sites of interest for substitutional mutagenesis include the HVRs and FRs. Conservative substitutions are shown in Table 1 under the heading of "conservative substitutions." More substantial changes are provided in Table 1 under the heading of "exemplary substitutions," and as further described below in reference to amino acid side chain classes. Amino acid substitutions may be introduced into an antibody and/or binding polypeptide of interest and the products screened for a desired activity, e.g., retained/improved antigen binding, decreased immunogenicity, or improved ADCC or CDC.

### TABLE 1

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |

(continued)

| Original Residue | Exemplary Substitutions | Preferred Substitutions |
|---|---|---|
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:

(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent antibody (*e.g.*, a humanized or human antibody). Generally, the resulting variant(s) selected for further study will have modifications *(e.g.,* improvements) in certain biological properties *(e.g.,* increased affinity, reduced immunogenicity) relative to the parent antibody and/or will have substantially retained certain biological properties of the parent antibody. An exemplary substitutional variant is an affinity matured antibody, which may be conveniently generated, *e.g.*, using phage display-based affinity maturation techniques such as those described herein. Briefly, one or more HVR residues are mutated and the variant antibodies displayed on phage and screened for a particular biological activity (*e.g.*, binding affinity).

Alterations *(e.g.,* substitutions) may be made in HVRs, *e.g.,* to improve antibody affinity. Such alterations may be made in HVR "hotspots," *i.e.,* residues encoded by codons that undergo mutation at high frequency during the somatic maturation process (*see, e.g.,* Chowdhury, Methods Mol. Biol. 207:179-196 (2008)), and/or SDRs (a-CDRs), with the resulting variant VH or VL being tested for binding affinity. Affinity maturation by constructing and reselecting from secondary libraries has been described, *e.g.,* in Hoogenboom et al. in Methods in Molecular Biology 178:1-37 (O'Brien et al., ed., Human Press, Totowa, NJ, (2001).) In some embodiments of affinity maturation, diversity is introduced into the variable genes chosen for maturation by any of a variety of methods (*e.g.*, error-prone PCR, chain shuffling, or oligonucleotide-directed mutagenesis). A secondary library is then created. The library is then screened to identify any antibody variants with the desired affinity. Another method to introduce diversity involves HVR-directed approaches, in which several HVR residues (*e.g.,* 4-6 residues at a time) are randomized. HVR residues involved in antigen binding may be specifically identified, *e.g.*, using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the antibody to bind antigen. For example, conservative alterations (*e.g.*, conservative substitutions as provided herein) that do not substantially reduce binding affinity may be made in HVRs. Such alterations may be outside of HVR "hotspots" or SDRs. In certain embodiments of the variant VH and VL sequences provided above, each HVR either is unaltered, or contains no more than one, two or three amino acid substitutions.

A useful method for identification of residues or regions of the antibody and/or the binding polypeptide that may be targeted for mutagenesis is called "alanine scanning mutagenesis" as described by Cunningham and Wells (1989) Science, 244:1081-1085. In this method, a residue or group of target residues (*e.g.*, charged residues such as arg, asp, his, lys, and glu) are identified and replaced by a neutral or negatively charged amino acid (*e.g.*, alanine or polyalanine) to determine whether the interaction of the antibody with antigen is affected. Further substitutions may be introduced at the amino acid locations demonstrating functional sensitivity to the initial substitutions. Alternatively, or additionally, a crystal structure of an antigen-antibody complex to identify contact points between the antibody and antigen. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include an antibody with an N-terminal methionyl residue. Other insertional variants of the antibody molecule include the fusion to the N- or C-terminus of the antibody to an enzyme (*e.g.,* for ADEPT) or a polypeptide which increases the serum half-life of the antibody.

## G. Antibody and Binding Polypeptide Derivatives

In certain embodiments, an antibody and/or binding polypeptide provided herein may be further modified to contain additional nonproteinaceous moieties that are known in the art and readily available. The moieties suitable for derivatization of the antibody and/or binding polypeptide include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (*e.g.,* glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody and/or binding polypeptide may vary, and if more than one polymer is attached, they can be the same or different molecules. In general, the number and/or type of polymers used for derivatization can be determined based on considerations including, but not limited to, the particular properties or functions of the antibody and/or binding polypeptide to be improved, whether the antibody derivative and/or binding polypeptide derivative will be used in a therapy under defined conditions, etc.

In another embodiment, conjugates of an antibody and/or binding polypeptide to nonproteinaceous moiety that may be selectively heated by exposure to radiation are provided. In one embodiment, the nonproteinaceous moiety is a carbon nanotube (Kam et al., Proc. Natal. Acad. Sci. USA 102: 11600-11605 (2005)). The radiation may be of any wavelength, and includes, but is not limited to, wavelengths that do not harm ordinary cells, but which heat the nonproteinaceous moiety to a temperature at which cells proximal to the antibody and/or binding polypeptide-nonproteinaceous moiety are killed.

## H. Recombinant Methods and Compositions

Antibodies and/or binding polypeptides may be produced using recombinant methods and compositions, *e.g.,* as described in U.S. Patent No. 4,816,567. In one embodiment, isolated nucleic acid encoding an anti-FGF19 antibody, anti-FGFR4 antibody, and/or anti-klotho antibody (*e.g.,* anti-KLB antibody). Such nucleic acid may encode an amino acid sequence comprising the VL and/or an amino acid sequence comprising the VH of the antibody (*e.g.,* the light and/or heavy chains of the antibody). In a further embodiment, one or more vectors (*e.g.,* expression vectors) comprising such nucleic acid encoding the antibody and/or binding polypeptide are provided. In a further embodiment, a host cell comprising such nucleic acid is provided. In one such embodiment, a host cell comprises (*e.g.,* has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, *e.g.,* a Chinese Hamster Ovary (CHO) cell or lymphoid cell (*e.g.,* Y0, NS0, Sp20 cell). In one embodiment, a method of making an antibody such as an anti-FGF19 antibody, anti-FGFR4 antibody, and/or anti-klotho antibody (*e.g.,* anti-KLB antibody) and/or binding polypeptide is provided, wherein the method comprises culturing a host cell comprising a nucleic acid encoding the antibody and/or binding polypeptide, as provided above, under conditions suitable for expression of the antibody and/or binding polypeptide, and optionally recovering the antibody and/or polypeptide from the host cell (or host cell culture medium).

For recombinant production of an antibody such as an anti-FGF19 antibody, anti-klotho antibody (*e.g.,* anti-KLB antibody), anti-FGFR4 antibody and/or a binding polypeptide, nucleic acid encoding the antibody and/or the binding polypeptide, *e.g.,* as described above, is isolated and inserted into one or more vectors for further cloning and/or expression in a host cell. Such nucleic acid may be readily isolated and sequenced using conventional procedures (*e.g.,* by using oligonucleotide probes that are capable of binding specifically to genes encoding the heavy and light chains of the antibody).

Suitable host cells for cloning or expression of vectors include prokaryotic or eukaryotic cells described herein. For example, antibodies may be produced in bacteria, in particular when glycosylation and Fc effector function are not needed. For expression of antibody fragments and polypeptides in bacteria, *see, e.g.,* U.S. Patent Nos. 5,648,237, 5,789,199, and 5,840,523. (*See* also Charlton, Methods in Mol. Bio., Vol. 248 (B.K.C. Lo, ed., Humana Press, Totowa, NJ, 2003), pp. 245-254, describing expression of antibody fragments in E. coli.) After expression, the antibody may be isolated from the bacterial cell paste in a soluble fraction and can be further purified.

In addition to prokaryotes, eukaryotic microbes such as filamentous fungi or yeast are suitable cloning or expression hosts for vectors, including fungi and yeast strains whose glycosylation pathways have been "humanized," resulting in the production of an antibody with a partially or fully human glycosylation pattern. *See* Gerngross, Nat. Biotech. 22:1409-1414 (2004), and Li et al., Nat. Biotech. 24:210-215 (2006).

Suitable host cells for the expression of glycosylated antibody and/or glycosylated binding polypeptides are also derived from multicellular organisms (invertebrates and vertebrates). Examples of invertebrate cells include plant and insect cells. Numerous baculoviral strains have been identified which may be used in conjunction with insect cells, particularly for transfection of Spodoptera frugiperda cells.

Plant cell cultures can also be utilized as hosts. *See, e.g.,* US Patent Nos. 5,959,177, 6,040,498, 6,420,548, 7,125,978, and 6,417,429 (describing PLANTIBODIES™ technology for producing antibodies in transgenic plants).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36:59 (1977)); baby hamster kidney cells (BHK); mouse sertoli cells (TM4 cells as described, *e.g.,* in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.,* in Mather et al., Annals N.Y. Acad. Sci. 383:44-68 (1982); MRC 5 cells; and FS4 cells. Other useful mammalian host cell lines include Chinese hamster ovary (CHO) cells, including DHFR⁻ CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production and/or binding polypeptide production, *see, e.g.*, Yazaki and Wu, Methods in Mol. Bio., Vol. 248 (B.K.C. Lo, ed., Humana Press, Totowa, NJ), pp. 255-268 (2003).

While the description relates primarily to production of antibodies and/or binding polypeptides by culturing cells transformed or transfected with a vector containing antibody- and binding polypeptide-encoding nucleic acid. It is, of course, contemplated that alternative methods, which are well known in the art, may be employed to prepare antibodies and/or binding polypeptides. For instance, the appropriate amino acid sequence, or portions thereof, may be produced by direct peptide synthesis using solid-phase techniques [*see, e.g.,* Stewart et al., Solid-Phase Peptide Synthesis, W.H. Freeman Co., San Francisco, CA (1969); Merrifield, J. Am. Chem. Soc., 85:2149-2154 (1963)]. In vitro protein synthesis may be performed using manual techniques or by automation. Automated synthesis may be accomplished, for instance, using an Applied Biosystems Peptide Synthesizer (Foster City, CA) using manufacturer's instructions. Various portions of the antibody and/or binding polypeptide may be chemically synthesized separately and combined using chemical or enzymatic methods to produce the desired antibody and/or binding polypeptide.

*IV. Methods of Screening and/or Identifying FGF19 modulators With Desired Function*

Techniques for generating FGF19 modulators, *e.g.*, FGF19 antagonists, such as antibodies, binding polypeptides, and/or small molecules have been described above. Additional FGF19 modulators, *e.g.*, FGF19 antagonists, such as antibodies, binding polypeptides, small molecules, and/or polynucleotides provided herein may be identified, screened for, or characterized for their physical/chemical properties and/or biological activities by various assays known in the art.

Provided herein are methods of screening for and/or identifying an FGF19 modulator, *e.g.*, FGF19 antagonist, which (A) inhibits FGF19 signaling, induces cell cycle arrest, inhibits cell proliferation, and/or promotes cell death and (B) has acceptable toxicity (*e.g.*, clinically tolerable and/or acceptable), said method comprising: (a) contacting (i) a cell, tissue, and/or sample, wherein the cell, tissue, and/or sample comprises one or more bile acid meabolism biomarker, and (ii) a reference cell, reference tissue, and/or reference sample with a candidate FGF19 modulators, *e.g.*, FGF19 antagonists, (b) determining (i) level of FGF19 signaling, distribution of cell cycle stage, level of cell proliferation, and/or level of cell death, and (ii) level of one or more bile acid meabolism biomarker, whereby decreased level of FGF19 signaling, a difference in distribution of cell cycle stage, decreased level of cell proliferation, and/or increased level of cell death and decreased and/or substantially similar levels of one or more bile acid meabolism biomarkers between the a cell, tissue, and/or sample, wherein the a cell, tissue, and/or sample comprises one or more biomarkers, and reference cell, reference tissue, and/or reference sample identifies the candidate FGF19 modulators, *e.g.*, FGF19 antagonists, as an FGF19 modulator which inhibits FGF19 signaling, induces cancer cell cycle arrest, inhibits cell proliferation, and/or promotes cell death and acceptable toxicity (*e.g.*, clinically tolerable and/or acceptable). In some embodiments, the FGF19 modulator is an FGF19 antagonist. In some embodiments, the cell, tissue, and/or sample is a cancer cell, cancer tissue, and/or cancer sample.

Methods of determining the level of FGF19 signaling are known in the art and are described in the Examples herein. In some embodiments, the levels of FGF19 signaling are determined using a luciferase reporter assay as described in the Examples. In some embodiments, the FGF19 modulator, *e.g.*, FGF19 antagonist, inhibits FGF19 signaling by reducing the level of FGF19 signaling by about any of 10, 20, 30, 40, 50, 60, 70, 80, 90, or 100%.

The growth inhibitory effects of an FGF19 modulator, *e.g.*, FGF19 antagonist, described herein may be assessed by methods known in the art, *e.g.,* using cells which express FGF19, FGFR4, and klotho *(e.g.,* KLB) either endogenously or following transfection with the respective gene(s). For example, appropriate tumor cell lines, and FGF19, FGFR4, and/or klotho *(e.g.,* KLB) polypeptide-transfected cells may be treated with an FGF19 modulator, *e.g.,* FGF19 antagonist, described herein at various concentrations for a few days *(e.g.,* 2-7) days and stained with crystal violet or MTT or analyzed by some other colorimetric assay. Another method of measuring proliferation would be by comparing $^3$H-thymidine uptake by the cells treated in the presence or absence an antibody, binding polypeptide, small molecule, and/or polynucleotides of the disclosure. After treatment, the cells are harvested and the amount of radioactivity incorporated into the DNA quantitated in a scintillation counter. Appropriate positive controls include treatment of a selected cell line with a growth inhibitory antibody known to inhibit growth of that cell line. Growth inhibition of tumor cells in vivo can be determined in various ways known in the art.

Methods of determining the distribution of cell cycle stage, level of cell proliferation, and/or level of cell death are known in the art. In some embodiments, cancer cell cycle arrest is arrest in G1.

In some embodiments, the FGF19 modulator, *e.g.*, FGF19 antagonist, will inhibit cancer cell proliferation of the cancer cell, cancer tissue, or cancer sample in vitro or in vivo by about 25-100% compared to the untreated cancer cell, cancer tissue, or cancer sample, more preferably, by about 30-100%, and even more preferably by about 50-100% or about 70-100%. For example, growth inhibition can be measured at an FGF19 modulator concentration of about 0.5 $\mu$g/ml to about 30 $\mu$g/ml or about 0.5 nM to about 200 nM in cell culture, where the growth inhibition is determined 1-10 days after exposure of the tumor cells to the FGF19 candidate antagonist. The FGF19 modulator, *e.g.*, FGF19 antagonist, is growth inhibitory in vivo if administration of the candidate FGF19 modulator, *e.g.*, candidate FGF19 antagonist, at about 1 $\mu$g/kg to about 100 mg/kg body weight results in reduction in tumor size or reduction of tumor cell proliferation within about 5 days to 3 months from the first administration of the candidate FGF19 modulator, *e.g.*, candidate FGF19 antagonist, preferably within about 5 to 30 days.

To select for an FGF19 modulator, *e.g.*, FGF19 antagonist, which induces cancer cell death, loss of membrane integrity as indicated by, *e.g.*, propidium iodide (PI), trypan blue or 7AAD uptake may be assessed relative to a reference. A PI uptake assay can be performed in the absence of complement and immune effector cells. FGF19, FGFR4, and/or klotho *(e.g.,* KLB) expressing tumor cells are incubated with medium alone or medium containing the appropriate an FGF19 modulator. The cells are incubated for a 3-day time period. Following each treatment, cells are washed and aliquoted into 35 mm strainer-capped 12 x 75 tubes (1 ml per tube, 3 tubes per treatment group) for removal of cell clumps. Tubes then receive PI (10 $\mu$g/ml). Samples may be analyzed using a FACSCAN® flow cytometer and FACSCONVERT® CellQuest software (Becton Dickinson). Those FGF19 modulators that induce statistically significant levels of cell death as determined by PI uptake may be selected as cell death-inducing antibodies, binding polypeptides, small molecules, and/or polynucleotides.

To screen for FGF19 modulator, *e.g.*, FGF19 antagonist, which bind to an epitope on or interact with a polypeptide bound by an antibody of interest, a routine cross-blocking assay such as that described in Antibodies, A Laboratory Manual, Cold Spring Harbor Laboratory, Ed Harlow and David Lane (1988), can be performed. This assay can be used to determine if a candidate FGF19 modulator binds the same site or epitope as a known antibody. Alternatively, or additionally, epitope mapping can be performed by methods known in the art. For example, the antibody and/or binding polypeptide sequence can be mutagenized such as by alanine scanning, to identify contact residues. The mutant antibody is initially tested for binding with polyclonal antibody and/or binding polypeptide to ensure proper folding. In a different method, peptides corresponding to different regions of a polypeptide can be used in competition assays with the candidate antibodies and/or polypeptides or with a candidate antibody and/or binding polypeptide and an antibody with a characterized or known epitope.

In some embodiments of any of the methods of screening and/or identifying, the candidate FGF19 modulator, *e.g.*, FGF19 antagonist, is an antibody, binding polypeptide, small molecule, or polynucleotide. In some embodiments, the candidate FGF19 modulator, *e.g.*, FGF19 antagonist, is an antibody. In some embodiments, the FGF19 modulator *(e.g.*, FGF19 antagonist) is a small molecule.

In one aspect, an FGF19 modulator, *e.g.*, FGF19 antagonist, is tested for its antigen binding activity, *e.g.*, by known methods such as ELISA, Western blot, etc.

## V. Pharmaceutical Formulations

Pharmaceutical formulations of an FGF19 modulator, *e.g.*, FGF19 antagonist, as described herein are prepared by mixing such antibody having the desired degree of purity with one or more optional pharmaceutically acceptable carriers (Remington's Pharmaceutical Sciences 16th edition, Osol, A. Ed.

(1980)), in the form of lyophilized formulations or aqueous solutions. In some embodiments, the FGF19 modulator is a small molecule, an antibody, binding polypeptide, and/or polynucleotide. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g.*, Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rHuPH20 (HYLENEX®, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

Exemplary lyophilized formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the FGF19 modulator, *e.g.,* FGF19 antagonist, which matrices are in the form of shaped articles, *e.g.,* films, or microcapsules.

The formulations to be used for in vivo administration are generally sterile. Sterility may be readily accomplished, *e.g.*, by filtration through sterile filtration membranes.

## *VI.* Articles of Manufacture

In another aspect of the disclosure, an article of manufacture containing materials useful for the treatment, prevention and/or diagnosis of the disorders described above is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is an FGF19 modulator (*e.g.*, FGF19 antagonist) described herein. The label or package insert indicates that the composition is used for treating the condition of choice. Moreover, the article of manufacture may comprise (a) a first container with a composition contained therein, wherein the composition comprises an FGF19 modulator (*e.g.*, FGF19 antagonist); and (b) a second container with a composition contained therein, wherein the composition comprises a further cytotoxic or otherwise therapeutic agent. In some embodiments, the label on the container indicates that the composition can be used for treating an individual based upon levels of one or bile acids.

In some embodiments, the article of manufacture comprises a container, a label on said container, and a composition contained within said container; wherein the composition includes one or more reagents (*e.g.*, primary antibodies that bind to one or more bile acid meabolism biomarkers or probes and/or primers to one or more of the bile acid meabolism biomarkers described herein), the label on the container indicating that the composition can be used to evaluate the presence of one or more bile acid meabolism biomarkers in a sample, and instructions for using the reagents for evaluating the presence of one or more bile acid meabolism biomarkers in a sample. The article of manufacture can further comprise a set of instructions and materials for preparing the sample and utilizing the reagents. In some embodiments, the article of manufacture may include reagents such as both a primary and secondary antibody, wherein the secondary antibody is conjugated to a label, *e.g.,* an enzymatic label. In some embodiments, the article of manufacture one or more probes and/or primers to one or more of the bile acid meabolism biomarkers described herein.

In some embodiments of any of the article of manufacture, the FGF19 modulator (*e.g.*, FGF19 antagonist) is an antibody, binding polypeptide, small molecule, or polynucleotide. In some embodiments, the antibody is a monoclonal antibody. In some embodiments, the antibody is a human, humanized, or chimeric antibody.

The article of manufacture in this embodiment may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

Other optional components in the article of manufacture include one or more buffers (*e.g.*, block buffer, wash buffer, substrate buffer, etc), other reagents such as substrate (*e.g.*, chromogen) which is chemically altered by an enzymatic label, epitope retrieval solution, control samples (positive and/or negative controls), control slide(s) etc.

It is understood that any of the above articles of manufacture may include an immunoconjugate described herein in place of or in addition to an FGF19 modulator (*e.g.*, FGF19 antagonist).

## VII. EXAMPLES

The following are examples of methods of the invention. It is understood that various other embodiments may be practiced, given the general description provided above.

### Materials and Methods for Materials and Methods

#### In vivo Study in Cynomolgus Monkeys.

Fully humanized anti-FGF19 antibody that showed binding to huFGF19 with high affinity (Kd = 118 pM) and effectively blocked binding of FGF19 to FGFR4 was used. Cynomolgus monkeys were deemed the most appropriate animal model for assessing safety because of similar binding affinity of anti-FGF19 to human and cynomolgus monkey FGF19 (data not shown), and because mice only express FGF15, an orthologue of FGF19. A toxicology study was conducted in healthy, naive cynomolgus monkeys, aged 2 - 3 years, and weighing ~3 kg. Animals were randomized into five groups (3/sex/group) and dosed with vehicle (20 mM histidine acetate, 8% trehalose dehydrate, 0.02% polysorbate 20, pH 5.5) or 3, 10, 30 and 100 mg/kg anti-FGF19 per animal as an IV bolus administered once every two weeks for 8 weeks (total of 5 doses). Standard clinical observations, body weight, clinical chemistry/hematology analyses, and anatomic pathology parameters (including organ weights, gross pathology, and histopathology) were assessed. Liver and ileal tissue samples were collected from all animals at scheduled and unscheduled necropsies and snap-frozen in liquid nitrogen for RNA analyses. Fecal samples were collected on Study Day 14 from two animals in each group and stored at -20° C. Fecal bile acids were measured by GC-MS analyses following a previously described procedure. Daggy BP et al. 1997. J Lipid Res 38(3):491-502. Mass spectrometry was used to identify all bile acids detected in the GC-MS analysis. The total bile acid output was determined from the sum of all individual bile acids, and from these concentrations, the contribution to the cholic acid and chenodeoxycholic acid pathways was determined as described previously (Setchell KD et al. 1987. Clin Chim Acta 162:257-75). Positively identified bile acids were further quantified and concentrations of bile acids normalized to $\mu$g/g wet weight as described previously in Setchell KDR et al. 1983. J Lipid Res. 24:1085-100.

#### Hepatocyte Toxicity Assay.

Cryopreserved primary cynomolgus hepatocytes were purchased from CellzDirect (Durham, NC) and plated at $0.6 \times 10^6$ cells/well in 0.1 mL in collagen I-coated plates according to the supplier's instructions. Cells were incubated overnight before adding test materials in triplicate and incubated for an additional 48 hours. Since this antibody is non-binding in rodents, dose concentrations (1.6 - 100 $\mu$g/mL) were selected based on in silico modeling using PK data from chimeric antibody administration in mouse. Tamoxifen (100 $\mu$M) served as a positive control and an isotype control antibody (gp120, Genentech Inc., CA) (100 $\mu$g/mL) was used as a negative control. At the end of incubation, cell viability was assessed by measuring ATP levels using CellTiter-Glo™ Luminescent Cell Viability Assay Kit and associated protocol (Promega; Madison, WI).

#### Hepatocyte Bile Acid Transport Studies using Cynomolgus Hepatocyte Sandwich Cultures.

Freshly isolated monkey hepatocytes plated in 6-well plates with Matrigel™ overlay were obtained from CellzDirect Inc. (Durham, NC). Bile acid uptake and efflux were evaluated using $d_8$-taurocholate as a probe substrate in cynomolgus monkey hepatocytes using B-Clear Technology™ (Qualyst Inc., NC). The cell cultures were treated with either anti-FGF19 antibody or isotype control antibody (100 $\mu$g/mL) for 6 and 24 h. The total mass of endogenous bile acids (taurocholate, TCA: glycocholate, GCA; taurochenodeoxycholate, TCDCA, glycochenodeoxycholate, GCDCA) at the end of the incubation period was determined in calcium free buffer and the total mass of compound taken up and excreted was determined in calcium-containing buffer using LC/MS/MS and quantified against standard curves prepared with stable isotope equivalents ($d_8$-TCA, $d_4$-GCA, $d_4$-TCDCA, or $d_4$-GCDCA). All mass values for $d_8$-taurocholate were normalized to milligram protein. Biliary excretion index and in vitro biliary clearance were calculated as described previously in Liu X. et al. 1999. Drug Metab Dispos 27(6):637-44.

*Intestinal Epithelial Cell (Caco-2) Permeability Studies using Transwell System.*

The Caco-2 human adenocarcinoma cell line and Eagle's Minimum Essential Medium (EMEM) were purchased from ATCC (American Type Culture Collection, Rockville, MD). Caco-2 cells were seeded on transwell inserts (0.4 $\mu$m pore size, Corning, NY) at a density of $1.5 \times 10^5$ cells/mL and cultured for 23 days with media changes 3 times per week. On day 23, the cells were treated with either isotype control antibody (100 $\mu$g/ mL), FGF19 (gp120, Genentech Inc., CA) (500 ng/ mL), anti-FGF19 (100 $\mu$g/ mL), DCA (Sigma-Aldrich, St. Louis, MO) (50 $\mu$M) or DCA (50 $\mu$M) plus anti-FGF19 (100 $\mu$g/mL) for 24 h prior to the permeability experiments. The monolayers were equilibrated for 30 min in transport buffer (HBSS with 10mM HEPES, pH 7.4) at 37°C and the transepithelial electrical resistance [TEER ($\Omega$ cm2), membrane area 1.12 cm2] of each well was measured using the volt-ohm meter and STX-2 electrode. The permeabilities of lucifer yellow (LY), and taurocholate were evaluated in the apical to basolateral (A-B) direction. The transport dose solutions consisted of transport buffer, test compound, and 100 $\mu$M of the monolayer integrity marker LY. The receiver wells consisted of transport buffer. The apparent permeability for taurocholate, and LY ($P$app) was calculated using the following equation: $P_{app} = \dfrac{dQ}{dt} \times \dfrac{1}{C_0 A}$ where d$Q$/d$t$ is the rate of compound appearance in the receiver compartment, $C0$ is the dt CoA concentration in the donor compartment and A is the surface area of the insert. LY was quantified using a SpectraMax® M5 Multi-mode plate reader (Molecular Devices, Sunnyvale, CA), Ex = 425 nm, Em= 515 nm. Samples were analyzed for compound on an API4000 (Applied Biosystems, Foster City, CA) with a 1200 series pump and degasser (Agilent Technologies, Santa Clara, CA), an Aria LX2 (Thermo Fisher Scientific, Inc., Waltham, MA) and a Leap HTS PAL Autosampler. The mobile phases consisted of water with 0.1 % formic acid (mobile phase A) and acetonitrile with 0.1 % formic acid (mobile phase B). All samples were injected onto a Synergi Hydro-RP column (Phenomenex, Torrance, CA) and analyzed using the multiple reaction monitoring (MRM) mode. Mass spectrometer data were processed using Analyst software version 1.4.2.

*Gene expression analyses.*

Total RNA was isolated from samples of liver and ileum of vehicle- or anti-FGF19-treated monkeys, primary cynomolgus monkey hepatocyte cultures and Caco-2 cell cultures using the RNeasy kit (Qiagen Inc., Valencia, CA) and DNase treated on column (Qiagen Inc., Valencia, CA) following the manufacturer's protocol. RNA concentration was determined using ND-1000 spectrophotometer (Nanodrop Products, Wilmington, DE). Quantitative RT-PCR was performed to determine the relative abundance of_ FGF19- and bile acid-regulated gene mRNAs. Human specific primers and fluorogenic probes for Cyp7$\alpha$1, BSEP, NTCP, OAT2, MRP2, MPR3, ASBT, IBABP, OST$\alpha$, OST$\beta$, FGF19 and 60s ribosomal protein L 19, (RPL19, internal control) were obtained from Applied Biosystems (Foster City, CA). Amplification reactions were performed using Taqman one-step RT-PCR master mix (ABI) on real-time Mx-3000Xp thermocycler (Stratagene, La Jolla, CA). Each sample was run in triplicate and results for genes of interest were normalized to the RPL19 housekeeping gene.

*Statistical analysis.*

Student's two-tailed $t$ test with Welch's correction was used to compare data between two groups. P-values <0.05 were considered statistically significant.

### Example 1 - In Vivo Cynomolgus Monkey Study Findings

The major clinically-observed toxicities associated with single dose administration of anti-FGF19 in the 10-100 mg/kg groups were severe diarrhea, low food consumption and decreased body weight, which resulted in unscheduled euthanasia of two animals (Table 1). Ultimately, all the animals in 10-100 mg/kg groups were euthanized after one dose due to poor clinical condition. Animals given 3 mg/kg had only mild clinical signs and minimal effects on clinical pathology parameters during the first two weeks of the study, therefore the dosing schedule of five total doses remained intact for all the animals in this group. The only test article-related clinical signs in the low-dose animals were periodic non-formed or liquid feces and decreased food consumption with no significant change in body weight (Table 1).

Table 1: Incidence of liquid/ non-formed feces, food consumption (Days 1-16) and body weight change in cynomolgus monkeys treated with vehicle (0) or 3-100 mg/kg anti-FGF19 antibody; (*reported to be dehydrated; M: male, F: female).

| Group (dose) mg/kg | Incidence of Liquid Feces | | Incidence of Non-Formed Feces | | Incidence of Low Qualitative Food Consumption | | Mean Body Weights $\pm$ SD (kg) | | | |
|---|---|---|---|---|---|---|---|---|---|---|
| | | | | | | | M | | F | |
| | M | F | M | F | M | F | Pre-dose | Terminal | Pre-dose | Terminal |
| 1 (0) | 0/5 | 0/5 | 0/5 | 1/5 | 0/5 | 2/5 | 3 $\pm$ 0.2 | 3 $\pm$ 0. 4 | 3 $\pm$ 0.3 | 3 $\pm$ 0.3 |
| 2 (3) | 1/5 | 2/5 | 4/5 | 4/5 | 2/5 | 3/5 | 3 $\pm$ 0.5 | 3 $\pm$ 0.5 | 3 $\pm$ 0.4 | 3 $\pm$ 0.4 |
| 3 (10) | 4/5 | 5/5 | 5/5 | 5/5 | 3/5 | 5/5 | 3 $\pm$ 0.3 | 2.9 $\pm$ 0.4 | 3.1 $\pm$ 0.4 | 2.9 $\pm$ 0.3 |
| 4 (30) | 5/5* | 5/5 | 5/5 | 5/5 | 4/5 | 5/5 | 3 $\pm$ 0.4 | 2.8 $\pm$ 0.4 | 3 $\pm$ 0.3 | 2.9 $\pm$ 0.5 |
| 5 (100) | 5/5 | 5/5* | 4/5 | 4/5 | 4/5 | 4/5 | 3 $\pm$ 0.3 | 2.8 $\pm$ 0.4 | 3 $\pm$ 0.2 | 2.9 $\pm$ 0.3 |

Substantial dose-related increases in aspartate aminotransferase (AST), alanine transaminase (ALT), total bile acid (TBA) and total bilirubin levels (TBIL) were observed across groups treated with 10, 30 and 100 mg/kg of anti-FGF19 (Figure 1A-1D, respectively). At their highest levels, mean activities of AST, ALT, and TBA were increased 21 - 30-fold, 13 - 27-fold, and 2 - 4-fold, respectively, vs. control animals. The 3 mg/kg treatment group showed minimal-to-mild increases in serum AST (2 - 3-fold) and serum TBA (1-fold), and mild-to-moderate increases in serum ALT (3 -6-fold), which did not become progressively worse with subsequent doses (Figure 1A-1C). Although increased ALT and TBA activities were consistent with hepatocellular injury and/or dysfunction, corresponding histopathologic changes were not observed in the majority of animals. Only two animals that underwent unscheduled euthanasia had mild-to-marked hepatocellular single cell necrosis (data not shown). Sections of stomach, duodenum, and ileum from all study animals were examined histopathologically and considered to be within normal limits, indicating that diarrhea and dehydration were the probable primary causes of moribundity and morbidity in these animals. Based on the collective data, single doses of 10, 30 and 100 mg/kg were considered to be not tolerable. Therefore, for the purposes of the current study, we analyzed tissue samples from only the vehicle and high dose (100 mg/kg) groups in an attempt to better understand the mechanisms of toxicity of anti-FGF19. Furthermore, based on the severity of clinical signs and degree of liver enzyme elevation, anti-FGF19 appeared to be more toxic in females than in males. Interestingly, a similar observation in gender effects was reported in the incidence of hepatocellular carcinoma in FGF19 transgenic mice, however the reason for this is unclear. Nicholes K. et al. 2002. Am J Pathol 160(6):2295-307.

### Example 2 - Fecal bile acid analysis from anti-FGF19-treated cynomolgus monkeys

High concentrations of bile acids (>0.5g/ 24 h) entering the large intestine can stimulate water secretion and intestinal motility and cause chronic watery diarrhea. Hofmann AF. 1967. Gastroenterology 52(4):752-7. Since the animals treated with anti-FGF19 showed elevated serum bile acid levels accompanied by severe diarrhea, it was next evaluated whether excess bile acids were present in the feces due to malabsorption. Upon GC-MS analysis of all the fecal bile acids as described in Setchell KDR et al. 1983. J Lipid Res. 24:1085-100, a substantial increase in absolute fecal bile acid concentrations was observed in the high-dose anti-FGF19-treated group when compared to the control group (p value < 0.0001). Further characterization of the positively identified bile acid profiles showed an increasing trend in the presence of secondary bile acids, LCA (derivative of CDCA) and DCA, and notably a large increase in $3\alpha$, $12\alpha$-hydroxy-$5\beta$-cholanoic acid (both derivatives of CA, all p value < 0.05) (Fig. 2A - 2D).

### Example 3 - The effect of anti-FGF19 treatment on primary cynomolgus monkey hepatocytes

Since the clinical chemistry findings indicated increased serum AST and ALT levels, it was first determined whether anti-FGF19 could cause direct hepatocyte cytotoxicity by treating primary monkey hepatocytes with anti-FGF19 in varying concentrations (1.56 - 100 $\mu$g/mL) for up to 48 h. No significant change in ATP levels were observed between the treated and untreated cells at all the concentrations studies (data not shown). These findings suggest that anti-FGF19 has no direct cytotoxicity on primary monkey hepatocytes.

### Example 4 - The effect of anti-FGF19 treatment on FGF19 and bile acid-regulated gene expression in cynomolgus monkey liver and in primary hepatocyte cultures

Significant increases in Cyp7$\alpha$1 (>4-fold), BSEP (>2-fold), MRP2 (>1.6-fold), and MRP3 (>1.3-fold) (all p value < 0.05) gene expression levels were observed in the liver tissue of monkeys treated with anti-FGF19 at 100 mg/kg compared to vehicle-treated controls (Figs. 3A - 3D). In contrast, NTCP (8% decrease), OAT2 (35% decrease) and MRP4 (64.9% decrease, p value < 0.04) expression levels were moderately reduced (Figs. 3E - 3G). In primary monkey hepatocyte cultures, anti-FGF19 (100 $\mu$g/ml, 24 h) caused similar increases in expression levels of CYP7$\alpha$1 (>11-fold), BSEP (>9-fold) (both p value < 0.05), MRP2 (>1.1-fold), MRP3 (>1.5-fold, p value < 0.05) genes (Figs. 4A - 4D) and a small decrease only in NTCP expression vs. control (Fig. 4F).

### Example 5 - The effect of anti-FGF19 treatment on bile acid transport function in hepatocytes

Treatment of monkey hepatocytes with anti-FGF19 for 6 and 24 hours caused minimal changes in uptake and efflux of bile acids as determined by the total mass of endogenous bile acids (taurocholate, TCA; glycocholate, GCA; taurochenodeoxycholate, TCDCA; glycochenodeoxycholate, GCDCA) in the presence and absence of calcium when compared with isotype control (Table 2). Some decrease in BEI was observed at 24 h but was not statistically significant due to high variability (Table 3).

Table 2: Effect of Anti-FGF19 (100 μg/mL) on bile acid uptake and efflux in B-CLEAR®-monkey hepatocytes. Endogenous bile acids (TCA, GCA, TCDCA, and GCDCA) accumulated in the presence and absence of calcium were quantified using LS/MS/MS. All data are mean of the % isotype control ± STDEV; n=3.

| Incubation time | Buffer | TCA | GCA | TDCA | GDCA |
|---|---|---|---|---|---|
| 6 hour | - | 126 ± 24.9 | 101 ± 8.46 | 142 ± 52.7 | 145 ± 48.5 |
| | + | 153 ± 81.5 | 189 ± 143 | 277 ± 290 | 265 ± 279 |
| 24 hour | - | 114 ± 28.2 | 115 ± 37.9 | 120 ± 29.9 | 129 ± 39.2 |
| | + | 100 ± 36.0 | 108.4 ± 34.7 | 105 ± 32.7 | 105 ± 40.8 |

Table 3: Biliary Excretion Index (BEI) of endogenous bile acids (TCA, GCA, TCDCA, and GCDCA) following incubation with anti-FGF19 (100 μg/mL) in B-CLEAR®-monkey hepatocytes (detailed in Material and Methods). All data are mean of the % isotype control ± STDEV; n=3.

| Incubation time | TCA | GCA | TDCA | GDCA |
|---|---|---|---|---|
| 6 hour | 173 ± 146 | 110 ± 67.2 | 127 ± 30.9 | 102 ± 23.0 |
| 24 hour | 65.2 ± 41.1 | 79.6 ± 41.9 | 79.9 ± 15.6 | 79.3 ± 23.7 |

*Example 6 - The effect of anti-FGF19 treatment on bile acid transporter gene expressions in cynomolgus monkey ileum and human intestinal epithelial cells (Caco-2)*

Ileal tissue samples from the 100 mg/ kg anti-FGF19 treated animals showed increased Ost-$\alpha$ (>2.7-fold), Ost-$\beta$ (>2.3-fold), and IBABP (>2.7-fold) mRNA expression levels compared to controls, but the changes were not statistically significant due to a high variability in the control group (Figs. 5A - 5C). To further ascertain whether these elevations were due to a direct effect of the anti-FGF19 or an indirect effect from the anti-FGF19-induced bile acids, we assessed the selected solute transporters' gene expression in Caco-2 cell cultures after treatment with either anti-FGF19 (100 μg/mL, 24 h) or DCA (50 μM, 24 h). In Caco-2 cells, anti-FGF19 antibody treatment did not cause any significant change in mRNA expression of these solute transporters, whereas treatment with DCA significantly increased IBABP (>30-fold, p value < 0.0001), Ost-$\alpha$ (2-fold, p value < 0.0015), and Ost-$\beta$ (1.3-fold, p value < 0.05) expression similar to that observed in vivo (Fig. 6A - 6C), indicating the changes observed in the ileal tissues is likely driven by the elevated bile acids and may not be a direct effect of anti-FGF19 antibody. In addition, anti-FGF19 treatment caused a modest reduction in ASBT mRNA expression levels in vivo and in vitro (Figs. 5D & 6D).

*Example 7 - The effect of anti-FGF19 treatment on intestinal epithelial cells (Caco-2) membrane permeability and transport function*

Pretreatment of Caco-2 monolayers with FGF19 protein (500 ng/ml, 24 h) did not alter taurocholate uptake or membrane permeability. However, treatment with anti-FGF19 (100 μg/mL, 24 h) greatly compromised taurocholate uptake (~64% reduction vs. control, p value < 0.002) in vitro, and a similar effect was observed with DCA (50 μM, 24 h) treatment (~80% reduction vs. control, p value < 0.001). Since bile acids have been shown to induce FGF19 expression in intestinal epithelial (LS174T) cells (Wistuba W. et al. 2007. World J Gastroenterol 13(31):4230-5), we determined whether DCA influenced Caco-2 transport function indirectly through the induction of FGF19 and whether this effect can be attenuated by pre-treating cells with anti-FGF19. Treatment of Caco-2 cells with DCA (50 μM, 24 h) significantly induced FGF19 mRNA expression (Fig. 7A, p value < 0.001); however pre-treatment with anti-FGF19 (100 μg/ml, 24 h) only partially rescued DCA-mediated decrease in taurocholate uptake (~30% recovery vs. DCA) (Fig. 7B). Overall, DCA inhibited ileal epithelial cell conjugated bile acid (taurocholate) uptake both directly and, in part, through induction of ileal epithelial cell FGF19 expression.

Fibroblast growth factor 19 (FGF19) represses cholesterol 7$\alpha$-hydroxylase (Cyp7$\alpha$1) and inhibits bile acid synthesis in vitro and in vivo. Previous studies have shown that anti-FGF19 antibody treatment reduces growth of colon tumor xenografts and prevents hepatocellular carcinomas in FGF19 transgenic mice and thus may be a useful cancer target. In a repeat-dose safety study in cynomolgus monkeys, anti-FGF19 treatment (3-100 mg/kg) demonstrated dose-related liver toxicity accompanied by severe diarrhea and low food consumption. The mechanism of anti-FGF19 toxicity was investigated using in vitro and in vivo approaches. The results show that anti-FGF19 antibody had no direct cytotoxic effect on monkey hepatocytes. Anti-FGF19 increased Cyp7$\alpha$1 as expected, but also increased bile acid efflux transporter gene (BSEP, MRP2, MRP3) expression and reduced NTCP and OAT2 expression in liver tissues from treated monkeys and in primary hepatocytes. In addition, anti-FGF19 treatment increased solute transporter gene (IBABP, OST-$\alpha$, OST-$\beta$) expression in ileal tissues from treated monkeys but not in Caco-2 cells. However, deoxycholic acid (DCA: a secondary bile acid) increased expression of FGF19 and these solute transporter genes in Caco-2 cells. GC-MS analysis of monkey feces showed an increase in total bile acids and cholic acid derivatives. These findings suggest that high doses of anti-FGF19 increase Cyp7$\alpha$1 expression and bile acid synthesis and alter the expression of bile transporters in the liver resulting in enhanced bile acid efflux and reduced uptake. Increased bile acids alter expression of solute transporters in the ileum causing diarrhea and the enhanced enterohepatic recirculation of bile acids leading to liver toxicity.

Multiple processes including absorption, distribution, metabolism, and excretion are known to influence the pharmacokinetics, pharmacodynamics and toxicity of drugs. In the present study, anti-FGF19 antibody treatment at doses >3mg/kg causes liver toxicity in cynomolgus monkeys as evidenced by elevated serum liver enzymes and bilirubin. Other adverse effects of treatment included acute diarrhea, low food consumption and morbidity. The studies suggest that these effects are related to dysregulation of bile acid metabolism and impaired bile acid reabsorption in the ileum.

FGF15 and $\beta$-klotho knockout mice show elevated hepatic Cyp7$\alpha$1 mRNA and fecal bile acid excretion (Inagaki T. et al. 2005. Cell Metab 2(4):217-25; Ito S. et al. 2005. J Clin Invest 115(8):2202-8; Wu X. et al. 2007. J Biol Chem 282(40):29069-7), and mutations in Cyp7$\alpha$1 gene have been identified in patients with hypercholesterolemia, premature gallstone diseases and premature atherosclerosis (Pullinger CR et al. 2002. J Clin Invest 110(1): 109-17) suggesting negative feedback repression of Cyp7$\alpha$1 transcription by bile acids is crucial for maintaining bile acid and cholesterol homeostasis. The data herein shows increased hepatic expression of Cyp7$\alpha$1 in anti-FGF19 treated groups and elevated bile acids in the serum and feces of monkeys. In addition, increased Cyp7$\alpha$1 expression in primary monkey hepatocytes after anti-FGF19 treatment further shows that anti-FGF19 directly affects Cyp7$\alpha$1 and results in increased bile acid synthesis in these animals.

Enterohepatic circulation of bile acids is mainly driven by the functioning of specific transporters expressed in the liver and intestine and alterations may contribute to elevations in serum bile acids. Expression profiles of 50 major transporter genes across human and cynomolgus monkeys showed no variability in organ-specific expression and conservation of transporter genes. Bleasby K. et al. 2006. Xenobiotica 36(10-11):963-88. While bile acid transport across the basolateral membrane of hepatocytes is largely mediated by NTCP as well as OATP, bile acid efflux occurs via MRP3 and MRP4 (compensatory role). The ATP-binding cassette transporters, BSEP and MRP2 mediate the secretion of bile acids across the canalicular membrane, which are either passively or actively absorbed in the distal ileum via ASBT. Induction of BSEP and suppression of NTCP gene expression by high levels of bile acids is thought to be an adaptive response to the accumulation of hydrophobic bile acids in hepatocytes. Anwer MS. 2004. Hepatology 39(3):581-90; Arrese M. and Ananthanarayanan M. 2004. Pflugers Arch 449(2):123-31. The current data showing that inhibition of FGF19 resulted in a reduction of NTCP and OATP expression levels and a substantially increased expression of BSEP, MRP2 and MRP3 both in vivo and in vitro suggest altered bile acid transport in the liver. Furthermore, the in vivo effects were recapitulated in vitro, suggesting that the in vitro primary hepatocyte model was a reasonable model of anti-FGF19 effects. Unfortunately, changes in bile acid transporter genes did not translate in to a significant functional effect in our hepatocytes sandwich culture model, which could be due to high variability observed in that model.

The intracellular transport of bile acids across the luminal aspect of enterocytes is facilitated by the IBABP and, Ost-$\alpha$ and Ost-$\beta$ facilitate transport from enterocytes for reentry to the portal blood to complete enterohepatic circulation. Alrefai WA and Gill RK. 2007. Pharm Res 24(10):1803-23. Increased expression of IBABP and Ost-$\alpha$ was observed in the ileal tissue from monkeys treated with anti-FGF19 that had diarrhea. In addition, increased expression of IBABP and Ost-$\alpha$ in bile acid-treated intestinal epithelial cells (Caco-2) was observed indicating changes consistent with bile acid absorption in the ileum. Furthermore, the data showing DCA increased FGF19 expression in Caco-2 cells and decreased taurocholate uptake are in agreement with previous studies showing bile acids directly up-regulate FGF19 and decrease trans-epithelial electrical resistance (TEER) in intestinal epithelial cells. Hughes R. et al. 2008. Nutr Cancer 60(2):259-66; Raimondi F. et al. 2008. Am J Physiol Gastrointest Liver Physiol 294(4):G906-13. Interestingly, in the current study, co-treatment of Caco-2 monolayers with DCA and anti-FGF19 only partially rescued DCA-mediated reduction in taurocholate uptake indicating that DCA-mediated decrease in transport function may not be fully dependent on FGF19. Several studies showing that bile acids regulate ileal transporter genes, such as ASBT, IBABP, Ost$\alpha$ and Ost$\beta$_ and their importance in bile acid transport in the small intestine and homeostasis are consistent with the data showing altered ileal transporter expression in response to bile acids in vivo and in vitro. Ballatori N. et al. 2008. Am J Physiol Gastrointest Liver Physiol 295(1):G179-G186; Dawson PA et al. 2003. J Biol Chem 278(36):33920-7; Dawson PA et al. 2005. J Biol Chem 280(8):6960-8; Lee H. et al. 2006. J Lipid Res 47(1):201-14; Nakahara M. et al. 2005. J Biol Chem 280(51):42283-9; Rao A. et al. 2008. Proc Natl Acad Sci USA 105(10):3891-6.

Chenodeoxycholic acid (CDCA) and cholic acid (CA) are the immediate products of bile acid synthetic pathways referred to as primary bile acids, which upon conjugation and dehydroxylation yields major secondary bile acids, LCA and DCA, respectively. Lefebvre P. et al. 2009. Physiol Rev 89:147-91. Under normal conditions most secondary bile acids are actively reabsorbed in the ileum and a small nonabsorbed fraction excreted in the feces. While the total fecal bile acid excretion reflects a measure of hepatic bile acid synthesis under steady-state conditions, elevated fecal bile acid excretion has been associated with altered ileal transporters and enterohepatic circulation. Lefebvre P. et al. 2009. Physiol Rev 89:147-91. The data showed changes in fecal bile acid composition associated with ileal transporter gene expression in animals treated with anti-FGF19 indicate perturbed enterohepatic circulation. In addition, the cathartic effect of an excess of fecal bile acids has been shown to be one of the underlying causes in several clinical settings associated with bile acid malabsorption manifested through chronic watery diarrhea and steatorrhea. Westergaard H. 2007. Curr Treat Options Gastroenterol 10(1):28-33. In the Examples, characterization of fecal bile acids showed an increase in cholic acid metabolites, including DCA and iso-lithocholic acid, which are highly cathartic as described in Hofmann AF. 1977. The J Infect Dis. 135, Supplement, S126-32, and may explain the diarrhea observed in the anti-FGF19 treated monkeys. While another study showed patients with bile acid malabsorption have reduced serum FGF19. Walters JR et al. 2009. Clin Gastroenterol Hepatol 7(11):1189-94, this is the first study showing antibody-mediated inhibition of FGF19 caused increased bile acid synthesis and subsequent malabsorption finds support.

In conclusion, the findings as illustrated in Figure 8, show that inhibition of FGF19 with anti-FGF19 de-represses Cyp7$\alpha$1 regulation and increases bile acid synthesis in vitro and in vivo and also alters hepatic bile acid transporter expression. As a result, elevated serum bile acids subsequently alter bile acid transporter expression in the intestine resulting in perturbation of enterohepatic circulation and the development of diarrhea and liver toxicity. This study is the first to demonstrate this relationship with bile acid homeostasis in a non-human primate as a result of on-target effects.

Although the foregoing invention has been described in some detail by way of illustration and example for purposes of clarity of understanding, the descriptions and examples should not be construed as limiting the scope of the invention.

## Claims

1. A method of determining whether an individual with cancer should continue or discontinue treatment comprising an FGF19 antagonist antibody, the method comprising measuring in a sample from the individual levels of one or more bile acid metabolism biomarkers, wherein i) elevated levels of one or more bile acid metabolism biomarkers indicates that the individual should discontinue treatment comprising the FGF19 antagonist antibody as the individual is more likely to develop toxicity to the treatment and ii) reduced levels of one or more bile acid metabolism biomarkers indicates that the individual should continue treatment comprising the FGF19 antagonist antibody as the individual is less likely to develop toxicity to the treatment, wherein the bile acid metabolism biomarker is BSEP, MRP2 and/or MRP3, wherein the anti-FGF19 antagonist antibody comprises: (i) a light chain comprising (a) hypervariable region (HVR)-L1 comprising amino acid sequence KASQDINSFLA (SEQ ID NO:1) or KASQDINSFLS (SEQ ID NO:7); (b) HVR-L2 comprising amino acid sequence RANRLVD (SEQ ID NO:2), RANRLVS (SEQ ID NO:8), or RANRLVE (SEQ ID NO:9); and (c) HVR-L3 comprising amino acid sequence LQYDEFPLT (SEQ ID NO:3); and (ii) a heavy chain comprising (a) HVR-H1 comprising amino acid sequence GFSLTTYGVH (SEQ ID NO:4); (b) HVR-H2 comprising amino acid sequence is GVIWPGGGTDYNAAFIS (SEQ ID NO:5); and (c) HVR-H3 comprising amino acid sequence VRKEYANLYAMDY (SEQ ID NO:6).

2. The method of claim 1, wherein the sample is a serum sample and/or a fecal sample.

3. The method of any one of claims 1-2, wherein the antibody comprises (i) human K subgroup 1 consensus framework sequence, or (ii) heavy chain human subgroup III consensus framework sequence.

## Patentansprüche

1. Verfahren zum Bestimmen, ob ein Individuum mit Krebs eine Behandlung, umfassend einen FGF19-Antagonisten-Antikörper, fortsetzen sollte oder nicht, wobei das Verfahren das Messen der Spiegel eines oder mehrerer Gallensäure-Stoffwechsel-Biomarker in einer Probe von dem Individuum umfasst, wobei i) erhöhte Spiegel eines oder mehrerer Gallensäure-Stoffwechsel-Biomarker angeben, dass das Individuum die Behandlung, umfassend den FGF19-Antagonisten-Antikörper, nicht fortsetzen sollte, da bei dem Individuum eine größere Wahrscheinlichkeit zur Entwicklung einer Toxizität auf die Behandlung besteht, und ii) reduzierte Spiegel eines oder mehrerer Gallensäure-Stoffwechsel-Biomarker angeben, dass das Individuum die Behandlung, umfassend den FGF19-Antagonisten-Antikörper, fortsetzen sollte, da bei dem Individuum eine geringere Wahrscheinlichkeit zur Entwicklung einer Toxizität auf die Behandlung besteht, wobei der Gallensäure-Stoffwechsel-Biomarker BSEP, MRP2 und/oder MRP3 ist, wobei der Anti-FGF19-Antagonisten-Antikörper Folgendes umfasst: (i) eine Leichtkette, umfassend (a) eine hypervariable Region (HVR)-L1, umfassend die Aminosäuresequenz KASQDINSFLA (SEQ ID NO:1) oder KASQDINSFLS (SEQ ID NO:7); (b) HVR-L2, umfassend die Aminosäuresequenz RANRLVD (SEQ ID NO:2), RANRLVS (SEQ ID NO:8) oder RANRLVE (SEQ ID NO:9); und (c) HVR-L3, umfassend die Aminosäuresequenz LQYDEFPLT (SEQ ID NO:3); und (ii) eine Schwerkette, umfassend (a) HVR-H1, umfassend die Aminosäuresequenz GFSLTTYGVH (SEQ ID NO:4); (b) HVR-H2, umfassend die Aminosäuresequenz GVIWPGGGTDYNAAFIS (SEQ ID NO:5); und (c) HVR-H3, umfassend die Aminosäuresequenz VRKEYANLYAMDY (SEQ ID NO:6).

2. Verfahren nach Anspruch 1, wobei die Probe eine Serumprobe und/oder eine Stuhlprobe ist.

3. Verfahren nach einem der Ansprüche 1-2, wobei der Antikörper Folgendes umfasst: (i) die Consensus-Gerüstsequenz der menschlichen K-Untergruppe 1, oder (ii) eine Consensus-Gerüstsequenz der menschlichen Schwerketten-Untergruppe III.

## Revendications

1. Procédé destiné à déterminer si un individu souffrant d'un cancer devrait poursuivre ou arrêter un traitement comprenant un anticorps antagoniste du FGF19, ledit procédé comprenant la mesure dans un échantillon des niveaux individuels d'au moins un biomarqueur du métabolisme des acides biliaires, dans lequel i) les niveaux élevés d'au moins un biomarqueur du métabolisme des acides biliaires indiquent que l'individu devrait arrêter son traitement comprenant l'anticorps antagoniste du FGF19 car l'individu est plus enclin à développer une toxicité liée au traitement et ii) les niveaux réduits d'au moins un biomarqueur du métabolisme des acides biliaires indiquent que l'individu devrait poursuivre le traitement comprenant l'anticorps antagoniste du FGF19 car l'individu est moins enclin à développer une toxicité liée au traitement, dans lequel le biomarqueur du métabolisme des acides biliaires est le BSEP, le MRP2 et/ou le MRP3, l'anticorps antagoniste anti-FGF19 comprenant : (i) une chaîne légère comprenant une (a) région hypervariable (HVR)-L1 comprenant la séquence d'acide aminés KASQDINSFLA (ID SEQ N° 1) ou KASQDINSFLS (ID SEQ N° 7) ; (b) HVR-L2 comprenant la séquence d'acide aminés RANRLVD (ID SEQ N° 2), RANRLVS (ID SEQ N° 8) ou RANRLVE (ID SEQ N° 9) et (c) HVR-L3 comprenant la séquence d'acide aminés LQYDEFPLT (ID SEQ N° 3) et (ii) une chaîne lourde comprenant (a) HVR-H1 comprenant la séquence d'acide aminés GFSLTTYGVH (ID SEQ N° 4) ; (b) HVR-H2 comprenant la séquence d'acide aminés GVIWPGGGTDYNAAFIS (ID SEQ N° 5) et (c) HVR-H3 comprenant la séquence d'acide aminés VRKEYANLYAMDY (ID SEQ N° 6).

2. Procédé selon la revendication 1, dans lequel l'échantillon est un échantillon de sérum et/ou un échantillon fécal.

3. Procédé selon l'une quelconque des revendications 1 ou 2, dans lequel l'anticorps comprend (i) une séquence charpente consensus humaine du sous-groupe K I ou (ii) une séquence charpente consensus humaine du sous-groupe III de la chaîne lourde..

Figure 1

Figure 2

# Figure 3

Figure 4

Figure 5

Figure 6

# Figure 7

# Figure 8

# Figure 9A

EP 2 804 630 B1

Kabat#  1 2 3 4 5 6 7 8 9 10 11 12 13 14 15 16 17 18 19 20 21 22 23  24 25 26 27 A B C D E F 28 29 30 31 32 33 34  35 36 37

Kabat - CDR L1
Chothia - CDR L1
Contact - CDR L1

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | A | B | C | D | E | F | 28 | 29 | 30 | 31 | 32 | 33 | 34 | 35 | 36 | 37 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| huKl | D | I | Q | M | T | Q | S | P | S | S | L | S | A | S | V | G | D | R | V | T | I | T | C | R | A | S | Q | | | | | | | S | I | S | N | Y | L | A | W | Y | Q |
| anti-FGF19 antibody 1 | D | I | K | M | T | Q | S | P | S | S | M | Y | A | S | L | G | E | R | V | T | I | P | C | K | A | S | Q | | | | | | | D | I | N | S | F | L | S | W | F | Q |
| anti-FGF19 antibody 2 | D | I | Q | M | T | Q | S | P | S | S | L | S | A | S | V | G | D | R | V | T | I | T | C | K | A | S | Q | | | | | | | D | I | N | S | F | L | S | W | Y | Q |
| anti-FGF19 antibody 3 | D | I | Q | M | T | Q | S | P | S | S | L | S | A | S | V | G | D | R | V | T | I | T | C | K | A | S | Q | | | | | | | D | I | N | S | F | L | A | W | Y | Q |
| anti-FGF19 antibody 4 | D | I | Q | M | T | Q | S | P | S | S | L | S | A | S | V | G | D | R | V | T | I | T | C | K | A | S | Q | | | | | | | D | I | N | S | F | L | A | W | Y | Q |

Kabat#  38 39 40 41 42 43 44 45 46 47 48 A 49  50 51 52 53 54 55 56  57 58 59 60 61 62 63 64 65 66 67 68 69 70 71 72 73 74 75 76 77 78 79 80

Kabat - CDR L2
Chothia - CDR L2
Contact - CDR L2

| | 38 | 39 | 40 | 41 | 42 | 43 | 44 | 45 | 46 | 47 | 48 | A | 49 | 50 | 51 | 52 | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| huKl | Q | K | P | G | K | A | P | K | L | L | I | | Y | A | A | S | S | L | E | S | G | V | P | S | R | F | S | G | S | G | S | G | T | D | F | T | L | T | I | S | S | L | Q | P |
| anti-FGF19 antibody 1 | Q | K | P | G | K | S | P | K | T | L | I | | Y | R | A | N | R | L | V | D | G | V | P | S | R | F | S | G | S | G | S | G | Q | D | Y | S | L | T | I | S | S | L | E | Y |
| anti-FGF19 antibody 2 | Q | K | P | G | K | A | P | K | L | L | I | | Y | R | A | N | R | L | V | D | G | V | P | S | R | F | S | G | S | G | S | G | T | D | F | T | L | T | I | S | S | L | Q | P |
| anti-FGF19 antibody 3 | Q | K | P | G | K | A | P | K | L | L | I | | Y | R | A | N | R | L | V | D | G | V | P | S | R | F | S | G | S | G | S | G | T | D | F | T | L | T | I | S | S | L | Q | P |
| anti-FGF19 antibody 4 | Q | K | P | G | K | A | P | K | L | L | I | | Y | R | A | N | R | L | V | S | G | V | P | S | R | F | S | G | S | G | S | G | T | D | F | T | L | T | I | S | S | L | Q | P |

Kabat#  81 82 83 84 85 86 87 88  89 90 91 92 93 94 95 A B C D E F 96 97  98 99 100 101 102 103 104 105 106 107 108

Kabat - CDR L3
Chothia - CDR L3
Contact - CDR L3

| | 81 | 82 | 83 | 84 | 85 | 86 | 87 | 88 | 89 | 90 | 91 | 92 | 93 | 94 | 95 | A | B | C | D | E | F | 96 | 97 | 98 | 99 | 100 | 101 | 102 | 103 | 104 | 105 | 106 | 107 | 108 |
|---|---|---|---|---|---|---|---|---|---|---|---|---|---|---|---|---|---|---|---|---|---|---|---|---|---|---|---|---|---|---|---|---|---|---|
| huKl | E | D | F | A | T | Y | Y | C | Q | Q | Y | N | S | L | P | | | | | | | W | T | F | G | Q | G | T | K | V | E | I | K | R |
| anti-FGF19 antibody 1 | E | D | M | G | I | Y | Y | C | L | Q | Y | D | E | F | P | | | | | | | L | T | F | G | A | G | T | K | V | E | I | K | R |
| anti-FGF19 antibody 2 | E | D | F | A | T | Y | Y | C | L | Q | Y | D | E | F | P | | | | | | | L | T | F | G | Q | G | T | K | V | E | I | K | R |
| anti-FGF19 antibody 3 | E | D | F | A | T | Y | Y | C | L | Q | Y | D | E | F | P | | | | | | | L | T | F | G | Q | G | T | K | V | E | I | K | R |
| anti-FGF19 antibody 4 | E | D | F | A | T | Y | Y | C | L | Q | Y | D | E | F | P | | | | | | | L | T | F | G | Q | G | T | K | V | E | I | K | R |

# Figure 9B

| Kabat# | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 | 29 | 30 | 31 | 32 | 33 | 34 | 35 | A | B | | 36 | 37 | 38 | 39 | 40 | 41 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | | | | | | | | | | | | | | | | | | | | | | | | | | Kabat - CDR H1 | | | | | | | | | | | | | | | | | | |
| | | | | | | | | | | | | | | | | | | | | | | | | Chothia - CDR H1 | | | | | | | | | | | | | | | | | | |
| | | | | | | | | | | | | | | | | | | | | | | | | Contact - CDR H1 | | | | | | | | | | | | | | | | | | |
| hum III | E | V | Q | L | V | E | S | G | G | G | L | V | Q | P | G | G | S | L | R | L | S | C | A | A | S | G | F | T | F | S | S | Y | A | M | S | | | | W | V | R | Q | A | P |
| anti-FGF19 antibody 1 | Q | V | Q | L | K | Q | S | G | P | G | L | V | Q | P | S | Q | S | L | S | I | T | C | T | V | S | G | F | S | L | T | T | Y | G | V | H | | | | W | V | R | Q | S | P |
| anti-FGF19 antibody 2 | E | V | Q | L | V | E | S | G | G | G | L | V | Q | P | G | G | S | L | R | L | S | C | A | A | S | G | F | S | L | T | T | Y | G | V | H | | | | W | V | R | Q | A | P |
| anti-FGF19 antibody 3 | E | V | Q | L | V | E | S | G | G | G | L | V | Q | P | G | G | S | L | R | L | S | C | A | A | S | G | F | S | L | T | T | Y | G | V | H | | | | W | V | R | Q | A | P |
| anti-FGF19 antibody 4 | E | V | Q | L | V | E | S | G | G | G | L | V | Q | P | G | G | S | L | R | L | S | C | A | A | S | G | F | S | L | T | T | Y | G | V | H | | | | W | V | R | Q | A | P |

| Kabat# | 42 | 43 | 44 | 45 | 46 | 47 | 48 | 49 | 50 | 51 | 52 | a | b | c | 53 | 54 | 55 | 56 | 57 | 58 | 59 | 60 | 61 | 62 | 63 | 64 | 65 | 66 | 67 | 68 | 69 | 70 | 71 | 72 | 73 | 74 | 75 | 76 | 77 | 78 | 79 | 80 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | | | | | | | | Kabat - CDR H2 | | | | | | | | | | | | | | | | | | | | | | | | | | | | | | | | | | |
| | | | | | | | | Chothia - CDR H2 | | | | | | | | | | | | | | | | | | | | | | | | | | | | | | | | | | |
| | | | | | | | Contact - CDR H2 | | | | | | | | | | | | | | | | | | | | | | | | | | | | | | | | | | | |
| hum III | G | K | G | L | E | W | V | S | V | I | S | G | | | D | G | G | S | T | Y | Y | A | D | S | V | K | G | R | F | T | I | S | R | D | N | S | K | N | T | L | Y | L |
| anti-FGF19 antibody 1 | G | K | G | L | E | W | L | G | V | I | W | P | | | | G | G | G | T | D | Y | N | A | A | F | I | S | R | L | S | I | T | K | D | N | S | K | S | Q | V | F | F |
| anti-FGF19 antibody 2 | G | K | G | L | E | W | V | G | V | I | W | P | | | | G | G | G | T | D | Y | N | A | A | F | I | S | R | F | T | I | S | R | D | N | S | K | N | T | L | Y | L |
| anti-FGF19 antibody 3 | G | K | G | L | E | W | V | G | V | I | W | P | | | | G | G | G | T | D | Y | N | A | A | F | I | S | R | F | T | I | S | R | D | N | S | K | N | T | L | Y | L |
| anti-FGF19 antibody 4 | G | K | G | L | E | W | V | G | V | I | W | P | | | | G | G | G | T | D | Y | N | A | A | F | I | S | R | F | T | I | S | R | D | N | S | K | N | T | L | Y | L |

| Kabat# | 81 | 82 | A | B | C | 83 | 84 | 85 | 86 | 87 | 88 | 89 | 90 | 91 | 92 | 93 | 94 | 95 | 96 | 97 | 98 | 99 | 100 | A | B | C | D | E | F | G | H | I | J | K | 101 | 102 | 103 | 104 | 105 | 106 | 107 | 108 | 109 | 110 | 111 | 112 | 113 |
|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|---|
| | | | | | | | | | | | | | | | | | | Kabat - CDR H3 | | | | | | | | | | | | | | | | | | | | | | | | | | | | | | |
| | | | | | | | | | | | | | | | | | Chothia - CDR H3 | | | | | | | | | | | | | | | | | | | | | | | | | | | | | | |
| | | | | | | | | | | | | | | | | | Contact - CDR H3 | | | | | | | | | | | | | | | | | | | | | | | | | | | | | | |
| hum III | Q | M | N | S | L | R | A | E | D | T | A | V | Y | Y | C | A | R | G | | | | | | | | | | | | | | | | | F | D | Y | W | G | Q | G | T | L | V | T | V | S | S |
| anti-FGF19 antibody 1 | K | M | N | S | L | L | A | N | D | T | A | I | Y | F | C | V | R | K | E | Y | A | N | L | Y | A | | | | | | | | | | M | D | Y | W | G | Q | G | T | L | L | T | V | S | A |
| anti-FGF19 antibody 2 | Q | M | N | S | L | R | A | E | D | T | A | V | Y | Y | C | V | R | K | E | Y | A | N | L | Y | A | | | | | | | | | | M | D | Y | W | G | Q | G | T | L | V | T | V | S | S |
| anti-FGF19 antibody 3 | Q | M | N | S | L | R | A | E | D | T | A | V | Y | Y | C | V | R | K | E | Y | A | N | L | Y | A | | | | | | | | | | M | D | Y | W | G | Q | G | T | L | V | T | V | S | S |
| anti-FGF19 antibody 4 | Q | M | N | S | L | R | A | E | D | T | A | V | Y | Y | C | V | R | K | E | Y | A | N | L | Y | A | | | | | | | | | | M | D | Y | W | G | Q | G | T | L | V | T | V | S | S |

## REFERENCES CITED IN THE DESCRIPTION

### Patent documents cited in the description

- US 2007248604 A
- US 4275149 A
- US 4737456 A
- US 4318980 A
- US 4016043 A
- US 4424279 A
- US 4018653 A
- US 67341107 A
- US 67197411 A
- WO 9316185 A
- US 5571894 A
- US 5587458 A
- US 5869046 A
- EP 404097 A
- WO 199301161 A
- US 6248516 B1
- US 4816567 A
- US 5821337 A
- US 7527791 B
- US 6982321 B
- US 7087409 B
- US 6075181 A
- US 6150584 A
- US 5770429 A
- US 7041870 B
- US 20070061900 A
- US 7189826 B
- US 5750373 A
- US 20050079574 A
- US 20050119455 A
- US 20050266000 A
- US 20070117126 A
- US 20070160598 A
- US 20070237764 A
- US 20070292936 A
- US 20090002360 A
- WO 9308829 A
- US 5731168 A
- WO 2009089004 A1
- US 4676980 A
- US 20060025576 A1
- US 20080069820 A
- WO 2008077546 A
- US 20030157108 A, Presta, L.
- US 20040093621 A
- WO 200061739 A
- WO 200129246 A
- US 20030115614 A
- US 20020164328 A
- US 20040132140 A
- US 20040110704 A
- US 20040110282 A
- US 20040109865 A
- WO 2003085119 A
- WO 2003084570 A
- WO 2005035586 A
- WO 2005035778 A
- WO 2005053742 A
- WO 2002031140 A
- US 20030157108 A1, Presta, L
- WO 2004056312 A1, Adams
- WO 2003085107 A
- WO 2003011878 A, Jean-Mairet
- US 6602684 B, Umana
- US 20050123546 A, Umana
- WO 199730087 A, Patel
- WO 199858964 A, Raju, S.
- WO 199922764 A, Raju, S.
- US 5500362 A
- WO 2006029879 A
- WO 2005100402 A
- US 6737056 B
- US 7332581 B
- WO 2004056312 A
- US 6194551 B
- WO 9951642 A
- US 20050014934 A1, Hinton
- US 7371826 B
- US 5648260 A
- US 5624821 A
- WO 9429351 A
- US 7521541 B
- US 5208020 A
- US 5416064 A
- EP 0425235 B1
- US 5635483 A
- US 5780588 A
- US 7498298 B
- US 5712374 A
- US 5714586 A
- US 5739116 A
- US 5767285 A
- US 5770701 A
- US 5770710 A
- US 5773001 A
- US 5877296 A
- US 6630579 B
- WO 9411026 A

- US 5556762 A
- US 4708871 A
- US 4833092 A
- US 5223409 A
- US 5403484 A
- US 5571689 A
- US 5663143 A
- WO 8403506 A
- WO 8403564 A
- WO 9534683 A
- US 5627024 A
- US 5766905 A
- WO 9814277 A
- WO 9820169 A
- WO 9820159 A
- WO 9820036 A
- WO 9735196 A
- WO 9746251 A
- WO 9747314 A
- WO 9709446 A
- US 5498538 A
- US 5432018 A
- WO 9815833 A
- US 5723286 A
- US 5580717 A
- US 5427908 A
- US 5498530 A
- US 5770434 A
- US 5734018 A
- US 5698426 A
- US 5763192 A
- US 5723323 A
- WO 0000823 A
- WO 0039585 A
- US 5648237 A
- US 5789199 A
- US 5840523 A
- US 5959177 A
- US 6040498 A
- US 6420548 B
- US 7125978 B
- US 6417429 B
- US 20050260186 A
- US 20060104968 A
- US 6267958 B
- US 6171586 B
- WO 2006044908 A

**Non-patent literature cited in the description**

- **CHIANG JY.** *J Hepatol,* 2004, vol. 40 (3), 539-51
- **RUSSELL DW.** *Annu Rev Biochem,* 2003, vol. 72, 137-74
- **CHIANG JY.** *J Lipid Res,* 2009, vol. 50 (10), 1955-66
- **DIETSCHY JM ; TURLEY SD.** *J Biol Chem,* 2002, vol. 277 (6), 3801-4
- **HAYES KC.** *Nutr Res Rev,* 1988, vol. 1 (1), 99-113
- **RUDEL LL et al.** *J Biol Chem,* 2002, vol. 277 (35), 31401-6
- **ALNOUTI Y.** *Toxicol Sci,* 2009, vol. 108 (2), 225-46
- **MAKISHIMA M. et al.** *Science,* 1999, vol. 284 (5418), 1362-5
- **PARKS DJ et al.** *Science,* 1999, vol. 284 (5418), 1365-8
- **ANANTHANARAYANAN M. et al.** *J Biol Chem,* 2001, vol. 276 (31), 28857-65
- **KAST HR et al.** *J Biol Chem,* 2002, vol. 277 (4), 2908-15
- **PLASS JR et al.** *Hepatology,* 2002, vol. 35 (3), 589-96
- **ELORANTA JJ ; KULLAK-UBLICK GA.** *Physiology,* 2008, vol. 23, 286-95
- **BALLATORI N. et al.** *Hepatology,* 2005, vol. 42 (6), 1270-9
- **DAWSON PA et al.** *J Biol Chem,* 2005, vol. 280 (8), 6960-8
- **BALLATORI N. et al.** *Am J Physiol Gastrointest Liver Physiol,* 2008, vol. 295 (1), G179-G186
- **DAWSON PA et al.** *J Biol Chem,* 2003, vol. 278 (36), 33920-7
- **NAKAHARA M. et al.** *J Biol Chem,* 2005, vol. 280 (51), 42283-9
- **INAGAKI T. et al.** *Cell Metab,* 2005, vol. 2 (4), 217-25
- **WRIGHT TJ et al.** *Dev Biol,* 2004, vol. 269 (1), 264-75
- **JONES S.** *Mol. Pharma.,* 2008, vol. 5 (1), 42-48
- **XIE MH et al.** *Cytokine,* 1999, vol. 11 (10), 729-35
- **YU C. et al.** *J Biol Chem,* 2000, vol. 275 (20), 15482-9
- **HOLT JA et al.** *Genes Dev,* 2003, vol. 17 (13), 1581-91
- **LIN BC et al.** *J Biol Chem,* 2007, vol. 282 (37), 27277-84
- **ITO S. et al.** *J Clin Invest,* 2005, vol. 115 (8), 2202-8
- **DESNOYERS LR et al.** *Oncogene,* 2008, vol. 27 (1), 85-97
- **SONG KH et al.** *Hepatology.,* 2009, vol. 49 (1), 297-305
- **FLATMAN et al.** *J. Chromatogr. B,* 2007, vol. 848, 79-87

- **KABAT et al.** Sequences of Proteins of Immunological Interest. Public Health Service, National Institutes of Health, 1991
- **KABAT et al.** Sequences of Proteins of Immunological Interest. NIH Publication 91-3242, 1991, vol. 1-3
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917
- **ALMAGRO ; FRANSSON.** *Front. Biosci.,* 2008, vol. 13, 1619-1633
- **AUSUBEL et al.** Current Protocols in Molecular Biology. Wiley InterScience Publishers, 1995
- **SAMBROOK et al.** Molecular Cloning: A Laboratory Manual. Cold Spring Harbor Press, 1989
- **NICOLAOU et al.** *Angew. Chem Intl. Ed. Engl.,* 1994, vol. 33, 183-186
- **WILMAN.** Prodrugs in Cancer Chemotherapy. *Biochemical Society Transactions,* 1986, vol. 14, 375-382
- Prodrugs: A Chemical Approach to Targeted Drug Delivery. **STELLA et al.** Directed Drug Delivery. Humana Press, 1985, 247-267
- Cell cycle regulation, oncogenes, and antineoplastic drugs. **MURAKAMI et al.** The Molecular Basis of Cancer. 1995, 13
- Current Protocols In Molecular Biology. 1995
- **LIU X. et al.** *Drug Metab Dispos,* 1999, vol. 27 (6), 637-44
- **CHEN et al.** *J. Mol. Biol.,* 1999, vol. 293, 865-881
- **PRESTA et al.** *Cancer Res.,* 1997, vol. 57, 4593-4599
- **HUDSON et al.** *Nat. Med.,* 2003, vol. 9, 129-134
- **PLUCKTHÜN.** The Pharmacology of Monoclonal Antibodies. Springer-Verlag, 1994, vol. 113, 269-315
- **HOLLINGER et al.** *Proc. Natal. Acad. Sci. USA,* 1993, vol. 90, 6444-6448
- **MORRISON et al.** *Proc. Natal. Acad. Sci. USA,* 1984, vol. 81, 6851-6855
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL'ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol.,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **VAN DIJK ; VAN DE WINKEL.** *Curr. Opin. Pharmacol.,* 2001, vol. 5, 368-74
- **LONBERG.** *Curr. Opin. Immunol.,* 2008, vol. 20, 450-459
- **LONBERG.** *Nat. Biotech.,* 2005, vol. 23, 1117-1125
- **KOZBOR.** *J. Immunol.,* 1984, vol. 133, 3001
- **BOERNER et al.** *J. Immunol.,* 1991, vol. 147, 86
- **LI et al.** *Proc. Natl. Acad. Sci. USA,* 2006, vol. 103, 3557-3562
- **NI.** *Xiandai Mianyixue,* 2006, vol. 26 (4), 265-268
- **VOLLMERS ; BRANDLEIN.** *Histology and Histopathology,* 2005, vol. 20 (3), 927-937
- **VOLLMERS ; BRANDLEIN.** *Methods and Findings in Exp. & Clin. Pharma.,* 2005, vol. 27 (3), 185-91
- **HOOGENBOOM et al.** Methods in Molecular Biology. Human Press, 2001, vol. 178, 1-37
- **MCCAFFERTY et al.** *Nature,* vol. 348, 552-554
- **CLACKSON et al.** *Nature,* 1991, vol. 352, 624-628
- **MARKS et al.** *J. Mol. Biol.,* 1992, vol. 222, 581-597
- **MARKS ; BRADBURY.** Methods in Molecular Biology. Human Press, 2003, vol. 248, 161-175
- **SIDHU et al.** *J. Mol. Biol.,* 2004, vol. 338 (2), 299-310
- **LEE et al.** *J. Mol. Biol.,* 2004, vol. 340 (5), 1073-1093
- **FELLOUSE.** *Proc. Natal. Acad. Sci. USA,* 2004, vol. 101 (34), 12467-12472
- **LEE et al.** *J. Immunol. Methods,* 2004, vol. 284 (1-2), 119-132
- **WINTER et al.** *Ann. Rev. Immunol.,* 1994, vol. 12, 433-455
- **GRIFFITHS et al.** *EMBO J,* 1993, vol. 12, 725-734
- **HOOGENBOOM ; WINTER.** *J. Mol. Biol.,* 1992, vol. 227, 381-388
- **MILSTEIN ; CUELLO.** *Nature,* 1983, vol. 305, 537

- **TRAUNECKER et al.** *EMBO J.,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **GRUBER et al.** *J. Immunol.,* 1994, vol. 152, 5368
- **TUTT et al.** *J. Immunol.,* 1991, vol. 147, 60
- **WRIGHT et al.** *TIBTECH,* 1997, vol. 15, 26-32
- **OKAZAKI et al.** *J. Mol. Biol.,* 2004, vol. 336, 1239-1249
- **YAMANE-OHNUKI et al.** *Biotech. Bioeng.,* 2004, vol. 87, 614
- **RIPKA et al.** *Arch. Biochem. Biophys.,* 1986, vol. 249, 533-545
- **KANDA, Y. et al.** *Biotech. Bioeng.,* 2006, vol. 94 (4), 680-688
- **RAVETCH ; KINET.** *Annu. Rev. Immunol.,* 1991, vol. 9, 457-492
- **HELLSTROM, I. et al.** *Proc. Nat'l Acad. Sci. USA,* 1986, vol. 83, 7059-7063
- **HELLSTROM, I et al.** *Proc. Nat'l Acad. Sci. USA,* 1985, vol. 82, 1499-1502
- **BRUGGEMANN, M. et al.** *J. Exp. Med.,* 1987, vol. 166, 1351-1361
- **CLYNES et al.** *Proc. Nat'l Acad. Sci. USA,* 1998, vol. 95, 652-656
- **GAZZANO-SANTORO et al.** *J. Immunol. Methods,* 1996, vol. 202, 163
- **CRAGG, M.S. et al.** *Blood,* 2003, vol. 101, 1045-1052
- **CRAGG, M.S. ; M.J. GLENNIE.** *Blood,* 2004, vol. 103, 2738-2743
- **PETKOVA, S.B. et al.** *Int'l. Immunol.,* 2006, vol. 18 (12), 1759-1769
- **SHIELDS et al.** *J. Biol. Chem.,* 2001, vol. 9 (2), 6591-6604
- **IDUSOGIE et al.** *J. Immunol.,* 2000, vol. 164, 4178-4184
- **GUYER et al.** *J. Immunol.,* 1976, vol. 117, 587
- **KIM et al.** *J. Immunol.,* 1994, vol. 24, 249
- **DUNCAN ; WINTER.** *Nature,* 1988, vol. 322, 738-40
- **HINMAN et al.** *Cancer Res.,* 1993, vol. 53, 3336-3342
- **LODE et al.** *Cancer Res.,* 1998, vol. 58, 2925-2928
- **KRATZ et al.** *Current Med. Chem.,* 2006, vol. 13, 477-523
- **JEFFREY et al.** *Bioorganic & Med. Chem. Letters,* 2006, vol. 16, 358-362
- **TORGOV et al.** *Bioconj. Chem.,* 2005, vol. 16, 717-721
- **NAGY et al.** *Proc. Natal. Acad. Sci. USA,* 2000, vol. 97, 829-834
- **DUBOWCHIK et al.** *Bioorg. & Med. Chem. Letters,* 2002, vol. 12, 1529-1532
- **KING et al.** *J. Med. Chem.,* 2002, vol. 45, 4336-4343
- **VITETTA et al.** *Science,* 1987, vol. 238, 1098
- **CHARI et al.** *Cancer Res.,* 1992, vol. 52, 127-131
- **GEYSEN et al.** *Proc. Nat'l Acad. Sci. USA,* 1984, vol. 81, 3998-4002
- **GEYSEN et al.** *Proc. Nat'l Acad. Sci. USA,* 1985, vol. 82, 178-182
- **GEYSEN et al.** *Synthetic Peptides as Antigens,* 1986, 130-149
- **GEYSEN et al.** *J. Immunol. Meth.,* 1987, vol. 102, 259-274
- **SCHOOFS et al.** *J. Immunol.,* 1988, vol. 140, 611-616
- **CWIRLA, S. E. et al.** *Proc. Natal. Acad. Sci. USA,* 1990, vol. 87, 6378
- **LOWMAN, H.B. et al.** *Biochemistry,* 1991, vol. 30, 10832
- **CLACKSON, T. et al.** *Nature,* 1991, vol. 352, 624
- **MARKS, J. D. et al.** *J. Mol. Biol.,* 1991, vol. 222, 581
- **KANG, A.S. et al.** *Proc. Natal. Acad. Sci. USA,* 1991, vol. 88, 8363
- **SMITH, G. P.** *Current Opin. Biotechnol.,* 1991, vol. 2, 668
- **SCOTT, J.K. ; SMITH, G. P.** *Science,* 1990, vol. 249, 386
- **REN et al.** *Gene,* 1998, vol. 215, 439
- **ZHU et al.** *Cancer Research,* 1998, vol. 58 (15), 3209-3214
- **JIANG et al.** *Infection & Immunity,* 1997, vol. 65 (11), 4770-4777
- **REN et al.** *Gene,* 1997, vol. 195 (2), 303-311
- **REN.** *Protein Sci.,* 1996, vol. 5, 1833
- **EFIMOV et al.** *Virus Genes,* 1995, vol. 10, 173
- **SMITH ; SCOTT.** *Methods in Enzymology,* 1993, vol. 217, 228-257
- **LI et al.** *Mol Biotech.,* 1998, vol. 9, 187
- **WAGNER, R.** *Nature,* 1994, vol. 372, 333-335
- **BERNOIST ; CHAMBON.** *Nature,* 1981, vol. 29, 304-310
- **YAMAMOTO et al.** *Cell,* 1980, vol. 22, 787-797
- **WAGNER et al.** *Proc. Nat'l Acad. Sci. USA,* 1981, vol. 78, 1441-1445
- **BRINSTER et al.** *Nature,* 1982, vol. 296, 39-42
- **CHOWDHURY.** *Methods Mol. Biol.,* 2008, vol. 207, 179-196
- **HOOGENBOOM et al.** *Methods in Molecular Biology,* vol. 178, 1-37
- Human Press. 2001
- **CUNNINGHAM ; WELLS.** *Science,* 1989, vol. 244, 1081-1085
- **KAM et al.** *Proc. Natal. Acad. Sci. USA,* 2005, vol. 102, 11600-11605

- **CHARLTON.** *Methods in Mol. Bio.,* vol. 248
- Humana Press. 2003, 245-254
- **GERNGROSS.** *Nat. Biotech.,* 2004, vol. 22, 1409-1414
- **LI et al.** *Nat. Biotech.,* 2006, vol. 24, 210-215
- **GRAHAM et al.** *J. Gen Virol.,* 1977, vol. 36, 59
- **MATHER.** *Biol. Reprod.,* 1980, vol. 23, 243-251
- **MATHER et al.** *Annals N.Y. Acad. Sci.,* 1982, vol. 383, 44-68
- **URLAUB et al.** *Proc. Natl. Acad. Sci. USA,* 1980, vol. 77, 4216
- **YAZAKI ; WU.** *Methods in Mol. Bio.,* vol. 248
- Humana Press. 2003, 255-268
- **STEWART et al.** Solid-Phase Peptide Synthesis. W.H. Freeman Co, 1969
- **MERRIFIELD.** *J. Am. Chem. Soc.,* 1963, vol. 85, 2149-2154
- **ED HARLOW ; DAVID LANE.** Antibodies, A Laboratory Manual. Cold Spring Harbor Laboratory, 1988
- Remington's Pharmaceutical Sciences. 1980
- **DAGGY BP et al.** *J Lipid Res,* 1997, vol. 38 (3), 491-502
- **SETCHELL KD et al.** *Clin Chim Acta,* 1987, vol. 162, 257-75
- **SETCHELL KDR et al.** *J Lipid Res.,* 1983, vol. 24, 1085-100
- **NICHOLES K. et al.** *Am J Pathol,* 2002, vol. 160 (6), 2295-307
- **HOFMANN AF.** *Gastroenterology,* 1967, vol. 52 (4), 752-7
- **WISTUBA W. et al.** *World J Gastroenterol,* 2007, vol. 13 (31), 4230-5
- **WU X. et al.** *J Biol Chem,* 2007, vol. 282 (40), 29069-7
- **PULLINGER CR et al.** *J Clin Invest,* 2002, vol. 110 (1), 109-17
- **BLEASBY K. et al.** *Xenobiotica,* 2006, vol. 36 (10-11), 963-88
- **ANWER MS.** *Hepatology,* 2004, vol. 39 (3), 581-90
- **ARRESE M. ; ANANTHANARAYANAN M.** *Pflugers Arch,* 2004, vol. 449 (2), 123-31
- **ALREFAI WA ; GILL RK.** *Pharm Res,* 2007, vol. 24 (10), 1803-23
- **HUGHES R. et al.** *Nutr Cancer,* 2008, vol. 60 (2), 259-66
- **RAIMONDI F. et al.** *Am J Physiol Gastrointest Liver Physiol,* 2008, vol. 294 (4), G906-13
- **LEE H. et al.** *J Lipid Res,* 2006, vol. 47 (1), 201-14
- **RAO A. et al.** *Proc Natl Acad Sci USA,* 2008, vol. 105 (10), 3891-6
- **LEFEBVRE P. et al.** *Physiol Rev,* 2009, vol. 89, 147-91
- **WESTERGAARD H.** *Curr Treat Options Gastroenterol,* 2007, vol. 10 (1), 28-33
- **HOFMANN AF.** *The J Infect Dis.,* 1977, vol. 135, S126-32
- **WALTERS JR et al.** *Clin Gastroenterol Hepatol,* 2009, vol. 7 (11), 1189-94

SEQUENCE LISTING

    <110> GENENTECH, INC. ET AL.

    <120> METHODS OF USING FGF19 MODULATORS

    <130> P4854R1 WO

    <140>
    <141>

    <150> 61/587,885
    <151> 2012-01-18

    <160> 20

    <170> PatentIn version 3.5

    <210> 1
    <211> 11
    <212> PRT
    <213> Artificial Sequence

    <220>
    <221> source
    <223> /note="Description of Artificial Sequence: Synthetic peptide"

    <400> 1

          Lys Ala Ser Gln Asp Ile Asn Ser Phe Leu Ala
          1              5              10

    <210> 2
    <211> 7

<212> PRT
<213> Artificial Sequence

<220>
<221> source
<223> /note="Description of Artificial Sequence: Synthetic peptide"

<400> 2

```
Arg Ala Asn Arg Leu Val Asp
1               5
```

<210> 3
<211> 9
<212> PRT
<213> Artificial Sequence

<220>
<221> source
<223> /note="Description of Artificial Sequence: Synthetic peptide"

<400> 3

```
Leu Gln Tyr Asp Glu Phe Pro Leu Thr
1               5
```

<210> 4
<211> 10
<212> PRT
<213> Artificial Sequence

<220>
<221> source
<223> /note="Description of Artificial Sequence: Synthetic peptide"

<400> 4

```
Gly Phe Ser Leu Thr Thr Tyr Gly Val His
1               5                   10
```

<210> 5
<211> 17
<212> PRT
<213> Artificial Sequence

<220>
<221> source
<223> /note="Description of Artificial Sequence: Synthetic peptide"

<400> 5

```
Gly Val Ile Trp Pro Gly Gly Gly Thr Asp Tyr Asn Ala Ala Phe Ile
1               5                   10                  15

Ser
```

<210> 6

<211> 13
<212> PRT
<213> Artificial Sequence

<220>
<221> source
<223> /note="Description of Artificial Sequence: Synthetic peptide"

<400> 6

```
Val Arg Lys Glu Tyr Ala Asn Leu Tyr Ala Met Asp Tyr
1               5                   10
```

<210> 7
<211> 11
<212> PRT
<213> Artificial Sequence

<220>
<221> source
<223> /note="Description of Artificial Sequence: Synthetic peptide"

<400> 7

```
Lys Ala Ser Gln Asp Ile Asn Ser Phe Leu Ser
1               5                   10
```

<210> 8
<211> 7
<212> PRT
<213> Artificial Sequence

<220>
<221> source
<223> /note="Description of Artificial Sequence: Synthetic peptide"

<400> 8

```
Arg Ala Asn Arg Leu Val Ser
1               5
```

<210> 9
<211> 7
<212> PRT
<213> Artificial Sequence

<220>
<221> source
<223> /note="Description of Artificial Sequence: Synthetic peptide"

<400> 9

```
Arg Ala Asn Arg Leu Val Glu
1               5
```

<210> 10
<211> 23

<212> PRT
<213> Homo sapiens

<400> 10

```
        Gly Phe Leu Pro Leu Ser His Phe Leu Pro Met Leu Pro Met Val Pro
        1               5                   10                  15

        Glu Glu Pro Glu Asp Leu Arg
                    20
```

<210> 11
<211> 108
<212> PRT
<213> Homo sapiens

<400> 11

```
        Asp Ile Gln Met Thr Gln Ser Pro Ser Ser Leu Ser Ala Ser Val Gly
        1               5                   10                  15

        Asp Arg Val Thr Ile Thr Cys Arg Ala Ser Gln Ser Ile Ser Asn Tyr
                    20                  25                  30

        Leu Ala Trp Tyr Gln Gln Lys Pro Gly Lys Ala Pro Lys Leu Leu Ile
                    35                  40                  45

        Tyr Ala Ala Ser Ser Leu Glu Ser Gly Val Pro Ser Arg Phe Ser Gly
            50                  55                  60

        Ser Gly Ser Gly Thr Asp Phe Thr Leu Thr Ile Ser Ser Leu Gln Pro
        65                  70                  75                  80

        Glu Asp Phe Ala Thr Tyr Tyr Cys Gln Gln Tyr Asn Ser Leu Pro Trp
                        85                  90                  95

        Thr Phe Gly Gln Gly Thr Lys Val Glu Ile Lys Arg
                    100                 105
```

<210> 12
<211> 108
<212> PRT
<213> Artificial Sequence

<220>
<221> source
<223> /note="Description of Artificial Sequence: Synthetic polypeptide"

<400> 12

```
Asp Ile Lys Met Thr Gln Ser Pro Ser Ser Met Tyr Ala Ser Leu Gly
1               5               10              15

Glu Arg Val Thr Ile Pro Cys Lys Ala Ser Gln Asp Ile Asn Ser Phe
            20              25              30

Leu Ser Trp Phe Gln Gln Lys Pro Gly Lys Ser Pro Lys Thr Leu Ile
        35              40              45

Tyr Arg Ala Asn Arg Leu Val Asp Gly Val Pro Ser Arg Phe Ser Gly
    50              55              60

Ser Gly Ser Gly Gln Asp Tyr Ser Leu Thr Ile Ser Ser Leu Glu Tyr
65              70              75              80

Glu Asp Met Gly Ile Tyr Tyr Cys Leu Gln Tyr Asp Glu Phe Pro Leu
            85              90              95

Thr Phe Gly Ala Gly Thr Lys Val Glu Ile Lys Arg
            100             105
```

<210> 13
<211> 108
<212> PRT
<213> Artificial Sequence

<220>
<221> source
<223> /note="Description of Artificial Sequence: Synthetic polypeptide"

<400> 13

```
Asp Ile Gln Met Thr Gln Ser Pro Ser Ser Leu Ser Ala Ser Val Gly
1               5               10              15

Asp Arg Val Thr Ile Thr Cys Lys Ala Ser Gln Asp Ile Asn Ser Phe
            20              25              30

Leu Ser Trp Tyr Gln Gln Lys Pro Gly Lys Ala Pro Lys Leu Leu Ile
            35              40              45

Tyr Arg Ala Asn Arg Leu Val Asp Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Leu Gln Tyr Asp Glu Phe Pro Leu
            85              90              95

Thr Phe Gly Gln Gly Thr Lys Val Glu Ile Lys Arg
            100             105
```

<210> 14
<211> 108
<212> PRT
<213> Artificial Sequence

<220>
<221> source
<223> /note="Description of Artificial Sequence: Synthetic polypeptide"

<400> 14

```
Asp Ile Gln Met Thr Gln Ser Pro Ser Ser Leu Ser Ala Ser Val Gly
1               5               10              15

Asp Arg Val Thr Ile Thr Cys Lys Ala Ser Gln Asp Ile Asn Ser Phe
            20              25              30

Leu Ala Trp Tyr Gln Gln Lys Pro Gly Lys Ala Pro Lys Leu Leu Ile
            35              40              45

Tyr Arg Ala Asn Arg Leu Val Asp Gly Val Pro Ser Arg Phe Ser Gly
    50              55              60

Ser Gly Ser Gly Thr Asp Phe Thr Leu Thr Ile Ser Ser Leu Gln Pro
65              70              75              80

Glu Asp Phe Ala Thr Tyr Tyr Cys Leu Gln Tyr Asp Glu Phe Pro Leu
```

                        85                    90                    95

        Thr Phe Gly Gln Gly Thr Lys Val Glu Ile Lys Arg
                    100                   105

<210> 15
<211> 108
<212> PRT
<213> Artificial Sequence

<220>
<221> source
<223> /note="Description of Artificial Sequence: Synthetic polypeptide"

<400> 15


        Asp Ile Gln Met Thr Gln Ser Pro Ser Ser Leu Ser Ala Ser Val Gly
        1               5               10                  15


        Asp Arg Val Thr Ile Thr Cys Lys Ala Ser Gln Asp Ile Asn Ser Phe
                    20                  25                  30


        Leu Ala Trp Tyr Gln Gln Lys Pro Gly Lys Ala Pro Lys Leu Leu Ile
                    35                  40                  45


        Tyr Arg Ala Asn Arg Leu Val Ser Gly Val Pro Ser Arg Phe Ser Gly
            50                  55                  60


        Ser Gly Ser Gly Thr Asp Phe Thr Leu Thr Ile Ser Ser Leu Gln Pro
        65                  70                  75                  80


        Glu Asp Phe Ala Thr Tyr Tyr Cys Leu Gln Tyr Asp Glu Phe Pro Leu
                        85                  90                  95


        Thr Phe Gly Gln Gly Thr Lys Val Glu Ile Lys Arg
                    100                   105

<210> 16
<211> 113
<212> PRT
<213> Homo sapiens

<400> 16

```
Glu Val Gln Leu Val Glu Ser Gly Gly Gly Leu Val Gln Pro Gly Gly
1               5               10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Val Ile Ser Gly Asp Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Gly Phe Asp Tyr Trp Gly Gln Gly Thr Leu Val Thr Val Ser
            100                 105                 110

Ser
```

<210> 17
<211> 119
<212> PRT
<213> Artificial Sequence

<220>
<221> source
<223> /note="Description of Artificial Sequence: Synthetic polypeptide"

<400> 17

```
Gln Val Gln Leu Lys Gln Ser Gly Pro Gly Leu Val Gln Pro Ser Gln
1               5               10              15

Ser Leu Ser Ile Thr Cys Thr Val Ser Gly Phe Ser Leu Thr Thr Tyr
            20              25              30

Gly Val His Trp Val Arg Gln Ser Pro Gly Lys Gly Leu Glu Trp Leu
        35              40              45

Gly Val Ile Trp Pro Gly Gly Gly Thr Asp Tyr Asn Ala Ala Phe Ile
    50              55              60

Ser Arg Leu Ser Ile Thr Lys Asp Asn Ser Lys Ser Gln Val Phe Phe
65              70              75              80

Lys Met Asn Ser Leu Leu Ala Asn Asp Thr Ala Ile Tyr Phe Cys Val
            85              90              95

Arg Lys Glu Tyr Ala Asn Leu Tyr Ala Met Asp Tyr Trp Gly Gln Gly
        100             105             110

Thr Leu Leu Thr Val Ser Ala
        115
```

<210> 18
<211> 119
<212> PRT
<213> Artificial Sequence

<220>
<221> source
<223> /note="Description of Artificial Sequence: Synthetic polypeptide"

<400> 18

```
Glu Val Gln Leu Val Glu Ser Gly Gly Gly Leu Val Gln Pro Gly Gly
1               5               10              15

Ser Leu Arg Leu Ser Cys Ala Ala Ser Gly Phe Ser Leu Thr Thr Tyr
            20              25              30

Gly Val His Trp Val Arg Gln Ala Pro Gly Lys Gly Leu Glu Trp Val
            35              40              45

Gly Val Ile Trp Pro Gly Gly Gly Thr Asp Tyr Asn Ala Ala Phe Ile
    50              55              60

Ser Arg Phe Thr Ile Ser Arg Asp Asn Ser Lys Asn Thr Leu Tyr Leu
65              70              75              80

Gln Met Asn Ser Leu Arg Ala Glu Asp Thr Ala Val Tyr Tyr Cys Val
                85              90              95

Arg Lys Glu Tyr Ala Asn Leu Tyr Ala Met Asp Tyr Trp Gly Gln Gly
            100             105             110

Thr Leu Val Thr Val Ser Ser
            115
```

<210> 19
<211> 119
<212> PRT
<213> Artificial Sequence

<220>
<221> source
<223> /note="Description of Artificial Sequence: Synthetic polypeptide"

<400> 19

```
Glu Val Gln Leu Val Glu Ser Gly Gly Gly Leu Val Gln Pro Gly Gly
1               5               10              15

Ser Leu Arg Leu Ser Cys Ala Ala Ser Gly Phe Ser Leu Thr Thr Tyr
            20              25              30
```

```
Gly Val His Trp Val Arg Gln Ala Pro Gly Lys Gly Leu Glu Trp Val
        35                  40                  45


Gly Val Ile Trp Pro Gly Gly Gly Thr Asp Tyr Asn Ala Ala Phe Ile
        50                  55                  60


Ser Arg Phe Thr Ile Ser Arg Asp Asn Ser Lys Asn Thr Leu Tyr Leu
65                  70                  75                  80


Gln Met Asn Ser Leu Arg Ala Glu Asp Thr Ala Val Tyr Tyr Cys Val
                85                  90                  95


Arg Lys Glu Tyr Ala Asn Leu Tyr Ala Met Asp Tyr Trp Gly Gln Gly
            100                 105                 110


Thr Leu Val Thr Val Ser Ser
            115
```

<210> 20
<211> 119
<212> PRT
<213> Artificial Sequence

<220>
<221> source
<223> /note="Description of Artificial Sequence: Synthetic polypeptide"

<400> 20

```
Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Ser Leu Thr Thr Tyr
            20                  25                  30


Gly Val His Trp Val Arg Gln Ala Pro Gly Lys Gly Leu Glu Trp Val
        35                  40                  45


Gly Val Ile Trp Pro Gly Gly Gly Thr Asp Tyr Asn Ala Ala Phe Ile
        50                  55                  60


Ser Arg Phe Thr Ile Ser Arg Asp Asn Ser Lys Asn Thr Leu Tyr Leu
65                  70                  75                  80


Gln Met Asn Ser Leu Arg Ala Glu Asp Thr Ala Val Tyr Tyr Cys Val
                85                  90                  95


Arg Lys Glu Tyr Ala Asn Leu Tyr Ala Met Asp Tyr Trp Gly Gln Gly
            100                 105                 110
```

```
Thr Leu Val Thr Val Ser Ser
                115
```